(19) Europäisches Patentamt
European Patent Office
Office européen des brevets

(11) Veröffentlichungsnummer: **0 569 810 A1**

(12) # EUROPÄISCHE PATENTANMELDUNG

(21) Anmeldenummer: **93107139.3**

(22) Anmeldetag: **03.05.93**

(51) Int. Cl.5: **C07D 261/06**, A01N 43/00, C07D 263/30, C07D 271/06, C07D 271/08, C07D 277/20, C07D 285/08

(30) Priorität: **14.05.92 DE 4215878**

(43) Veröffentlichungstag der Anmeldung:
**18.11.93 Patentblatt 93/46**

(84) Benannte Vertragsstaaten:
**BE CH DE ES FR GB IT LI NL**

(71) Anmelder: **BAYER AG**

**D-51368 Leverkusen(DE)**

(72) Erfinder: **Kirsten, Rolf, Dr.**
**Carl-Langhans-Strasse 27**
**W-4019 Monheim(DE)**
Erfinder: **Wolf, Hilmar, Dr.**
**Zum Bräuhaus 14**
**W-4018 Langenfeld(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Sulfonylierte Carbonsäureamide Herbizide.**

(57) Die Erfindung betrifft neue sulfonylierte Carbonsäureamide der allgemeinen Formel (I)

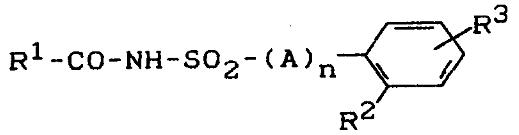

(I)

in welcher
n, A, $R^1$, $R^2$ und $R^3$ die in der Beschreibung angegebenen Definitionen haben sowie deren Salze, Verfahren zu ihrer Herstellung und ihre Verwendung als Herbizide.

EP 0 569 810 A1

Die Erfindung betrifft neue sulfonylierte Carbonsäureamide, Verfahren zu ihrer Herstellung und ihre Verwendung als Herbizide.

Einige sulfonylierte Carbonsäureamide, wie z.B. 2-Dimethylamino-N-(4-methyl-phenylsulfonyl)-5-thiazolcarboxamid (vgl. J. Org. Chem. 46 (1981), 2790-2793), N-(4-Methyl-phenylsulfonyl)-5-phenyl-1,3,4-oxadiazol-2-carboxamid (vgl. Farm. Zh. (Kiev) 1980, 65 - zitiert in Chem. Abstracts 94: 15648g) und 5-(N-Isopropyl-phenylamino)-N-(4-methyl-phenylsulfonyl)-2-oxazol-carboxamid (vgl. J. Heterocycl. Chem. 17 (1980), 711-715) sind bereits bekannt. Über die biologischen Eigenschaften dieser Verbindungen ist jedoch nichts bekannt geworden.

Weiter sind verschiedene sulfonylierte Carbonsäureamide als potentielle Herbizide bekanntgeworden (vgl. EP-A 244166/US-P 4838925, DE-OS 4029753 und EP-A 459244); diese Verbindungen haben jedoch keine größere Bedeutung erlangt.

Es wurden nun die neuen sulfonylierten Carbonsäureamide der allgemeinen Formel (I)

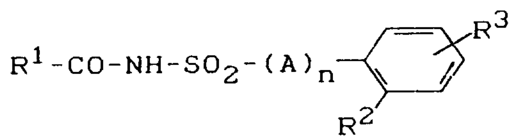

(I)

in welcher

n für die Zahlen 0 oder 1 steht,

A für Sauerstoff, Imino (NH) oder Methylen ($CH_2$) steht,

$R^1$ für einen fünfgliedrigen Heteroarylrest aus der Reihe Oxazolyl, Isoxazolyl, Thiazolyl, Isothiazolyl, Oxadiazolyl, Thiadiazolyl steht, welcher gegebenenfalls einfach oder mehrfach, insbesondere einfach oder zweifach, gleich oder verschieden durch Halogen oder durch gegebenenfalls halogen-substituiertes $C_1$-$C_6$-Alkyl, $C_1$-$C_4$-Alkoxy-$C_1$-$C_4$-alkyl, Phenyl, $C_1$-$C_6$-Alkoxy, $C_1$-$C_6$-Alkylthio, $C_1$-$C_6$-Alkylsulfinyl, $C_1$-$C_6$-Alkylsulfonyl, $C_1$-$C_6$-Alkylamino oder Di-($C_1$-$C_4$-alkyl)-amino substituiert ist,

$R^2$ für Fluor, Chlor, Brom, Cyano, Nitro, Carboxy, Phenyl oder für jeweils gegebenenfalls durch Fluor und/oder Chlor substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, für Di-($C_1$-$C_2$-alkyl)-aminosulfonyl, für N-($C_1$-$C_2$-Alkyl)-N-($C_1$-$C_2$-alkoxy)-aminosulfonyl oder für gegebenenfalls durch Fluor, Chlor oder $C_1$-$C_2$-Alkoxy substituiertes $C_1$-$C_4$-Alkoxy-carbonyl steht, und

$R^3$ für Wasserstoff, Fluor, Chlor oder Methyl steht,

sowie Salze von Verbindungen der Formel (I) gefunden.

Man erhält die neuen sulfonylierten Carbonsäureamide der Formel (I), wenn man

(a) Carbonsäureamide der allgemeinen Formel (II)

$R^1$-CO-$NH_2$ (II)

in welcher

$R^1$ die oben angegebene Bedeutung hat,

mit Sulfonylierungsmitteln der allgemeinen Formel (III)

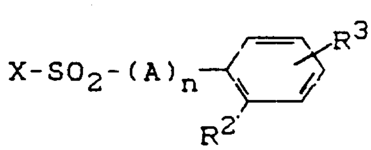

(III)

in welcher

n, A, $R^2$ und $R^3$ die oben angegebene Bedeutung haben und

X für Halogen steht,

gegebenenfalls in Gegenwart eines Säureakzeptors und gegebenenfalls in Gegenwart eines Verdünnungsmittels umsetzt, oder wenn man

(b) Carbonsäuren der allgemeinen Formel (IV)

$R^1$-COOH (IV)

in welcher
$R^1$ die oben angegebene Bedeutung hat,
oder reaktionsfähige Derivate der Carbonsäuren der Formel (IV)
mit Aminosulfonylverbindungen der allgemeinen Formel (V)

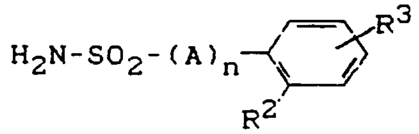

(V)

in welcher
n, A, $R^1$ und $R^2$ die oben angegebene Bedeutung haben,
oder mit reaktionsfähigen Derivaten der Verbindungen der Formel (V)
gegebenenfalls in Gegenwart von Reaktionshilfsmitteln und gegebenenfalls in Gegenwart von Verdünnungsmitteln umsetzt und gegebenenfalls die nach Verfahren (a) oder (b) erhaltenen Produkte nach üblichen Methoden in Salze überführt.
Ein weiterer möglicher Herstellungsweg für bestimmte erfindungsgemäße Verbindungen der Formel (I) - insbesondere für den Fall, daß $R^1$ für 2-Dialkylamino-thiazol-5-yl steht - ist nachstehend skizziert (vgl. J. Org. Chem. 46 (1981), 2790-2793):

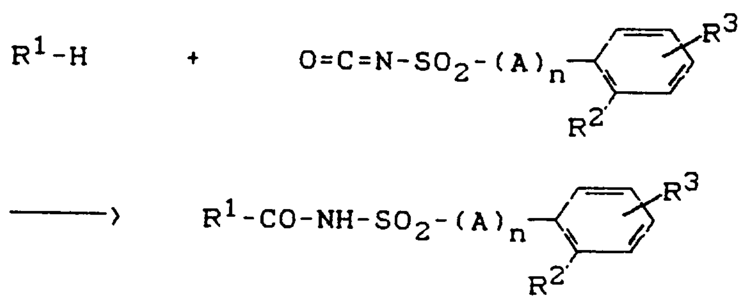

Die neuen sulfonylierten Carbonsäureamide der allgemeinen Formel (I) zeichnen sich durch starke herbizide Wirksamkeit aus.

Gegenstand der Erfindung sind vorzugsweise sulfonylierte Carbonsäureamide der Formel (I), in welcher

n für die Zahlen 0 oder 1 steht,

A für Sauerstoff, Imino (NH) oder Methylen ($CH_2$) steht,

$R^1$ für einen fünfgliedrigen Heteroarylrest aus der Reihe Oxazolyl, Isoxazolyl, Thiazolyl, Isothiazolyl, 1,2,4-Oxadiazolyl, 1,3,4-Oxadiazolyl, 1,2,4-Thiadiazolyl, 1,3,4-Thiadiazolyl steht, welcher gegebenenfalls durch Fluor, Chlor, Brom, Methyl, Ethyl, Propyl, Isopropyl, Butyl, Isobutyl, sec-Butyl, tert-Butyl, Fluormethyl, Chlormethyl, Difluormethyl, Dichlormethyl, Trifluormethyl, Trichlormethyl, Chlorfluormethyl, Chlordifluormethyl, Fluordichlormethyl, Brommethyl, Dibrommethyl, Methoxymethyl, Phenyl, Methoxy, Ethoxy, Propoxy, Isopropyloxy, Difluormethoxy, Trifluormethoxy, Chlordifluormethoxy, Methylthio, Ethylthio, Propylthio, Isopropylthio, Difluormethylthio, Trifluormethylthio, Chlordifluormethylthio, Methylsulfinyl, Ethylsulfinyl, Propylsulfinyl, Methylsulfonyl, Ethylsulfonyl, Propylsulfonyl, Methylamino, Ethylamino, Propylamino, Isopropylamino und/oder Dimethylamino substituiert ist,

$R^2$ für Fluor, Chlor, Brom, Cyano, Nitro, Carboxy, Phenyl, Methyl, Ethyl, Trifluormethyl, Methoxy, Ethoxy, 2-Chlor-ethoxy, Difluormethoxy, Trifluormethoxy, Methylthio, Ethylthio, Propylthio, Methylsulfinyl, Ethylsulfinyl, Propylsulfinyl, Methylsulfonyl, Ethylsulfonyl, Propylsulfonyl, Dimethylaminosulfonyl, Diethylaminosulfonyl, N-Methoxy-N-methylaminosulfonyl, Methoxycarbonyl, Ethoxycarbonyl, Propoxycarbonyl, Isopropoxycarbonyl, Chlorethoxycarbonyl oder Methoxyethoxycarbonyl, und

$R^3$ für Wasserstoff, Fluor, Chlor oder Methyl steht.

Die Erfindung betrifft weiter vorzugsweise Salze von Verbindungen der Formel (I) mit Basen, wie z.B. Natrium-, Kalium- oder Calcium-hydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen, Tri-($C_1$-$C_4$-alkyl)-aminen oder auch mit Tris-(2-hydroxyethyl)amin.

Die Erfindung betrifft insbesondere sulfonylierte Carbonsäureamide der Formel (I), in welcher

n für die Zahlen 0 oder 1 steht,

A für Methylen steht,

$R^1$ für 5-Methyl-1,2,4-oxadiazol-3-yl, 5-Ethyl-1,2,4-oxadiazol-3-yl, 5-Propyl-1,2,4-oxadiazol-3-yl, 5-Isopropyl-1,2,4-oxadiazol-3-yl, 4-Methyl-1,3-oxazol-2-yl, 4-Ethyl-1,3-oxazol-2-yl, 5-Methyl-1,3-oxazol-2-yl, 5-Ethyl-1,3-oxazol-2-yl oder 4,5-Dimethyl-1,3-oxazol-2-yl steht,

$R^2$ für Trifluormethyl, Phenyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Methoxycarbonyl oder Ethoxycarbonyl, und

$R^3$ für Wasserstoff, Chlor oder Methyl steht.

Die oben aufgeführten allgemeinen oder in Vorzugsbereichen aufgeführten Restedefinitionen gelten für die Endprodukte der Formel (I) sowie für die jeweiligen Ausgangsstoffe entsprechend. Diese Restedefinitionen können auch zwischen den jeweiligen Vorzugsbereichen beliebig kombiniert werden.

Verwendet man beim erfindungsgemäßen Verfahren (a) beispielsweise 2-Trifluormethoxy-phenylmethansulfonsäurechlorid und 4,5-Dimethyl-1,3-oxazol-2-carbonsäureamid als Ausgangsstoffe, so kann der Reaktionsablauf durch das folgende Formelschema skizziert werden:

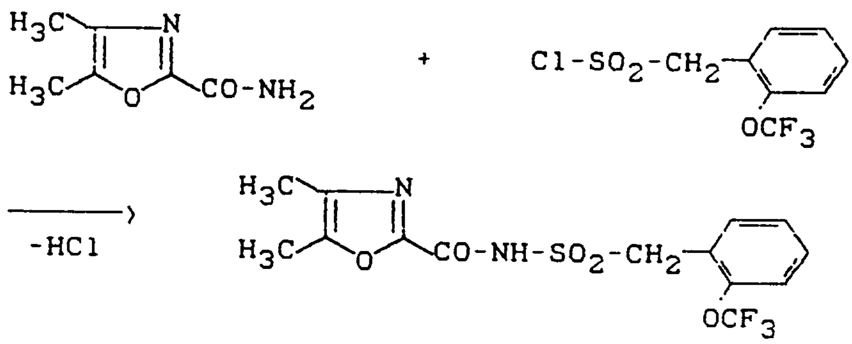


Verwendet man beim erfindungsgemäßen Verfahren (b) beispielsweise 5-Methyl-1,3-oxazol-2-carbonsäuremethylester und 2-Ethoxycarbonyl-benzolsulfonamid als Ausgangsstoffe, so kann der Reaktionsablauf durch das folgende Formelschema skizziert werden:

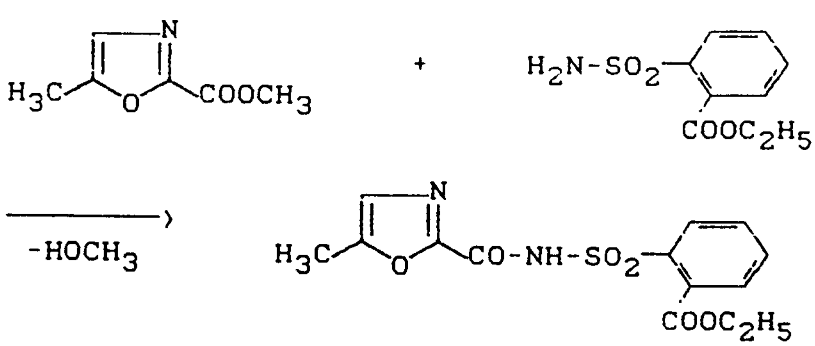


Die beim erfindungsgemäßen Verfahren (a) zur Herstellung von Verbindungen der Formel (I) als Ausgangsstoffe zu verwendenden Carbonsäureamide sind durch die Formel (II) allgemein definiert.

In Formel (II) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde.

Als Beispiele für die Ausgangsstoffe der Formel (II) seien genannt:
5-Methyl-, 5-Ethyl-, 5-Propyl-, 5-Isopropyl-, 5-Butyl-, 5-Isobutyl-, 5-sec-Butyl- und 5-tert-Butyl-isoxazol-3-carbonsäureamid, 2-Methyl-, 2-Ethyl-, 2-Propyl-, 2-Isopropyl-, 2-Butyl-, 2-Isobutyl-, 2-sec-Butyl- und 2-tert-Butyl-oxazol-4-carbonsäureamid, 5-Methyl-, 5-Ethyl-, 5-Propyl-, 5-Isopropyl- und 4,5-Dimethyl-1,3-oxazol-2-carbonsäureamid, 5-Methyl-, 5-Ethyl-, 5-Propyl-, 5-Isopropyl-, 5-Butyl-, 5-Isobutyl-, 5-sec-Butyl-, 5-tert-Butyl- und 5-Phenyl-1,2,4-oxadiazol-3-carbonsäureamid, 2-Chlor-, 2-Methyl-, 2-Ethyl-, 2-Propyl-, 2-Isopropyl-, 2-Butyl-, 2-Isobutyl-, 2-sec-Butyl, 2-tert-Butyl-, 2-Trifluormethyl-, 2-Methoxy-, 2-Ethoxy-, 2-Propoxy- und 2-Isopropoxy-thiazol-5-carbonsäureamid, 2,4-Dichlor-thiazol-5-carbonsäureamid, 2-Methyl-4-chlor-thiazol-5-carbonsäureamid, 2-Methoxy-4-chlor-thiazol-5-carbonsäureamid, 4-Methyl-, 4-Ethyl-, 4-Propyl-, 4-Isopropyl-, 4-Butyl-, 4-Isobutyl-, 4-sec-Butyl- und 4-tert-Butyl-thiazol-2-carbonsäureamid, 5-Methyl-, 5-Ethyl-, 5-Propyl-, 5-Isopropyl-, 5-Butyl-, 5-Isobutyl-, 5-sec-Butyl-und 5-tert-Butyl-thiazol-2-carbonsäureamid.

Die Carbonsäureamide der Formel (II) sind bekannt und/oder können nach bekannten Verfahren hergestellt werden (vgl. RO-P 67530 zit. in Chem. Abstracts 95, 7258k; Helv. Chim. Acta 27 (1944), 1437 - 1438; Chem. Ber. 73 (1940), 1240 - 1252; 3. Prakt. Chem. 314 (1972), 447 - 454).

Die beim erfindungsgemäßen Verfahren (a) weiter als Ausgangsstoffe benötigten Sulfonylierungsmittel sind durch die Formel (III) allgemein definiert.

In Formel (III) haben n, A, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A, $R^2$ und $R^3$ angegeben wurden und

X steht vorzugsweise für Fluor, Chlor oder Brom, insbesondere für Chlor.

Als Beispiele für die Ausgangsstoffe der Formel (III) seien genannt:
2-Chlor-, 2,6-Dichlor-, 2,5-Dichlor-, 2-Fluor-, 2-Brom-, 2-Cyano-, 2-Nitro-, 2-Methyl-, 2-Chlor-6-methyl-, 2-Ethoxy-, 2-Trifluormethyl-, 2-Methoxy-, 2-Methylthio-, 2-Difluormethoxy-, 2-Trifluormethoxy-, 2-(2-Chlorethoxy)-, 2-Dimethylaminosulfonyl-, 2-Phenyl-, 2-Methoxycarbonyl-, 2-Ethoxycarbonyl-, 2-Propoxycarbonyl- und 2-Isopropoxycarbonyl-benzolsulfonsäurechlorid sowie (2-Chlor-phenyl)-, (2-Cyano-phenyl)-, (2-Methoxycarbonyl-phenyl)-und (2-Trifluormethoxy-phenyl)-methansulfonsäurechlorid.

Die Sulfonylierungsmittel der Formel (III) sind bekannt und/oder können nach bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalimetallhydride wie z.B. Natrium- und Kaliumhydrid, Erdalkalimetallhydride wie z.B. Calciumhydrid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen -20 °C und +100 °C, vorzugsweise bei Temperaturen zwischen 0 °C und +80 °C.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) als Ausgangsstoffe benötigten Carbonsäuren sind durch die Formel (IV) allgemein definiert.

In Formel (IV) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde.

Als Beispiele für die Ausgangsstoffe der Formel (IV) seien genannt:
5-Methyl-, 5-Ethyl-, 5-Propyl-, 5-Isopropyl-, 5-Butyl-, 5-Isobutyl-, 5-sec-Butyl- und 5-tert-Butyl-isoxazol-3-carbonsäure,2-Methyl-, 2-Ethyl-, 2-Propyl-, 2-Isopropyl-, 2-Butyl-, 2-Isobutyl-, 2-sec-Butyl- und 2-tert-Butyl-oxazol-4-carbonsäure, 5-Methyl-, 5-Ethyl-, 5-Propyl-, 5-Isopropyl- und 4,5-Dimethyl-1,3-oxazol-2-carbonsäure, 5-Methyl-, 5-Ethyl-, 5-Propyl-, 5-Isopropyl-, 5-Butyl-, 5-Isobutyl-, 5-sec-Butyl-, 5-tert-Butyl- und 5-Phenyl-1,2,4-oxadiazol-3-carbonsäure, 2-Chlor-, 2-Methyl-, 2-Ethyl-, 2-Propyl-, 2-Isopropyl-, 2-Butyl-, 2-Isobutyl-, 2-sec-Butyl, 2-tert-Butyl-, 2-Trifluormethyl-, 2-Methoxy-, 2-Ethoxy-, 2-Propoxy-, und 2-Isopropoxy-thiazol-5-carbonsäure, 2,4-Dichlor-thiazol-5-carbonsäure, 2-Methyl-4-chlor-thiazol-5-carbonsäure, 2-Methoxy-4-chlor-thiazol-5-carbonsäure, 4-Methyl-, 4-Ethyl-, 4-Propyl-, 4-Isopropyl-, 4-Butyl-, 4-Isobutyl-, 4-sec-Butyl- und 4-tert-Butyl-thiazol-2-carbonsäure, 5-Methyl-, 5-Ethyl-, 5-Propyl-, 5-Isopropyl-, 5-Butyl-, 5-Isobutyl-, 5-sec-Butyl- und 5-tert-Butyl-thiazol-2-carbonsäure.

Die Carbonsäuren der Formel (IV) sind bekannt und/oder können nach bekannten Verfahren hergestellt werden (vgl. GB-P 1245517; DE-OS 2940189; DE-OS 3013908; Helv. Chim. Acta 27 (1944), 1437-1438; Chem. Ber. 73 (1940), 1240-1252; J. Chem. Soc. 1946, 87-91; loc. cit, 1947, 96-102; Helv. Chim. Acta 29 (1946), 1957-1959; J. Prakt. Chem. 314 (1972), 447-454; Chem. Ber. 94 (1961), 757-761).

Anstatt der Carbonsäuren der Formel (IV) werden beim erfindungsgemäßen Verfahren (b) gegebenenfalls die davon abgeleiteten Carbonsäurechloride oder davon abgeleitete Alkylester (vorzugsweise Methylester oder Ethylester), Aralkylester (vorzugsweise Benzylester) oder Arylester (vorzugsweise Phenylester, in der Phenylgruppe gegebenenfalls durch Cyano, Nitro, Chlor, Fluor, Brom und/oder Methyl substituiert) als Ausgangsstoffe ("reaktionsfähige Derivate") eingesetzt.

Man erhält die Carbonsäurechloride aus den entsprechenden Carbonsäuren nach üblichen Methoden, beispielsweise durch Umsetzung mit üblichen "Chlorierungsmitteln" wie z.B. Phosgen, Oxalylchlorid oder Thionylchlorid, gegebenenfalls in Gegenwart von Reaktionshilfsmitteln, wie z.B. Pyridin, und gegebenenfalls in Gegenwart von Verdünnungsmitteln, wie z.B. Chloroform oder Tetrachlormethan, bei Temperaturen zwischen 0°C und 100°C.

Die entsprechenden Ester der Formel (IV) können aus den entsprechenden Carbonsäurechloriden und geeigneten Alkoholen oder Phenolen nach üblichen Methoden, im allgemeinen durch Umsetzung in Gegenwart eines Säureakzeptors, wie z.B. Triethylamin oder Pyridin, und in Gegenwart eines Verdünnungsmittels, wie z.B. Acetonitril, bei Temperaturen zwischen 0°C und 100°C erhalten werden.

Man kann die genannten Ester jedoch auch direkt aus den Carbonsäuren der Formel (IV) in Gegenwart von Kondensationshilfsmitteln, wie z.B. Dicyclohexylcarbodiimid, gegebenenfalls in Gegenwart von Reaktionshilfsmitteln, wie z.B. 4-Dimethylamino-pyridin, und gegebenenfalls in Gegenwart von Verdünnungsmitteln, wie z.B. Methylenchlorid oder Chloroform, bei Temperaturen zwischen 0°C und 100°C erhalten.

Die beim erfindungsgemäßen Verfahren (b) weiter als Ausgangsstoffe benötigten Aminosulfonylverbindungen sind durch die Formel (V) allgemein definiert.

In Formel (V) haben n, A, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, A, $R^2$ und $R^3$ angegeben wurden.

Als Beispiele für die Ausgangsstoffe der Formel (V) seien genannt:
2-Chlor-, 2,6-Dichlor-, 2,5-Dichlor-, 2-Fluor-, 2-Brom-, 2-Cyano-, 2-Nitro-, 2-Methyl-, 2-Chlor-6-methyl-, 2-Trifluormethyl-, 2-Methoxy-, 2-Ethoxy-, 2-Methylthio-, 2-Difluor-methoxy, 2-Trifluormethoxy-, 2-(2-Chlorethoxy)-, 2-Dimethylaminosulfonyl-, 2-Phenyl-, 2-Methoxycarbonyl-, 2-Ethoxycarbonyl-, 2-Propoxycarbonyl- und 2-Isopropoxycarbonyl-benzolsulfonsäureamid sowie (2-Chlor-phenyl)-, (2-Cyano-phenyl)-, (2-Methoxycarbonyl-phenyl)- und (2-Trifluormethoxy-phenyl)-methansulfonsäureamid.

Die Aminosulfonylverbindungen der Formel (V) sind bekannt und/oder können nach bekannten Verfahren hergestellt werden (vgl. J. Org. Chem. 33 (1968), 2104; J. Org. Chem. 25 (1960), 1824; DE-AS 2 308 262; EP-A 23 140, 23 141, 23 422, 35 893, 48 143, 51 466, 64 322, 70 041, 44 808 und 44 809; US-P 2 929 820, 4 282 242; 4 348 220 und 4 372 778 sowie Angew. Chem. 93 (1981), 151).

Anstatt der Aminosulfonylverbindungen der Formel (V) werden beim erfindungsgemäßen Verfahren (b) gegebenenfalls die davon abgeleiteten Sulfonylisocyanate ("reaktionsfähige Derivate") als Ausgangsstoffe eingesetzt. Diese Verbindungen sind bekannt und/oder können nach bekannten Verfahren hergestellt werden (vgl. J. Org. Chem. 31 (1966), 2658-2661; EP-A 7687; EP-A 46626; EP-A 21641; EP-A 23140; EP-A 23141; EP-A 70041; EP-A 23422, EP-A 64322; EP-A 34431; EP-A 35893; EP-A 51466, EP-A 44808; EP-A

173312; DE-OS 3132944; EP-A 87780; EP-A 271780).

Zur Reaktion mit den Sulfonylisocyanaten können außer den Carbonsäuren der Formel (IV) auch deren Alkalimetallsalze, insbesondere die Lithium-, Natrium- oder Kaliumsalze, eingesetzt werden.

Das erfindungsgemäße Verfahren (b) zur Herstellung der neuen Verbindungen der Formel (I) wird vorzugsweise unter Verwendung von Verdünnungsmitteln durchgeführt. Es kommen hierbei vorzugsweise die gleichen Verdünnungsmittel in Betracht, die oben für das erfindungsgemäße Verfahren (a) angegeben sind.

Das erfindungsgemäße Verfahren (b) wird gegebenenfalls in Gegenwart eines Reaktionshilfsmittels durchgeführt. Geeignete Reaktionshilfsmittel sind hierbei Verbindungen, mit denen Carbonsäuren in reaktionsfähige Intermediate überführt werden, die dann in situ mit nucleophilen Verbindungen, wie beispielsweise den Sulfonsäureamiden der Formel (V) zu entsprechenden Carbonsäurederivaten umgesetzt werden. Als Beispiele für derartige Reaktionshilfsmittel seien Carbonyldiimidazol und 2-Chlor-1-methyl-pyridiniumiodid genannt.

Für die Umsetzung der Carbonsäusrechloride und Ester gemäß Formel (IV) mit Aminosulfonylverbindungen der Formel (V) sind auch Säureakzeptoren, wie sie oben für das erfindungsgemäße Verfahren (a) angegeben sind, als Reaktionshilfsmittel geeignet.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt nach üblichen Methoden.

Aus den erfindungsgemäßen Verbindungen der allgemeinen Formel (I) können gegebenenfalls Salze hergestellt werden. Man erhält solche Salze in einfacher weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol, Methylenchlorid oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in Getreide sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate,

Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

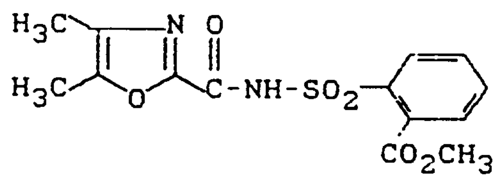

(Verfahren (a))

2,8 g (0,02 Mol) 4,5-Dimethyl-oxazol-2-carbonsäureamid und 3,4 g (0,06 Mol) Kaliumhydroxidpulver werden in 150 ml Dioxan 15 Minuten bei 40°C gerührt. Danach werden 50 ml Dioxan bei 40°C unter vermindertem Druck abdestilliert. Man kühlt die Suspension auf Raumtemperatur ab und tropft 7 g (0,027 Mol) 2-Chlorsulfonyl-benzoesäuremethylester, gelöst in 20 ml Dioxan, zu, Das Reaktionsgemisch wird 2 Tage bei Raumtemperatur gerührt, anschließend bei 40°C unter vermindertem Druck eingeengt und der Rückstand in 50 ml 1N-Salzsäure verrührt. Die Kristalle werden abgesaugt, mit Wasser neutral gewaschen und im Exsikkator getrocknet.

Man erhält 6,3 g (93% der Theorie) N-(2-Methoxycarbonylphenylsulfonyl)-4,5-dimethyl-oxazol-2-carbonsäureamid in Form farbloser Kristalle vom Schmelzpunkt 114°C.

### Beispiel 2

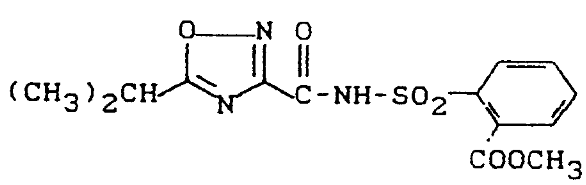

(Verfahren (b))

Eine Mischung aus 5,3 g (0,03 Mol) 5-Isopropyl-1,2,4-oxadiazol-3-carbonsäure-natriumsalz, 7,2 g (0,03 Mol) 2-Methoxycarbonyl-phenylsulfonylisocyanat, 0,15 g (0,0012 Mol) 4-Dimethylamino-pyridin und 150 ml Toluol wird 4 Stunden unter Rückfluß erhitzt. Dann wird im Wasserstrahlvakuum eingeengt, der Rückstand mit 150 ml 10%-iger Kaliumcarbonat-Lösung verrührt und abgesaugt. Der Nutschkuchen wird mit 100 ml

1N-Salzsäure verrührt und dreimal mit je 50 ml Methylenchlorid extrahiert. Die vereinigten organischen Extraktionslösungen werden mit Natriumsulfat getrocknet und filtriert. Das Filtrat wird im Wasserstrahlvakuum eingeengt, der Rückstand mit Diethylether verrührt und das kristalline Produkt durch Absaugen isoliert.

Man erhält 2,1 g (20% der Theorie) N-(2-Methoxycarbonylphenylsulfonyl)-5-isopropyl-1,2,4-oxadiazol-3-carbonsäureamid vom Schmelzpunkt 107°C.

Beispiel 3

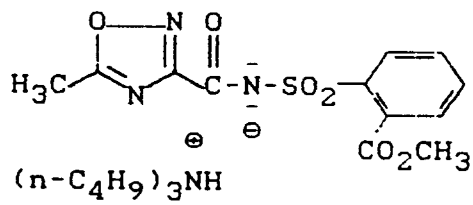

(Salzbildung)

3,25 g (0,01 Mol) N-(2-Methoxycarbonylphenyl-sulfonyl)-5-methyl-1,2,4-oxadiazol-3-carbonsäureamid und 1,85 g (0,01 Mol) Tributylamin werden in 50 ml Methanol bei Raumtemperatur gerührt, bis eine klare Lösung entstanden ist. Man destilliert das Methanol bei 40°C unter vermindertem Druck ab. Es verbleiben 5,0 g (98% der Theorie) des Tributylammoniumsalzes der eingesetzten Verbindung als farbloses Öl.

$^1$H-NMR ($CDCl_3$) δ-Werte:

0,92 (t, 3H); 1,30-1,39 (m, 6H); 1,59-1,67 (m, 6H); 2,59 (s, 3H); 3,08-3,12 (m, 6H); 3,88 (s, 3H); 7,43-7,51 (m, 3H); 8,17-8,20 (m, 1H)

Analog Beispiel 3 erhält man auch das entsprechende Natriumsalz (3a),

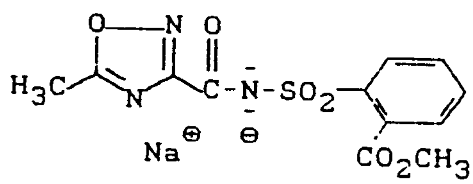

Fp.: >100°C (Zersetzung)

das entsprechende Triethylammoniumsalz (3b)

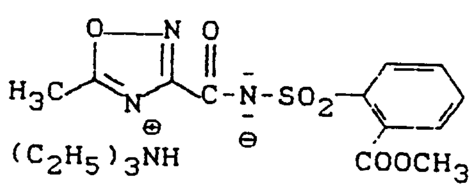

Fp.: 128°C

und das entsprechende Tris-(2-hydroxyethyl)-ammoniumsalz (3c)

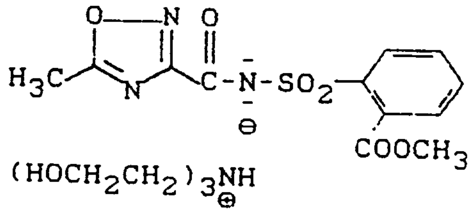

(Öl)

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

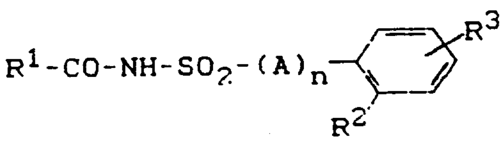 (I)

Tabelle 1: Beispiele für die Verbindungen der Formel (I)

| Bsp.-Nr. | $R^1$ | A | n | $R^2$ | (Position-) $R^3$ | Schmelzpunkt (°C) |
|---|---|---|---|---|---|---|
| 4 | 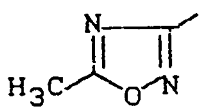 | - | 0 | $CO_2CH(CH_3)_2$ | - | 108 |
| 5 | 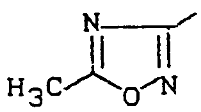 | $CH_2$ | 1 | $COOCH_3$ | - | 132 |
| 6 | 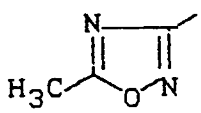 | $CH_2$ | 1 | $OCF_3$ | - | 214 |
| 7 | 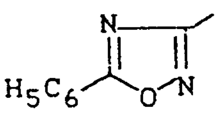 | - | 0 | $COOC_2H_5$ | - | 150 |
| 8 | 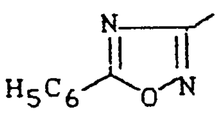 | - | 0 | $COOC_3H_7$ | - | 144 |
| 9 | 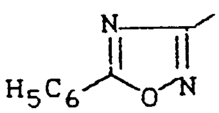 | - | 0 | $COOCH_3$ | - | 146 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 10 | 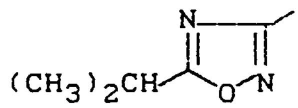 | - | 0 | $SOC_2H_5$ | - | 170 |
| 11 | 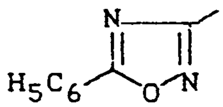 | - | 0 | $SOC_2H_5$ | - | 184 |
| 12 | 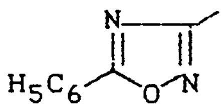 | - | 0 | $SO_2C_2H_5$ | - | 208 |
| 13 | 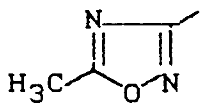 | O | 1 | $CH_3$ | - | 178 |
| 14 | 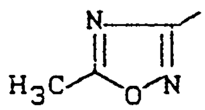 | - | 0 | $SCH_3$ | - | 130 |
| 15 | 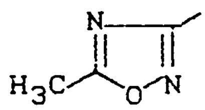 | - | 0 | $SC_3H_7$ | - | 121 |
| 16 | 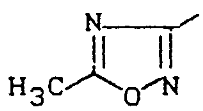 | - | 0 | $SOC_2H_5$ | - | 166 |
| 17 | 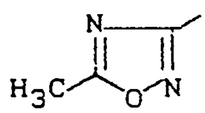 | - | 0 | $SO_2C_2H_5$ | - | 165 |
| 18 | 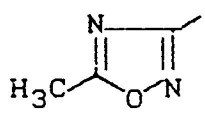 | - | 0 | $SOC_3H_7$ | - | 162 |
| 19 | 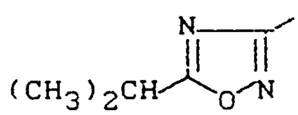 | - | 0 | $COOC_2H_5$ | - | 84 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 20 | 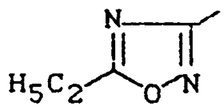 | - | 0 | $COOCH_3$ | - | 121 |
| 21 | 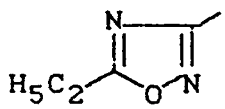 | - | 0 | $COOC_2H_5$ | - | 84 |
| 22 | 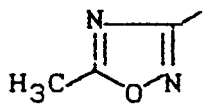 | - | 0 | $COOCH_2CH_2Cl$ | - | 93 |
| 23 | 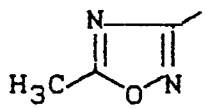 | - | 0 | $COOCH_2CH_2OCH_3$ | - | 129 |
| 24 | 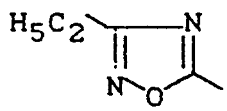 | - | 0 | $COOCH_3$ | - | 118 |
| 25 | 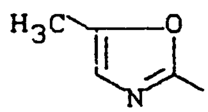 | - | 0 | $COOCH_3$ | - | 122 |
| 26 | 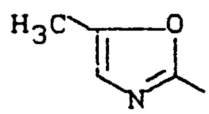 | - | 0 | $COOC_2H_5$ | - | 116 |
| 27 | 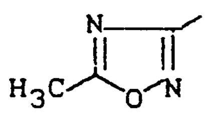 | - | 0 | $OCH_3$ | - | 161 |
| 28 | 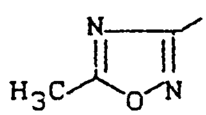 | - | 0 | $OC_2H_5$ | - | 165 |
| 29 | 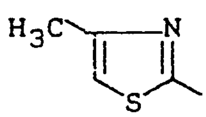 | - | 0 | $COOCH_3$ | - | 121 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 30 | 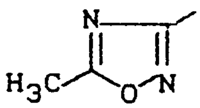 | - | 0 | COOH | - | 178 |
| 31 | 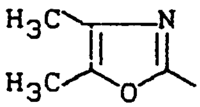 | - | 0 | $COOC_2H_5$ | - | 108 |
| 32 | 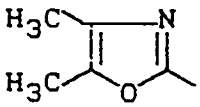 | - | 0 | $OCH_3$ | - | 174 |
| 33 | 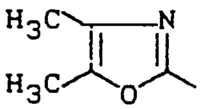 | - | 0 | $SC_2H_5$ | - | 124 |
| 34 | 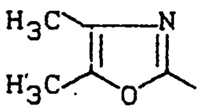 | - | 0 | $C_6H_5$ | - | 140 |
| 35 | 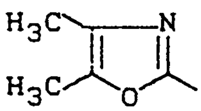 | - | 0 | $OCF_3$ | - | 110 |
| 36 | 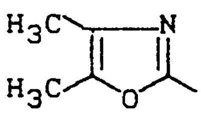 | - | 0 | F | - | 104 |
| 37 | 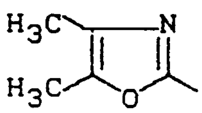 | - | 0 | Cl | (3-)Cl | 125 |
| 38 | 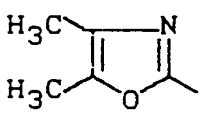 | - | 0 | Cl | (5-)$CH_3$ | 191 |
| 39 | 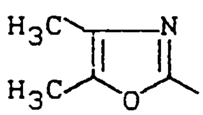 | - | 0 | $SC_3H_7$ | - | 121 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 40 | 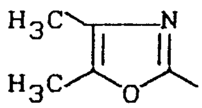 | - | 0 | $COOCH_2CH_2Cl$ | - | 115 |
| 41 | 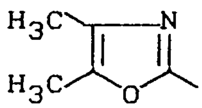 | $CH_2$ | 1 | $COOCH_3$ | - | 113 |
| 42 | 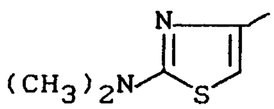 | - | 0 | $COOCH_3$ | - | |
| 43 | 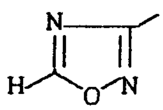 | - | 0 | $COOCH_3$ | - | 148 |
| 44 | 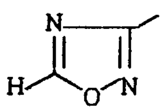 | - | 0 | $COOC_2H_5$ | - | |
| 45 | 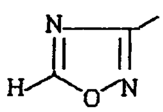 | - | 0 | $OCH_3$ | - | |
| 46 | 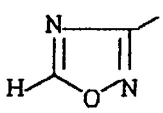 | - | 0 | $OC_2H_5$ | - | |
| 47 | 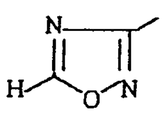 | - | 0 | $SC_2H_5$ | - | |
| 48 | 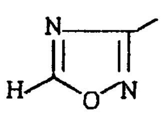 | - | 0 | $C_6H_5$ | - | |
| 49 | 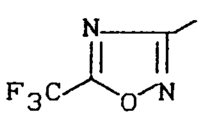 | - | 0 | $COOCH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 50 | 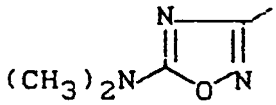 | - | 0 | $COOC_2H_5$ | - | |
| 51 | 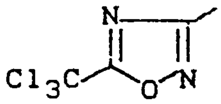 | - | 0 | $OC_2H_5$ | - | |
| 52 | 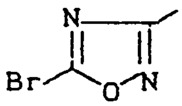 | - | 0 | $COOCH_3$ | - | |
| 53 | 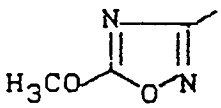 | - | 0 | $COOC_2H_5$ | - | |
| 54 | 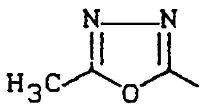 | - | 0 | $COOCH_3$ | - | |
| 55 | 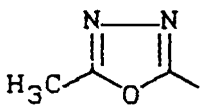 | - | 0 | $COOC_2H_5$ | - | |
| 56 | 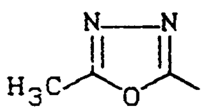 | - | 0 | $OC_2H_5$ | - | |
| 57 | 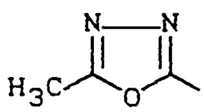 | - | 0 | Cl | - | 179° C (Zers.) |
| 58 | 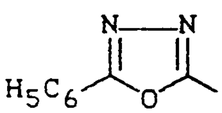 | - | 0 | Cl | - | 195° C (Zers.) |
| 59 | 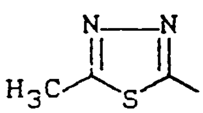 | - | 0 | $COOCH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 60 | 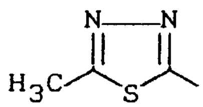 | - | 0 | $COOC_2H_5$ | - | |
| 61 | 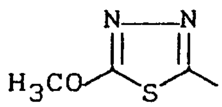 | - | 0 | $COOCH_3$ | - | |
| 62 | 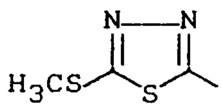 | - | 0 | $OC_2H_5$ | - | |
| 63 | 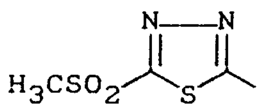 | - | 0 | $SC_2H_5$ | - | |
| 64 | 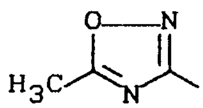 | - | 0 | Cl | (6)-Cl | |
| 65 | 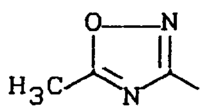 | - | 0 | $CO_2CH_3$ | (5)-Cl | |
| 66 | 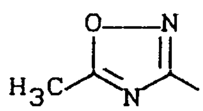 | - | 0 | $O-C_3H_7-n$ | - | 115 |
| 67 | 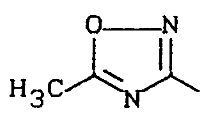 | - | 0 | $O-C_3H_7-i$ | - | 76 |
| 68 | 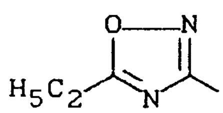 | - | 0 | Cl | (6)-Cl | |
| 69 | 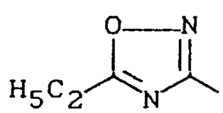 | - | 0 | $CO_2CH_3$ | (5)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 70 | 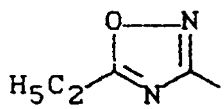 | - | 0 | $O-C_3H_{7-n}$ | - | |
| 71 | 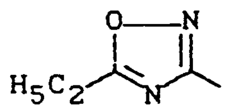 | - | 0 | $O-C_3H_{7-i}$ | - | |
| 72 | 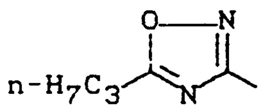 | $CH_2$ | 1 | $CO_2CH_3$ | - | |
| 73 | 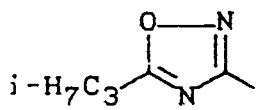 | - | 0 | $CO_2CH_3$ | - | |
| 74 | 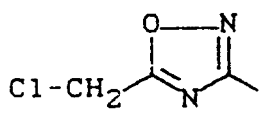 | - | 0 | $CO_2CH_3$ | - | |
| 75 | 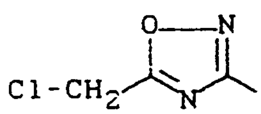 | - | 0 | $CO_2C_2H_5$ | - | |
| 76 | 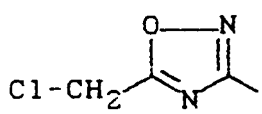 | - | 0 | Cl | - | |
| 77 | 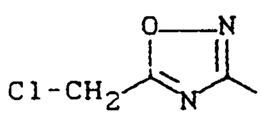 | - | 0 | Br | - | |
| 78 | 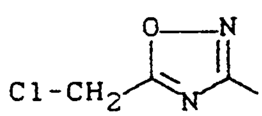 | - | 0 | F | - | |
| 79 | 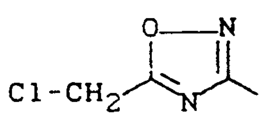 | - | 0 | $C_6H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 80 | 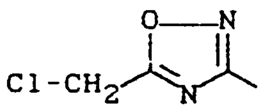 | - | 0 | $OCH_3$ | - | |
| 81 | 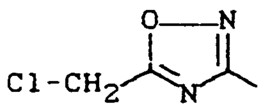 | - | 0 | $OC_2H_5$ | - | |
| 82 | 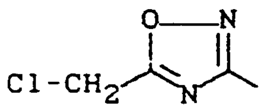 | - | 0 | $OC_3H_7$ | - | |
| 83 | 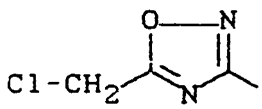 | - | 0 | $SCH_3$ | - | |
| 84 | 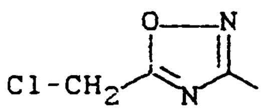 | - | 0 | $SC_2H_5$ | - | |
| 85 | 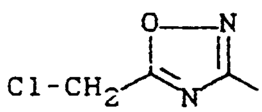 | - | 0 | $CO_2CH_3$ | (5)-Cl | |
| 86 | 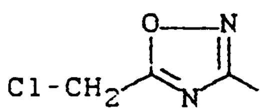 | - | 0 | $CO_2CH_2CH_2Cl$ | - | |
| 87 | 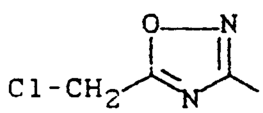 | - | 0 | Cl | (6)-Cl | |
| 88 | 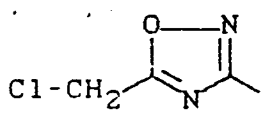 | - | 0 | Cl | (6)-$CH_3$ | |
| 89 | 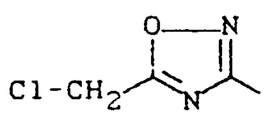 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 90 | 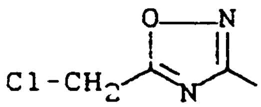 | - | 0 | $OCHF_2$ | - | |
| 91 | 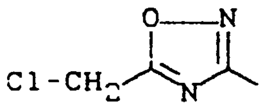 | $CH_2$ | 1 | $OCF_3$ | - | |
| 92 | 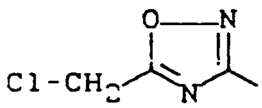 | O | 1 | $CH_3$ | - | |
| 93 | 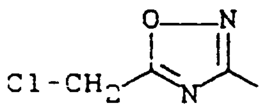 | - | 0 | $CH_3$ | - | |
| 94 | 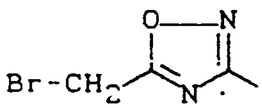 | - | 0 | $CO_2CH_3$ | - | |
| 95 | 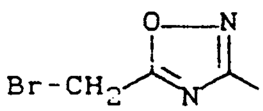 | - | 0 | $CO_2CH_3$ | (5)-Cl | |
| 96 | 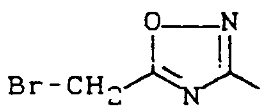 | - | 0 | $CO_2C_2H_5$ | - | |
| 97 | 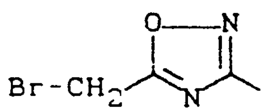 | - | 0 | Cl | - | |
| 98 | 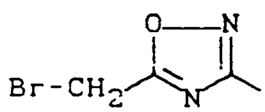 | - | 0 | $OCH_3$ | - | |
| 99 | 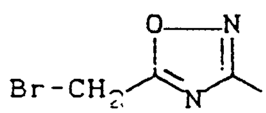 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 100 | 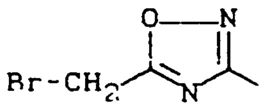 | - | 0 | $OC_3H_7$ | - | |
| 101 | 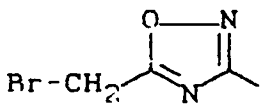 | - | 0 | $C_6H_5$ | - | |
| 102 | 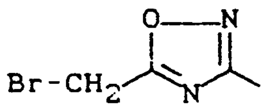 | - | 0 | Cl | (6)-Cl | |
| 103 | 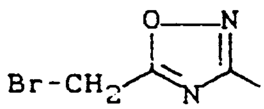 | - | 0 | Cl | (6)-$CH_3$ | |
| 104 | 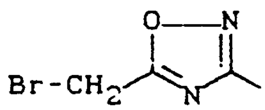 | - | 0 | $SCH_3$ | - | |
| 105 | 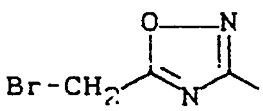 | - | 0 | $OCF_3$ | - | |
| 106 | 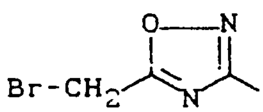 | - | 0 | $SC_2H_5$ | - | |
| 107 | 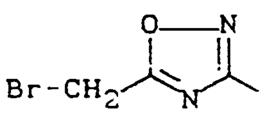 | - | 0 | $OCHF_2$ | - | |
| 108 | 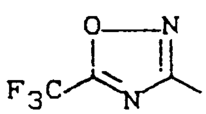 | $CH_2$ | 1 | $CO_2CH_3$ | - | |
| 109 | 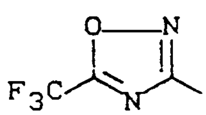 | - | 0 | $CO_2C_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 110 | 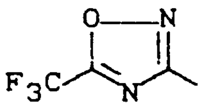 | - | 0 | $C_6H_5$ | - | |
| 111 | 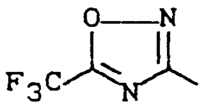 | - | 0 | Cl | - | |
| 112 | 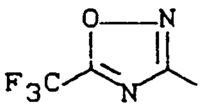 | - | 0 | Cl | (6)-Cl | |
| 113 | 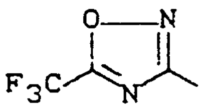 | - | 0 | $OCH_3$ | - | |
| 114 | 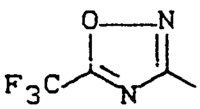 | - | 0 | $OC_2H_5$ | - | |
| 115 | 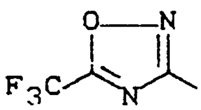 | - | 0 | $SO_2N(CH_3)_2$ | - | |
| 116 | 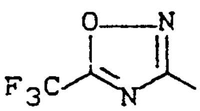 | - | 0 | $SCH_3$ | - | |
| 117 | 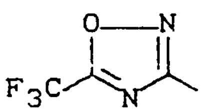 | - | 0 | $SC_2H_5$ | - | |
| 118 | 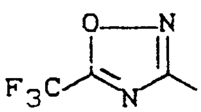 | - | 0 | $SOC_2H_5$ | - | |
| 119 | 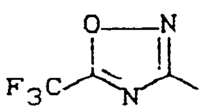 | - | 0 | $SO_2C_3H_7$-n | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 120 | 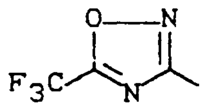 | - | 0 | $CH_3$ | - | |
| 121 | 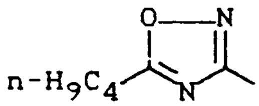 | - | 0 | $CO_2CH_3$ | - | |
| 122 | 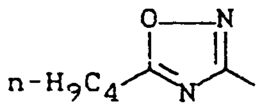 | - | 0 | $OCF_3$ | - | |
| 123 | 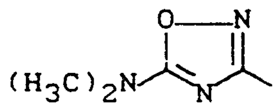 | - | 0 | $CO_2CH_3$ | - | |
| 124 | 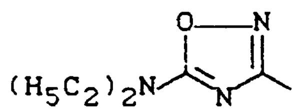 | - | 0 | $CO_2CH_3$ | - | |
| 125 | 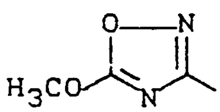 | - | 0 | $CO_2CH_3$ | - | |
| 126 | 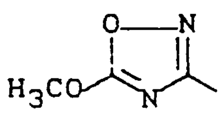 | $CH_2$ | 1 | $CO_2C_2H_5$ | - | |
| 127 | 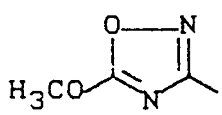 | - | 0 | $OCH_3$ | - | |
| 128 | 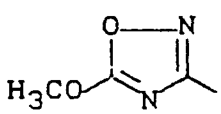 | - | 0 | $OC_2H_5$ | - | |
| 129 | 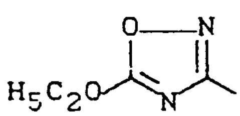 | - | 0 | $CO_2CH_3$ | (5)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 130 | 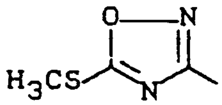 | - | 0 | $CO_2CH_3$ | - | |
| 131 | 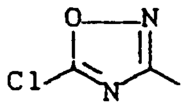 | - | 0 | $CO_2CH_3$ | - | |
| 132 | 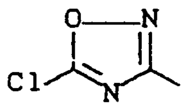 | - | 0 | $CO_2C_2H_5$ | - | |
| 133 | 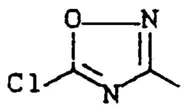 | - | 0 | $CO_2CH_3$ | (5)-Cl | |
| 134 | 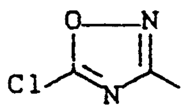 | $CH_2$ | 1 | $CO_2CH_3$ | - | |
| 135 | 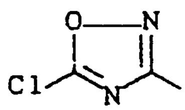 | - | 0 | $OCH_3$ | - | |
| 136 | 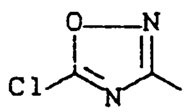 | - | 0 | $OC_2H_5$ | - | |
| 137 | 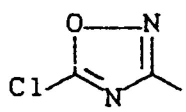 | - | 0 | $OC_3H_7$-n | - | |
| 138 | 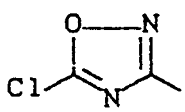 | - | 0 | $SCH_3$ | - | |
| 139 | 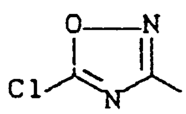 | - | 0 | $SC_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 140 | 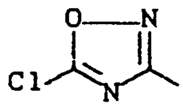 | - | 0 | $SOC_2H_5$ | - | |
| 141 | 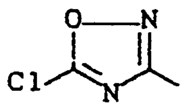 | - | 0 | $SO_2CH_3$ | - | |
| 142 | 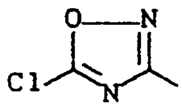 | - | 0 | Cl | - | |
| 143 | 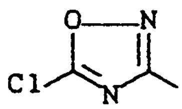 | - | 0 | Br | - | |
| 144 | 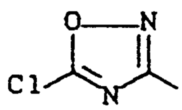 | - | 0 | Cl | (6)-Cl | |
| 145 | 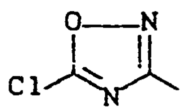 | - | 0 | Cl | (6)-$CH_3$ | |
| 146 | 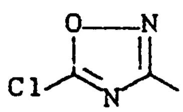 | - | 0 | $OCF_3$ | - | |
| 147 | 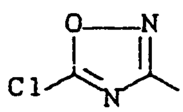 | - | 0 | $OCHF_2$ | - | |
| 148 | 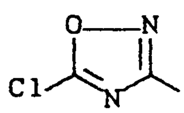 | - | 0 | CN | - | |
| 149 | 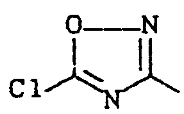 | $CH_2$ | 1 | $OCHF_2$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 150 | 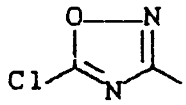 | - | 0 | $CH_3$ | - | |
| 151 | 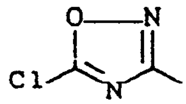 | - | 0 | $C_6H_5$ | - | |
| 152 | 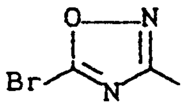 | $CH_2$ | 1 | $CO_2CH_3$ | - | |
| 153 | 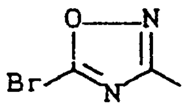 | - | 0 | $CO_2C_2H_5$ | - | |
| 154 | 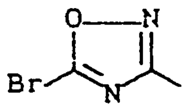 | - | 0 | $CO_2C_3H_7$-i | - | |
| 155 | 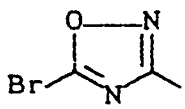 | - | 0 | $CO_2CH_2CH_2OCH_3$ | - | |
| 156 | 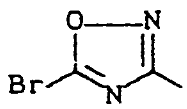 | - | 0 | $CO_2CH_3$ | (5)-Cl | |
| 157 | 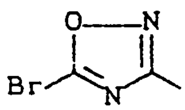 | - | 0 | $OCF_3$ | - | |
| 158 | 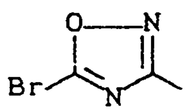 | - | 0 | $OCHF_2$ | - | |
| 159 | 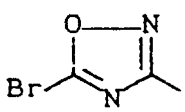 | - | 0 | $OCH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 160 | 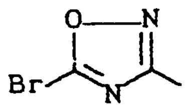 | - | 0 | $OC_2H_5$ | - | |
| 161 | 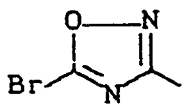 | - | 0 | $OC_3H_7$ | - | |
| 162 | 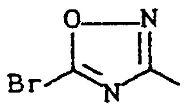 | - | 0 | Cl | - | |
| 163 | 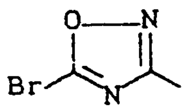 | $CH_2$ | 1 | Cl | - | |
| 164 | 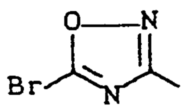 | - | 0 | Cl | (6)-Cl | |
| 165 | 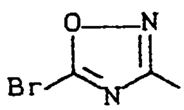 | - | 0 | Br | - | |
| 166 | 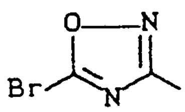 | - | 0 | F | - | |
| 167 | 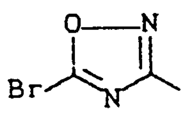 | - | 0 | $SCH_3$ | - | |
| 168 | 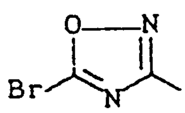 | - | 0 | $SC_2H_5$ | - | |
| 169 | 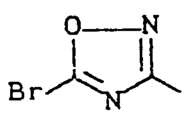 | - | 0 | $SC_3H_{7-n}$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 170 | 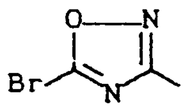 | - | 0 | $SOC_2H_5$ | - | |
| 171 | 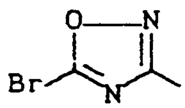 | - | 0 | $SO_2C_2H_5$ | - | |
| 172 | 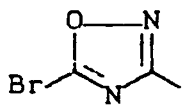 | - | 0 | 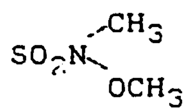 | - | |
| 173 | 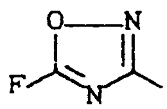 | - | 0 | $CO_2CH_3$ | - | |
| 174 | 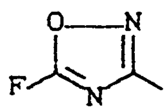 | - | 0 | Cl | - | |
| 175 | 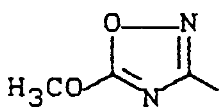 | - | 0 | Cl | - | |
| 176 | 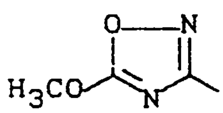 | - | 0 | $C_6H_5$ | - | |
| 177 | 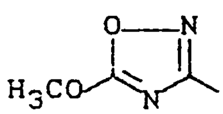 | - | 0 | $SCH_3$ | - | |
| 178 | 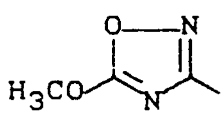 | - | 0 | $SO_2C_2H_5$ | - | |
| 179 | 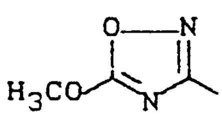 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 180 | 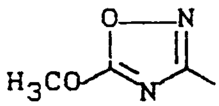 | - | 0 | $SO_2N(CH_3)_2$ | - | |
| 181 | 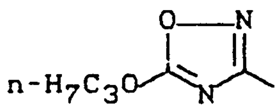 | - | 0 | $CO_2CH_3$ | - | |
| 182 | 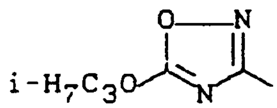 | - | 0 | $CO_2CH_3$ | - | |
| 183 | 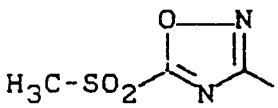 | - | 0 | $CO_2CH_3$ | - | |
| 184 | 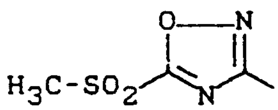 | - | 0 | $CO_2C_2H_5$ | - | |
| 185 | 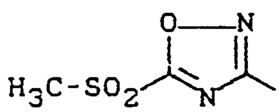 | - | 0 | $CO_2C_2H_5$ | (5)-Cl | |
| 186 | 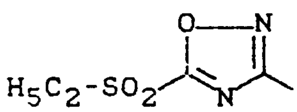 | - | 0 | $CO_2CH_3$ | - | |
| 187 | 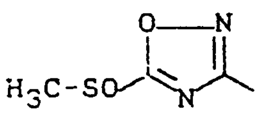 | - | 0 | $CO_2C_2H_5$ | - | |
| 188 | 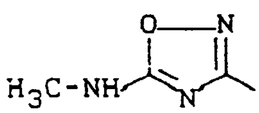 | - | 0 | $CO_2CH_3$ | - | |
| 189 | 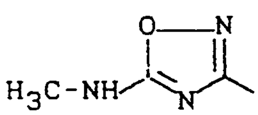 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 190 | 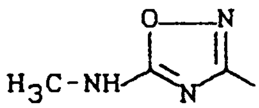 | - | 0 | Cl | - | |
| 191 | 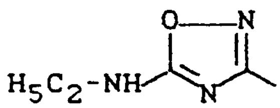 | - | 0 | Cl | (6)-Cl | |
| 192 | 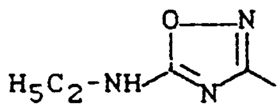 | - | 0 | Cl | (6)-$CH_3$ | |
| 193 | 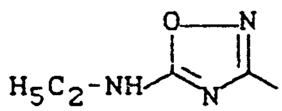 | - | 0 | $OC_2H_5$ | - | |
| 194 | 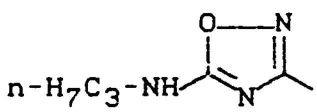 | - | 0 | $CO_2CH_3$ | - | |
| 195 | 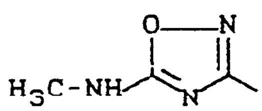 | - | 0 | $OCF_3$ | - | |
| 196 | 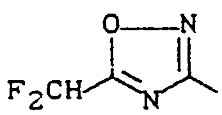 | - | 0 | $CO_2CH_3$ | - | |
| 197 | 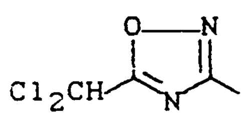 | - | 0 | $CO_2CH_3$ | - | |
| 198 | 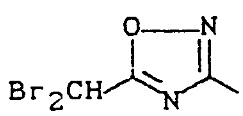 | - | 0 | $CO_2CH_3$ | - | |
| 199 | 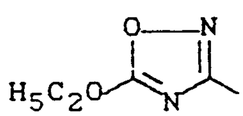 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 200 | 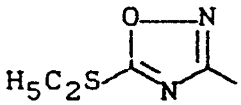 | - | 0 | $CO_2C_2H_5$ | - | |
| 201 | 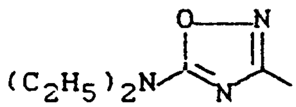 | - | 0 | $CO_2CH_3$ | - | |
| 202 | 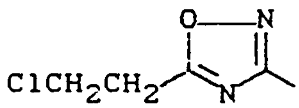 | - | 0 | $CO_2CH_3$ | - | |
| 203 | 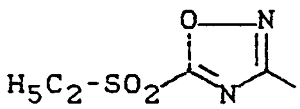 | - | 0 | $CO_2CH_3$ | - | |
| 204 | 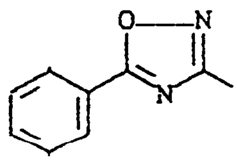 | - | 0 | $CO_2CH_3$ | (6-)Cl | |
| 205 | 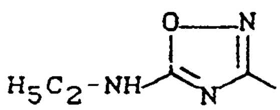 | - | 0 | $CO_2CH_3$ | - | |
| 206 | 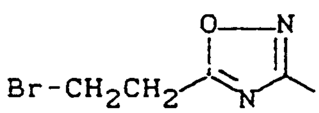 | - | 0 | $CO_2CH_3$ | - | |
| 207 | 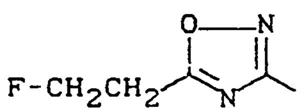 | - | 0 | $CO_2CH_3$ | - | |
| 208 | 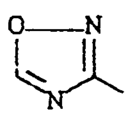 | $CH_2$ | 1 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 209 | 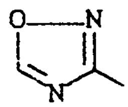 | $CH_2$ | 1 | $CO_2C_2H_5$ | - | |
| 210 | 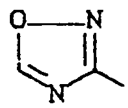 | - | 0 | $CO_2C_3H_{7-n}$ | - | |
| 211 | 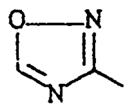 | - | 0 | $CO_2C_3H_{7-i}$ | - | |
| 212 | 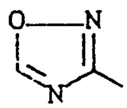 | $CH_2$ | 1 | $OCF_3$ | - | |
| 213 | 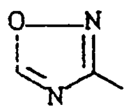 | - | 0 | $CO_2CH_3$ | (5)-Cl | |
| 214 | 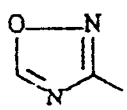 | - | 0 | Cl | - | |
| 215 | 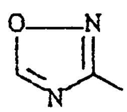 | - | 0 | Br | - | |
| 216 | 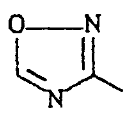 | - | 0 | F | - | |
| 217 | 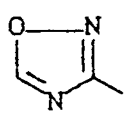 | - | 0 | Cl | (6)-Cl | |
| 218 | 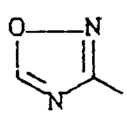 | - | 0 | Cl | (6)-$CH_3$ | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 219 | 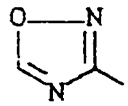 | $CH_2$ | 1 | $OCH_3$ | - | |
| 220 | 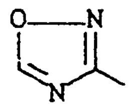 | $CH_2$ | 1 | $OC_2H_5$ | - | |
| 221 | 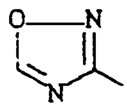 | - | 0 | $CF_3$ | - | |
| 222 | 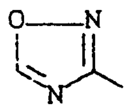 | - | 0 | $OCF_3$ | - | |
| 223 | 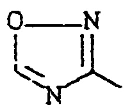 | - | 0 | $OCHF_2$ | - | |
| 224 | 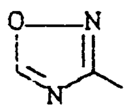 | - | 0 | $CH_3$ | - | |
| 225 | 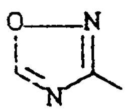 | - | 0 | $SO_2N(CH_3)_2$ | - | |
| 226 | 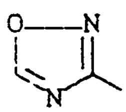 | - | 0 | $SCH_3$ | - | |
| 227 | 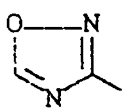 | - | 0 | $SOCH_3$ | - | |
| 228 | 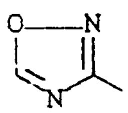 | - | 0 | $SO_2CH_3$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 229 | 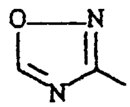 | - | 0 | Cl | (6-)F | |
| 230 | 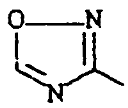 | - | 0 | $SOC_2H_5$ | - | |
| 231 | 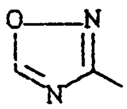 | - | 0 | $SO_2C_2H_5$ | - | |
| 232 | 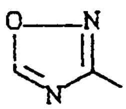 | - | 0 | $SC_3H_{7-n}$ | - | |
| 233 | 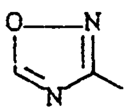 | - | 0 | $SOC_3H_{7-n}$ | - | |
| 234 | 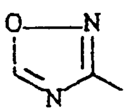 | - | 0 | $SO_2C_3H_{7-n}$ | - | |
| 235 | 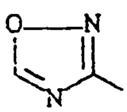 | $CH_2$ | 1 | $SO_2N(CH_3)_2$ | - | |
| 236 | 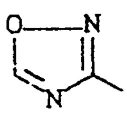 | - | 0 | $CH_3$<br>$SO_2N$<br>$OCH_3$ | - | |
| 237 | 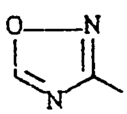 | $CH_2$ | 1 | $CH_3$ | - | |
| 238 | 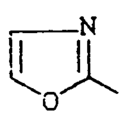 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 239 | 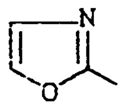 | - | 0 | $CO_2CH_3$ | (6)-Cl | |
| 240 | 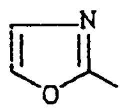 | - | 0 | $OCH_3$ | - | |
| 241 | 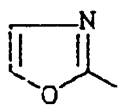 | - | 0 | $OC_2H_5$ | - | |
| 242 | 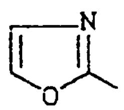 | - | 0 | Cl | - | |
| 243 | 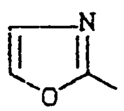 | - | 0 | Cl | (6)-Cl | |
| 244 | 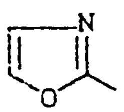 | - | 0 | $SCH_3$ | - | |
| 245 | 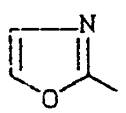 | - | 0 | $SC_3H_7$ | - | |
| 246 | 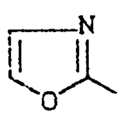 | - | 0 | $SOC_2H_5$ | - | |
| 247 | 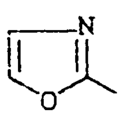 | - | 0 | $SO_2C_2H_5$ | - | |
| 248 | 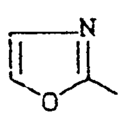 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 249 | 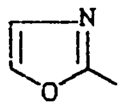 | - | 0 | $C_6H_5$ | - | |
| 250 | 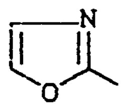 | - | 0 | $OCF_3$ | - | |
| 251 | 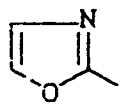 | - | 0 | $OCHF_2$ | - | |
| 252 | 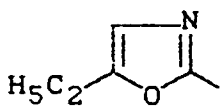 | - | 0 | $CO_2CH_3$ | - | |
| 253 | 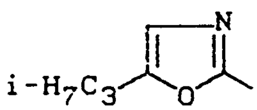 | - | 0 | $CO_2C_2H_5$ | - | |
| 254 | 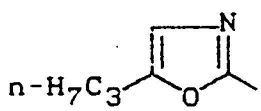 | - | 0 | $CO_2CH_3$ | - | |
| 255 | 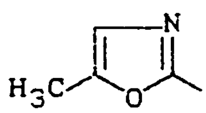 | - | 0 | $SCH_3$ | - | |
| 256 | 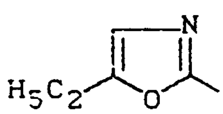 | - | 0 | $SC_2H_5$ | - | |
| 257 | 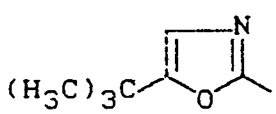 | - | 0 | $SOC_2H_5$ | - | |
| 258 | 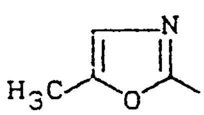 | - | 0 | $SO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 259 | 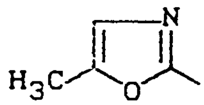 | - | 0 | $C_6H_5$ | - | |
| 260 | 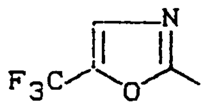 | - | 0 | $CO_2CH_3$ | - | |
| 261 | 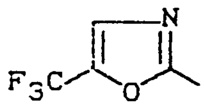 | - | 0 | $OCH_3$ | - | |
| 262 | 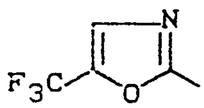 | - | 0 | Cl | - | |
| 263 | 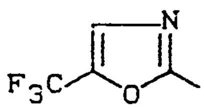 | - | 0 | OEt | - | |
| 264 | 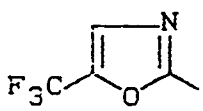 | - | 0 | $OCF_3$ | - | |
| 265 | 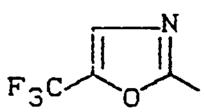 | - | 0 | $SC_2H_5$ | - | |
| 266 | 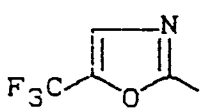 | - | 0 | Cl | (6)-$CH_3$ | |
| 267 | 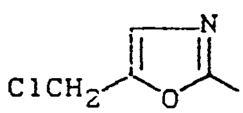 | - | 0 | $CO_2CH_3$ | - | |
| 268 | 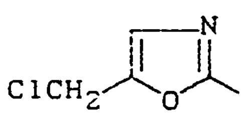 | - | 0 | $CO_2C_3H_{7}$-i | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 269 | 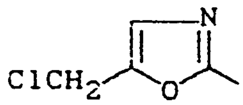 | - | 0 | $CO_2CH_3$ | (5)-Cl | |
| 270 | 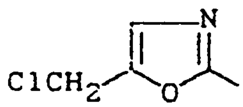 | - | 0 | $OCH_3$ | - | |
| 271 | 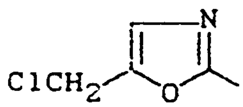 | - | 0 | $SC_2H_5$ | - | |
| 272 | 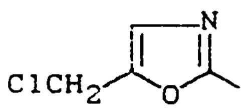 | $CH_2$ | 1 | $CO_2CH_3$ | - | |
| 273 | 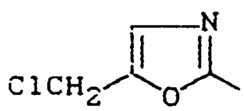 | - | 0 | $C_6H_5$ | - | |
| 274 | 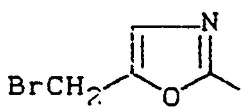 | - | 0 | $CO_2CH_3$ | - | |
| 275 | 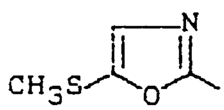 | - | 0 | $CO_2CH_3$ | - | |
| 276 | 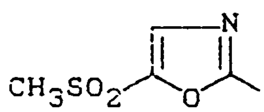 | - | 0 | $CO_2C_2H_5$ | - | |
| 277 | 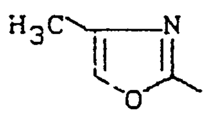 | - | 0 | $CO_2CH_3$ | - | |
| 278 | 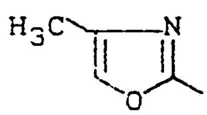 | - | 0 | $CO_2CH_3$ | (6-)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 279 | 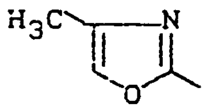 | - | 0 | $OCH_3$ | - | |
| 280 | 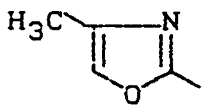 | - | 0 | $OC_2H_5$ | - | |
| 281 | 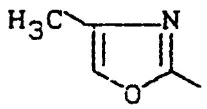 | - | 0 | Cl | - | |
| 282 | 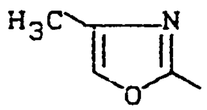 | - | 0 | Cl | (6)-Cl | |
| 283 | 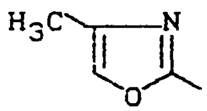 | - | 0 | $SO_2N(CH_3)_2$ | - | |
| 284 | 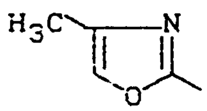 | - | 0 | $SCH_3$ | - | |
| 285 | 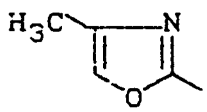 | - | 0 | $SC_2H_5$ | - | |
| 286 | 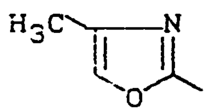 | - | 0 | $OCHF_2$ | - | |
| 287 | 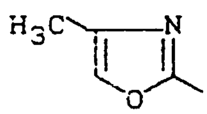 | - | 0 | $SO_2CH_3$ | - | |
| 288 | 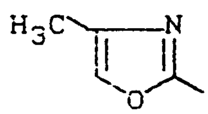 | - | 0 | $C_6H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 289 | 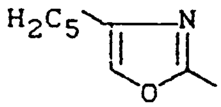 | - | 0 | $CO_2CH_3$ | - | |
| 290 | 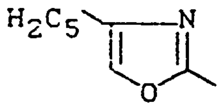 | - | 0 | $CO_2C_2H_5$ | - | |
| 291 | 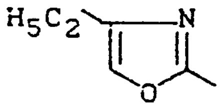 | - | 0 | F | - | |
| 292 | 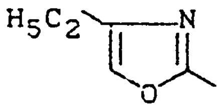 | - | 0 | Cl | - | |
| 293 | 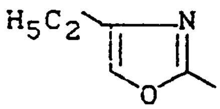 | - | 0 | $OC_2H_5$ | - | |
| 294 | 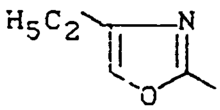 | - | 0 | $OCH_3$ | - | |
| 295 | 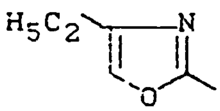 | - | 0 | $OC_3H_7$ | - | |
| 296 | 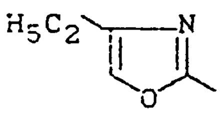 | - | 0 | $SCH_3$ | - | |
| 297 | 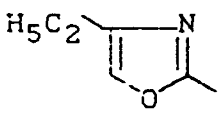 | - | 0 | $SO_2C_2H_5$ | - | |
| 298 | 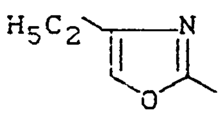 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 299 | 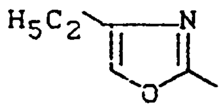 | - | 0 | $OCHF_2$ | - | |
| 300 | 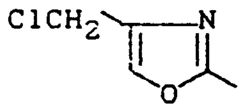 | - | 0 | $CO_2CH_3$ | - | |
| 301 | 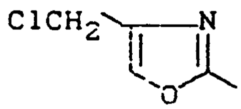 | - | 0 | $CF_3$ | - | |
| 302 | 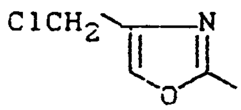 | - | 0 | Cl | (6)-Cl | |
| 303 | 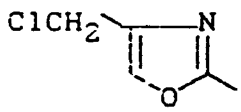 | - | 0 | $OCH_3$ | - | |
| 304 | 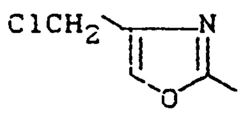 | - | 0 | $OC_2H_5$ | - | |
| 305 | 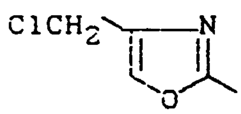 | - | 0 | $SCH_3$ | - | |
| 306 | 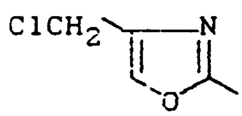 | - | 0 | $SO-C_2H_5$ | - | |
| 307 | 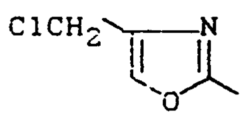 | - | 0 | $SO_2C_3H_7$-n | - | |
| 308 | 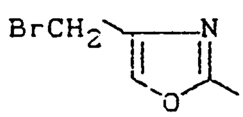 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 309 | 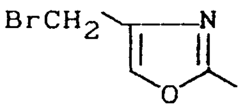 | - | 0 | $C_6H_5$ | - | |
| 310 | 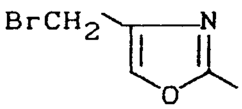 | - | 0 | $CO_2CH_2CH_2Cl$ | - | |
| 311 | 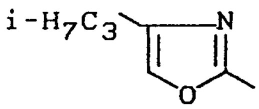 | - | 0 | $CO_2CH_3$ | - | |
| 312 | 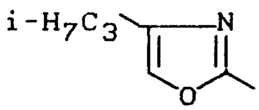 | - | 0 | $CO_2C_3H_7$-n | - | |
| 313 | 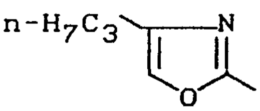 | - | 0 | $CO_2CH_3$ | - | |
| 314 | 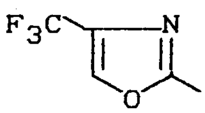 | - | 0 | $CO_2CH_3$ | - | |
| 315 | 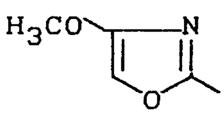 | - | 0 | $CO_2CH_3$ | - | |
| 316 | 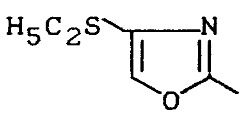 | - | 0 | $CO_2CH_3$ | - | |
| 317 | 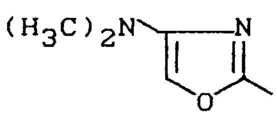 | - | 0 | $CO_2C_2H_5$ | - | |
| 318 | 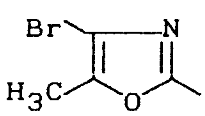 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 319 | 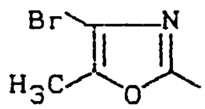 | - | 0 | $OC_2H_5$ | - | |
| 320 | 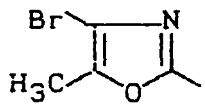 | - | 0 | $OCF_3$ | - | |
| 321 | 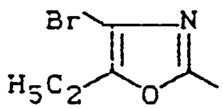 | - | 0 | 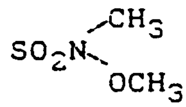 | - | |
| 322 | 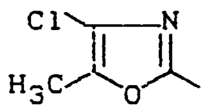 | - | 0 | $CO_2CH_3$ | - | |
| 323 | 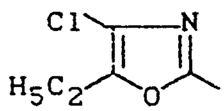 | - | 0 | $CO_2CH_3$ | (5)-Cl | |
| 324 | 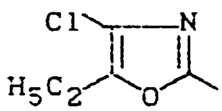 | - | 0 | $SC_2H_5$ | - | |
| 325 | 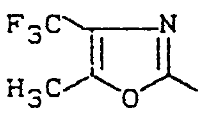 | - | 0 | $CO_2CH_3$ | - | |
| 326 | 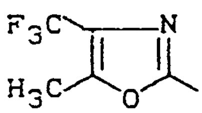 | - | 0 | Br | - | |
| 327 | 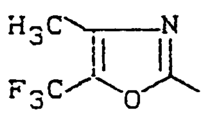 | - | 0 | $OC_2H_5$ | - | |
| 328 | 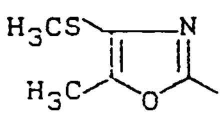 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 329 | 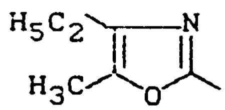 | - | 0 | $CO_2CH_3$ | (6)-Cl | |
| 330 | 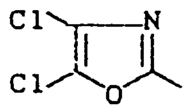 | - | 0 | $CO_2CH_3$ | - | |
| 331 | 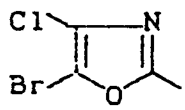 | - | 0 | $CO_2CH_3$ | - | |
| 332 | 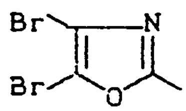 | - | 0 | $CO_2C_2H_5$ | - | |
| 333 | 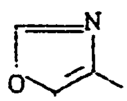 | - | 0 | $CO_2CH_3$ | - | |
| 334 | 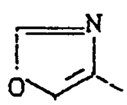 | - | 0 | $OCHF_2$ | - | |
| 335 | 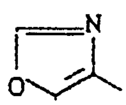 | - | 0 | Cl | - | |
| 336 | 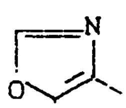 | - | 0 | $OC_2H_5$ | - | |
| 337 | 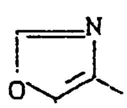 | - | 0 | $SCH_3$ | - | |
| 338 | 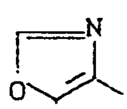 | - | 0 | $SO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 339 | 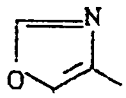 | - | 0 | $CF_3$ | - | |
| 340 | 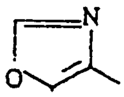 | - | 0 | $C_6H_5$ | - | |
| 341 | 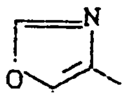 | - | 0 | $CO_2CH_3$ | (5)-Cl | |
| 342 | 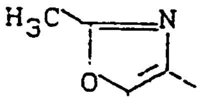 | - | 0 | $CO_2CH_3$ | (5)-Cl | |
| 343 | 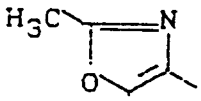 | - | 0 | $CO_2C_2H_5$ | - | |
| 344 | 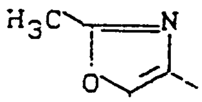 | - | 0 | $OCH_3$ | - | |
| 345 | 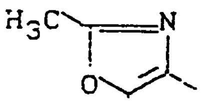 | - | 0 | $SC_2H_5$ | - | |
| 346 | 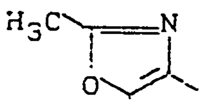 | - | 0 | $SO_2C_2H_5$ | - | |
| 347 | 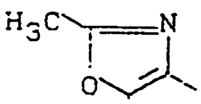 | - | 0 | $OC_2H_5$ | - | |
| 348 | 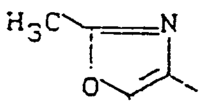 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 349 | 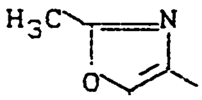 | - | 0 | $C_6H_5$ | - | |
| 350 | 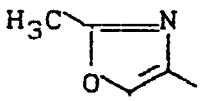 | - | 0 | Cl | - | |
| 351 | 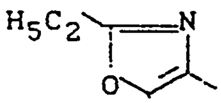 | - | 0 | $CO_2CH_3$ | - | |
| 352 | 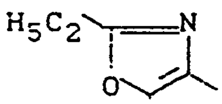 | - | 0 | $SCH_3$ | - | |
| 353 | 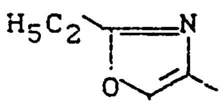 | - | 0 | $SO_2C_3H_7$-n | - | |
| 354 | 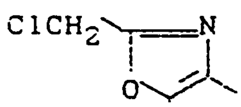 | - | 0 | $CO_2CH_3$ | - | |
| 355 | 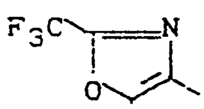 | - | 0 | $CO_2CH_3$ | - | |
| 356 | 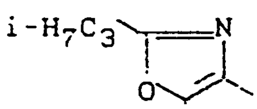 | - | 0 | $OCH_3$ | - | |
| 357 | 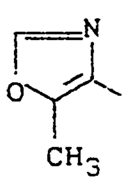 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 358 | 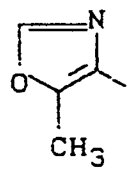 | - | 0 | $CO_2C_2H_5$ | - | |
| 359 | 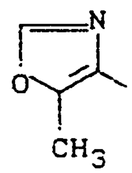 | - | 0 | $OC_3H_7$-i | - | |
| 360 | 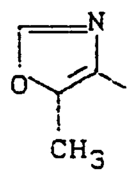 | - | 0 | $SCH_3$ | - | |
| 361 | 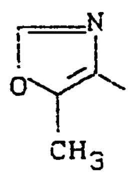 | - | 0 | $SO_2C_3H_7$-n | - | |
| 362 | 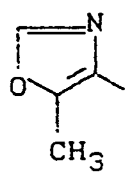 | - | 0 | $SO_2N(CH_3)_2$ | - | |
| 363 | 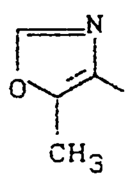 | - | 0 | Cl | - | |
| 364 | 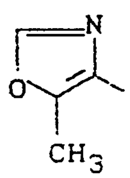 | - | 0 | Cl | (6)-$CH_3$ | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 365 | 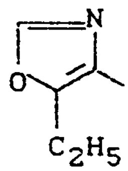 | - | 0 | $CO_2CH_3$ | - | |
| 366 | 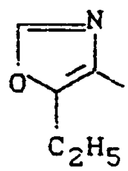 | - | 0 | $CO_2CH_3$ | (6)-Cl | |
| 367 | 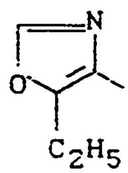 | - | 0 | $OCF_3$ | - | |
| 368 | 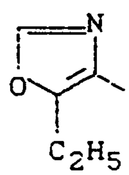 | - | 0 | $C_6H_5$ | - | |
| 369 | 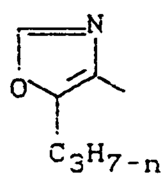 | - | 0 | $CO_2CH_3$ | - | |
| 370 | 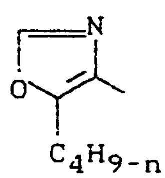 | - | 0 | $CO_2C_2H_5$ | - | |
| 371 | 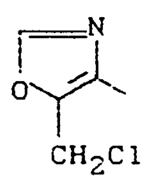 | - | 0 | $CO_2CH_3$ | (5)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 372 | 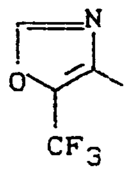 | - | 0 | $CO_2CH_2CH_2Cl$ | - | |
| 373 | 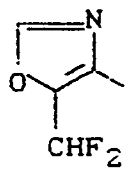 | - | 0 | $OC_2H_5$ | - | |
| 374 | 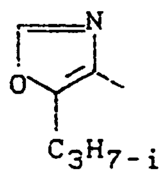 | - | 0 | $SOC_2H_5$ | - | |
| 375 | 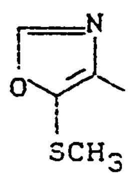 | - | 0 | $CO_2CH_3$ | - | |
| 376 | 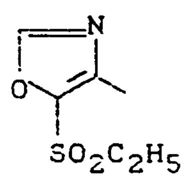 | - | 0 | $CO_2CH_3$ | - | |
| 377 | 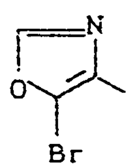 | - | 0 | $CO_2C_2H_5$ | - | |
| 378 | 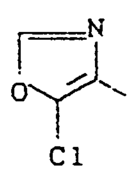 | - | 0 | $CO_2CH_3$ | - | |

Tabelle 1 - Fortsetzung

| Bsp.-Nr. | $R^1$ | A | n | $R^2$ | (Posi- tion-) $R^3$ | Schmelz- punkt (°C) |
|---|---|---|---|---|---|---|
| 379 | 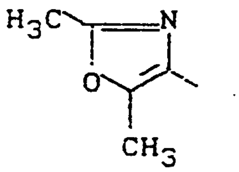 | - | 0 | $CO_2CH_3$ | - | |
| 380 | 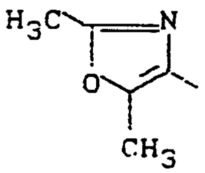 | - | 0 | $OC_2H_5$ | - | |
| 381 | 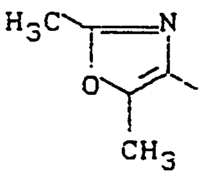 | - | 0 | $OCH_2CH_2Cl$ | - | |
| 382 | 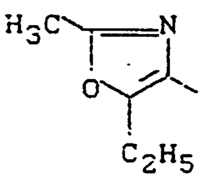 | - | 0 | $CO_2CH_3$ | - | |
| 383 | 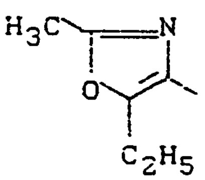 | - | 0 | $SO_2C_2H_5$ | - | |
| 384 | 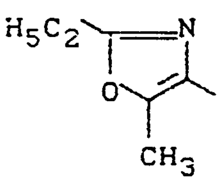 | - | 0 | Cl | - | |
| 385 | 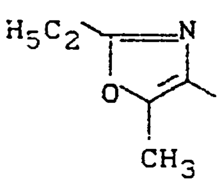 | - | 0 | Cl | (6)-Cl | |

<u>Tabelle 1</u> - Fortsetzung

| Bsp.-Nr. | $R^1$ | A | n | $R^2$ | (Position-) $R^3$ | Schmelzpunkt (°C) |
|---|---|---|---|---|---|---|
| 386 | 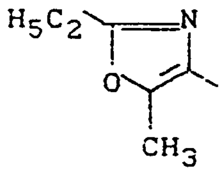 | - | 0 | $OCF_3$ | - | |
| 387 | 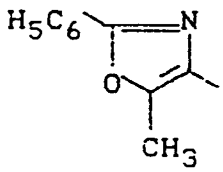 | - | 0 | $CO_2CH_3$ | - | |
| 388 | 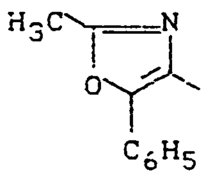 | - | 0 | $CO_2CH_3$ | - | |
| 389 | 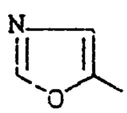 | - | 0 | $CO_2CH_3$ | - | |
| 390 | 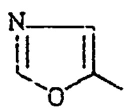 | - | 0 | $CF_3$ | - | |
| 391 | 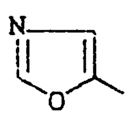 | - | 0 | $OC_3H_7$ | - | |
| 392 | 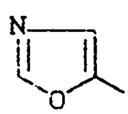 | - | 0 | $SCH_3$ | - | |
| 393 | 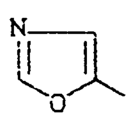 | - | 0 | $SO_2C_3H_{7-i}$ | - | |
| 394 | 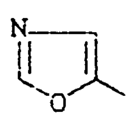 | - | 0 | Cl | (6)-$CH_3$ | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 395 | 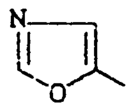 | - | 0 | $C_6H_5$ | - | |
| 396 | 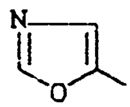 | - | 0 | $OCF_3$ | - | |
| 397 | 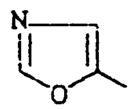 | - | 0 | $CO_2CH_3$ | (5)-Cl | |
| 398 | 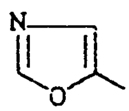 | - | 0 | F | - | |
| 399 | 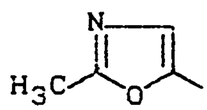 | - | 0 | $CO_2CH_3$ | - | |
| 400 | 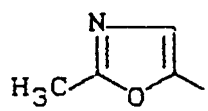 | - | 0 | Br | - | |
| 401 | 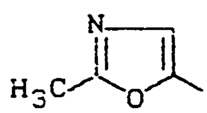 | - | 0 | $OC_3H_{7-i}$ | - | |
| 402 | 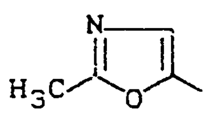 | - | 0 | $SC_3H_{7-i}$ | - | |
| 403 | 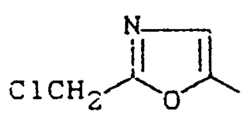 | - | 0 | $CO_2CH_3$ | - | |
| 404 | 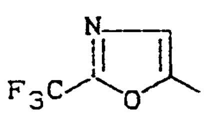 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 405 | 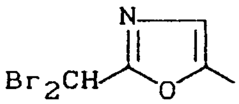 | - | 0 | $CO_2C_2H_5$ | - | |
| 406 | 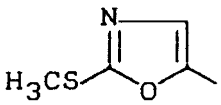 | - | 0 | $SO_2C_2H_5$ | - | |
| 407 | 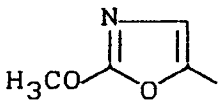 | - | 0 | $CO_2CH_3$ | - | |
| 408 | 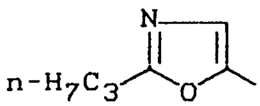 | - | 0 | $C_6H_5$ | - | |
| 409 | 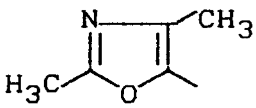 | - | 0 | $CO_2CH_3$ | - | 195 |
| 410 | 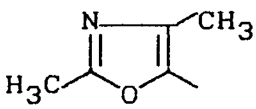 | - | 0 | $OC_2H_5$ | - | |
| 411 | 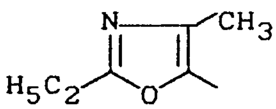 | - | 0 | $OCF_3$ | - | |
| 412 | 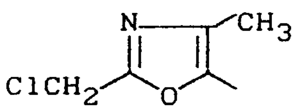 | - | 0 | $OCHF_2$ | - | |
| 413 | 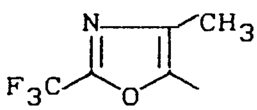 | - | 0 | $C_6H_5$ | - | |
| 414 | 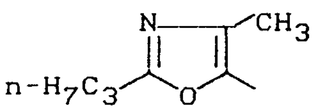 | - | 0 | $CO_2CH_3$ | (5)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 415 | 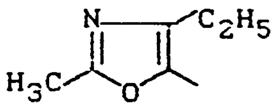 | - | 0 | $CH_3$ | - | |
| 416 | 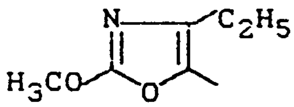 | - | 0 | $SCH_3$ | - | |
| 417 | 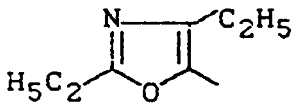 | - | 0 | $SO_2C_2H_5$ | - | |
| 418 | 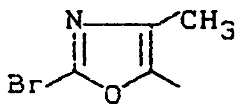 | - | 0 | $SO_2N(CH_3)_2$ | - | |
| 419 | 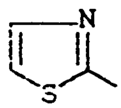 | - | 0 | $CO_2CH_3$ | - | |
| 420 | 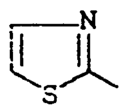 | - | 0 | $CO_2C_3H_7$-n | - | |
| 421 | 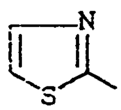 | - | 0 | Cl | (6)-$CH_3$ | |
| 422 | 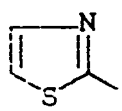 | - | 0 | $OCH_3$ | - | |
| 423 | 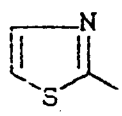 | - | 0 | $OC_2H_5$ | - | |
| 424 | 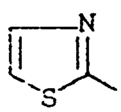 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 425 | 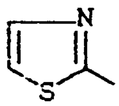 | - | 0 | $OCHF_2$ | - | |
| 426 | 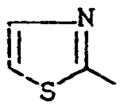 | - | 0 | $SCH_3$ | - | |
| 427 | 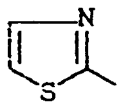 | - | 0 | $SC_2H_5$ | - | |
| 428 | 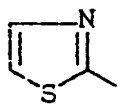 | - | 0 | $SOC_3H_7$ | - | |
| 429 | 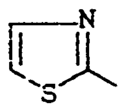 | - | 0 | $SOC_2H_5$ | - | |
| 430 | 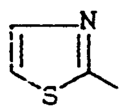 | - | 0 | $SO_2CH_3$ | - | |
| 431 | 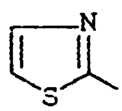 | - | 0 | Br | - | |
| 432 | 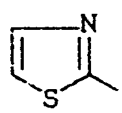 | - | 0 | $CF_3$ | - | |
| 433 | 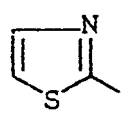 | NH | 1 | Cl | - | |
| 434 | 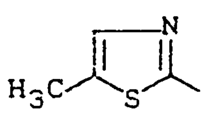 | - | 0 | $CO_2CH_3$ | (5)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 435 | 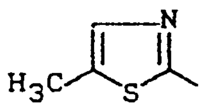 | - | 0 | Cl | (6)-Cl | |
| 436 | 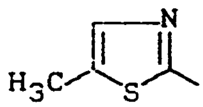 | - | 0 | $OC_3H_{7-n}$ | - | |
| 437 | 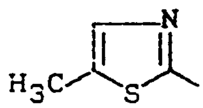 | - | 0 | $OCF_3$ | - | |
| 438 | 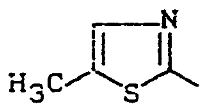 | - | 0 | $SC_2H_5$ | - | |
| 439 | 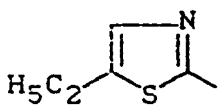 | - | 0 | $CO_2CH_3$ | - | |
| 440 | 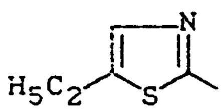 | - | 0 | $CO_2C_3H_{7-n}$ | - | |
| 441 | 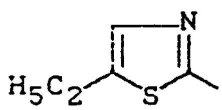 | - | 0 | $CO_2CH_3$ | (6)-Cl | |
| 442 | 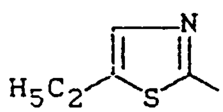 | - | 0 | $CO_2CH_3$ | (5)-Cl | |
| 443 | 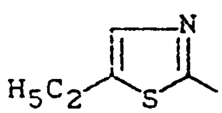 | - | 0 | $OCF_3$ | - | |
| 444 | 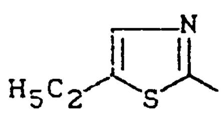 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 445 | 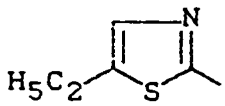 | - | 0 | $SC_2H_5$ | - | |
| 446 | 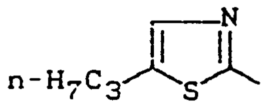 | - | 0 | $CO_2CH_3$ | - | |
| 447 | 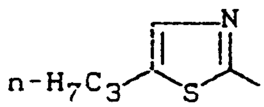 | - | 0 | Cl | (6)-$CH_3$ | |
| 448 | 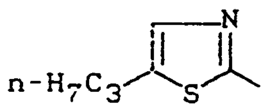 | - | 0 | $SO_2C_2H_5$ | - | |
| 449 | 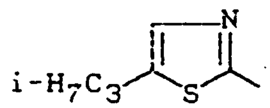 | - | 0 | $CO_2C_2H_5$ | - | |
| 450 | 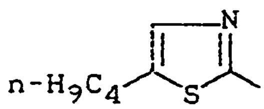 | - | 0 | $CO_2CH_3$ | - | |
| 451 | 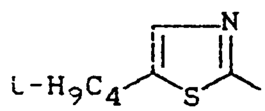 | - | 0 | $CO_2CH_3$ | (5)-Cl | |
| 452 | 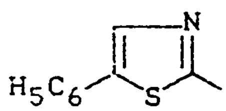 | - | 0 | $CO_2CH_3$ | - | |
| 453 | 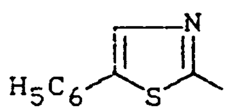 | - | 0 | $OCH_3$ | - | |
| 454 | 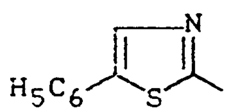 | - | 0 | $SO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 455 | 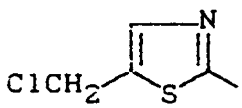 | - | 0 | $CO_2CH_3$ | - | |
| 456 | 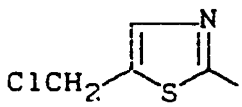 | - | 0 | $C_6H_5$ | - | |
| 457 | 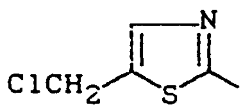 | - | 0 | $SC_3H_7$-n | - | |
| 458 | 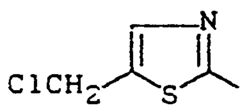 | - | 0 | Br | - | |
| 459 | 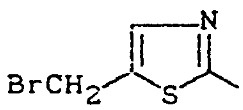 | - | 0 | $CO_2CH_3$ | - | |
| 460 | 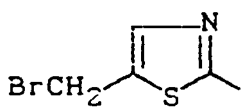 | $CH_2$ | 1 | $CO_2CH_3$ | - | |
| 461 | 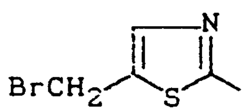 | - | 0 | $OCF_3$ | - | |
| 462 | 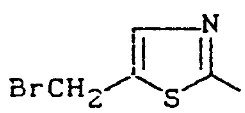 | - | 0 | $CF_3$ | - | |
| 463 | 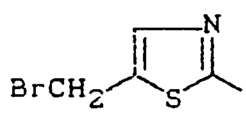 | - | 0 | $SC_3H_7$-i | - | |
| 464 | 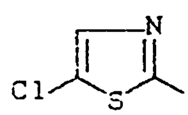 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 465 | 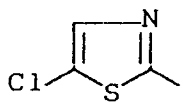 | - | 0 | $CH_3$ | - | |
| 466 | 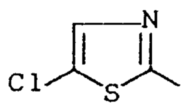 | 0 | 1 | $CH_3$ | - | |
| 467 | 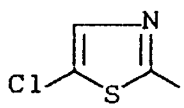 | - | 0 | $OC_2H_5$ | - | |
| 468 | 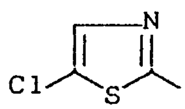 | - | 0 | Cl | (6)-$CH_3$ | |
| 469 | 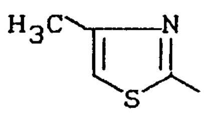 | - | 0 | $CO_2C_2H_5$ | - | |
| 470 | 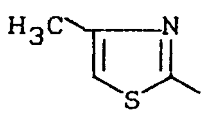 | - | 0 | $CO_2CH_3$ | (5)-Cl | |
| 471 | 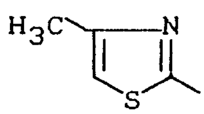 | - | 0 | $OCH_3$ | - | |
| 472 | 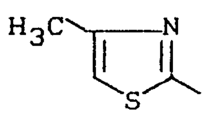 | - | 0 | $OC_3H_7$-i | - | |
| 473 | 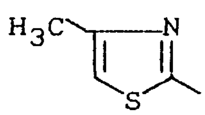 | - | 0 | Cl | - | 157 |
| 474 | 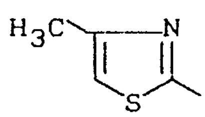 | - | 0 | Cl | (6)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 475 | 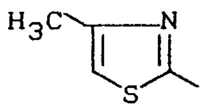 | - | 0 | $SO_2N(CH_3)OCH_3$ | - | |
| 476 | 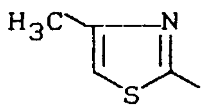 | - | 0 | $SC_2H_5$ | - | 189 |
| 477 | 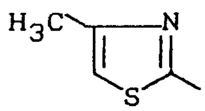 | - | 0 | $OCHF_2$ | - | |
| 478 | 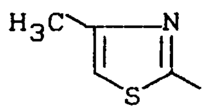 | - | 0 | $SO_2CH_3$ | - | |
| 479 | 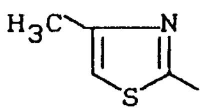 | - | 0 | $C_6H_5$ | - | 172 |
| 480 | 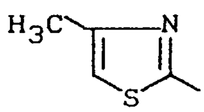 | - | 0 | $CF_3$ | - | |
| 481 | 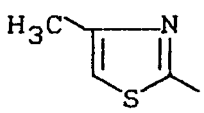 | - | 0 | $CH_3$ | - | |
| 482 | 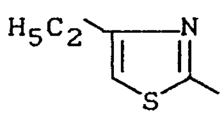 | - | 0 | $CO_2CH_3$ | - | |
| 483 | 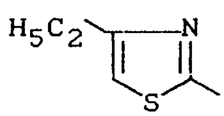 | - | 0 | $CO_2CH_3$ | (5)-Cl | |
| 484 | 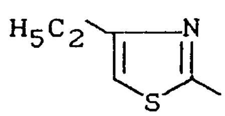 | $CH_2$ | 1 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 485 | 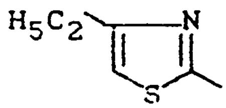 | NH | 1 | Cl | - | |
| 486 | 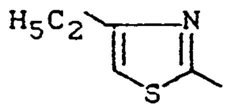 | - | 0 | Cl | - | |
| 487 | 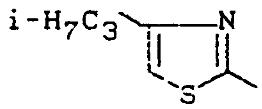 | - | 0 | $OCH_3$ | - | |
| 488 | 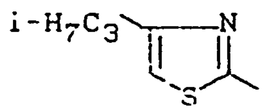 | - | 0 | $CO_2CH_3$ | - | |
| 489 | 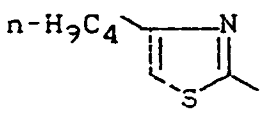 | - | 0 | $CO_2CH_3$ | - | |
| 490 | 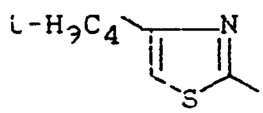 | - | 0 | $CO_2CH_3$ | - | |
| 491 | 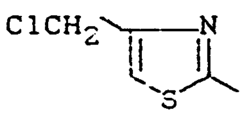 | - | 0 | $CO_2CH_3$ | - | |
| 492 | 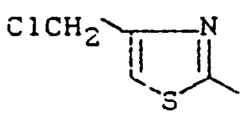 | - | 0 | $CF_3$ | - | |
| 493 | 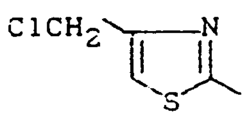 | - | 0 | Cl | (6)-Cl | |
| 494 | 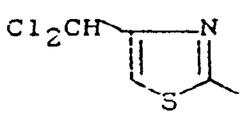 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 495 | 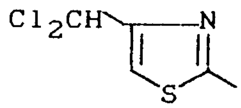 | - | 0 | $SCH_3$ | - | |
| 496 | 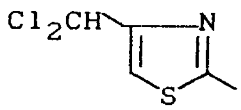 | - | 0 | $SO_2C_2H_5$ | - | |
| 497 | 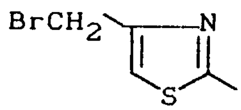 | - | 0 | $CO_2CH_3$ | - | |
| 498 | 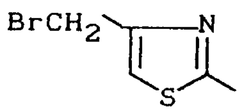 | - | 0 | $C_6H_5$ | - | |
| 499 | 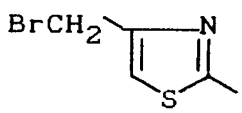 | $CH_2$ | 1 | $CO_2CH_3$ | - | |
| 500 | 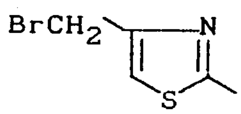 | - | 0 | $OC_2H_5$ | - | |
| 501 | 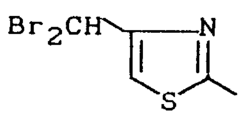 | - | 0 | $SC_2H_5$ | - | |
| 502 | 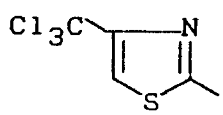 | - | 0 | $CO_2CH_3$ | - | |
| 503 | 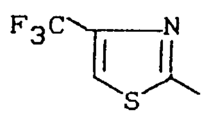 | - | 0 | $CO_2CH_3$ | - | |
| 504 | 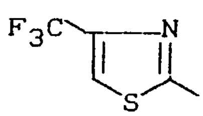 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 505 | 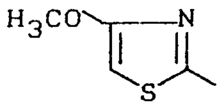 | - | 0 | $CO_2CH_3$ | - | |
| 506 | 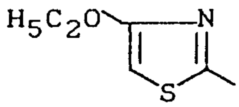 | - | 0 | $CO_2C_2H_5$ | - | |
| 507 | 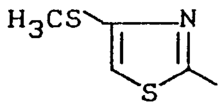 | - | 0 | $CO_2CH_3$ | - | |
| 508 | 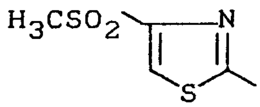 | - | 0 | $OCF_3$ | - | |
| 509 | 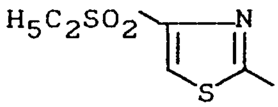 | - | 0 | $CO_2CH_3$ | - | |
| 510 | 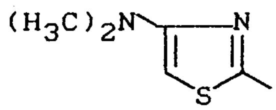 | - | 0 | $CO_2CH_3$ | - | |
| 511 | 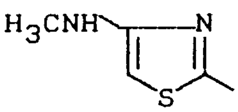 | - | 0 | $CO_2C_2H_5$ | - | |
| 512 | 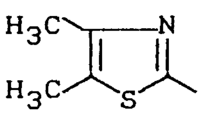 | - | 0 | $CO_2CH_3$ | - | |
| 513 | 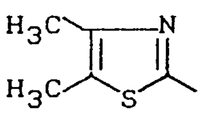 | - | 0 | $CO_2C_3H_7$-n | - | |
| 514 | 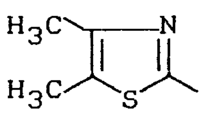 | - | 0 | Cl | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 515 | 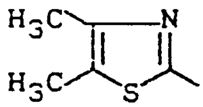 | - | 0 | $C_6H_5$ | - | |
| 516 | 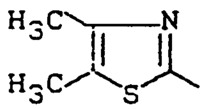 | - | 0 | $OCH_3$ | - | |
| 517 | 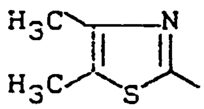 | - | 0 | $SC_2H_5$ | - | |
| 518 | 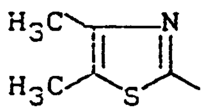 | - | 0 | $OCF_3$ | - | |
| 519 | 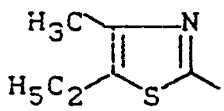 | - | 0 | $CO_2CH_3$ | - | |
| 520 | 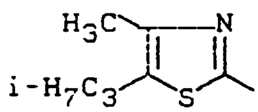 | - | 0 | $SCH_3$ | - | |
| 521 | 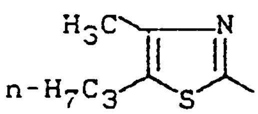 | - | 0 | Cl | (6)-Cl | |
| 522 | 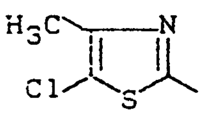 | - | 0 | $CO_2CH_3$ | (5)-Cl | |
| 523 | 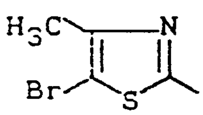 | - | 0 | $SC_2H_5$ | - | |
| 524 | 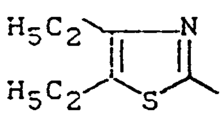 | - | 0 | $SO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 525 | 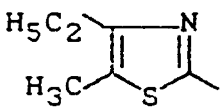 | - | 0 | $OC_2H_5$ | - | |
| 526 | 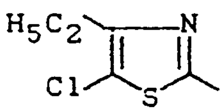 | - | 0 | Br | - | |
| 527 | 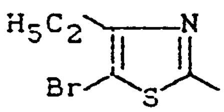 | - | 0 | $CO_2CH_2CH_2Cl$ | - | |
| 528 | 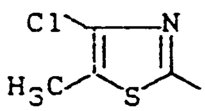 | - | 0 | $CF_3$ | - | |
| 529 | 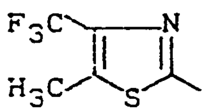 | - | 0 | $CO_2CH_3$ | - | |
| 530 | 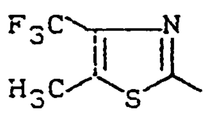 | - | 0 | $OC_2H_5$ | - | |
| 531 | 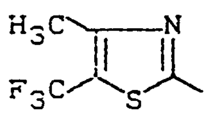 | - | 0 | $CO_2C_2H_5$ | - | |
| 532 | 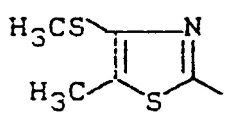 | - | 0 | $CO_2CH_3$ | (6)-Cl | |
| 533 | 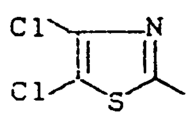 | - | 0 | $CO_2CH_3$ | - | |
| 534 | 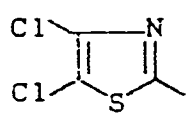 | - | 0 | $SC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 535 | 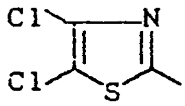 | - | 0 | $OC_2H_5$ | - | |
| 536 | 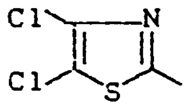 | - | 0 | $OCF_3$ | - | |
| 537 | 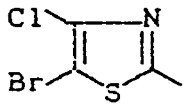 | - | 0 | $C_6H_5$ | - | |
| 538 | 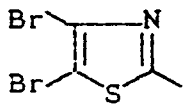 | - | 0 | $CO_2CH_3$ | - | |
| 539 | 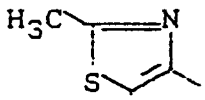 | - | 0 | $CO_2C_2H_5$ | - | |
| 540 | 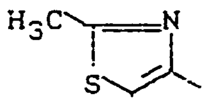 | - | 0 | $CO_2CH_3$ | (5)-Cl | |
| 541 | 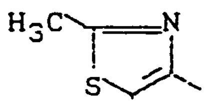 | - | 0 | $OC_2H_5$ | - | |
| 542 | 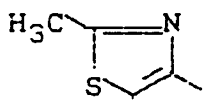 | - | 0 | $OCH_2CH_2Cl$ | - | |
| 543 | 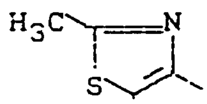 | - | 0 | $SC_2H_5$ | - | |
| 544 | 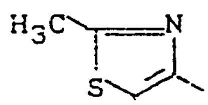 | - | 0 | $SO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 545 | 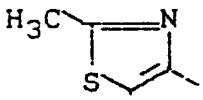 | - | 0 | $OCF_3$ | - | |
| 546 | 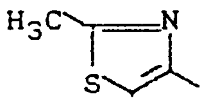 | - | 0 | Br | - | |
| 547 | 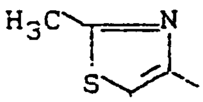 | - | 0 | $SOC_3H_7$-n | - | |
| 548 | 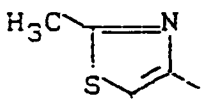 | $CH_2$ | 1 | $CO_2CH_3$ | - | |
| 549 | 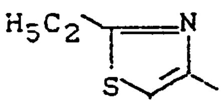 | - | 0 | $CO_2CH_3$ | - | |
| 550 | 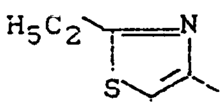 | - | 0 | $SCH_3$ | - | |
| 551 | 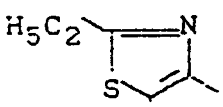 | - | 0 | $SO_2C_3H_7$-n | - | |
| 552 | 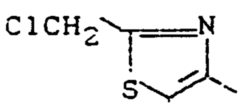 | - | 0 | $CO_2CH_3$ | - | |
| 553 | 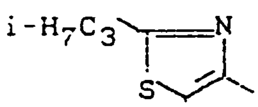 | - | 0 | $OCH_3$ | - | |
| 554 | 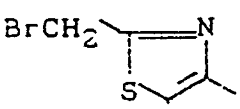 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 555 | 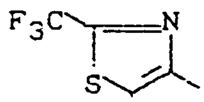 | - | 0 | $CO_2CH_3$ | - | |
| 556 | 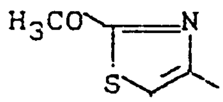 | - | 0 | $CO_2CH_3$ | (5)-Cl | |
| 557 | 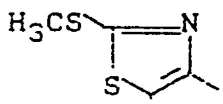 | - | 0 | $CO_2C_2H_5$ | - | |
| 558 | 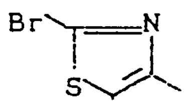 | - | 0 | $OC_2H_5$ | - | |
| 559 | 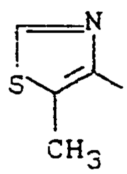 | - | 0 | $CO_2CH_3$ | - | |
| 560 | 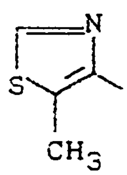 | - | 0 | Cl | - | |
| 561 | 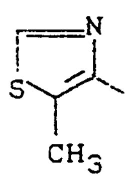 | - | 0 | $OC_2H_5$ | - | |
| 562 | 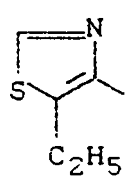 | - | 0 | $C_6H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 563 | 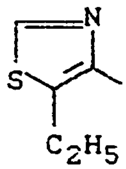 | - | 0 | $SCH_3$ | - | |
| 564 | 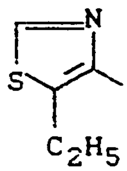 | - | 0 | $SO_2N(CH_3)_2$ | - | |
| 565 | 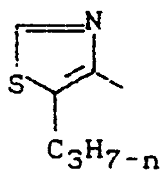 | - | 0 | $OCF_3$ | - | |
| 566 | 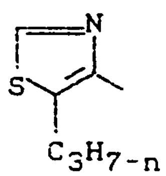 | - | 0 | $SO_2C_3H_7$-n | - | |
| 567 | 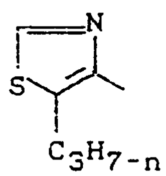 | - | 0 | $CO_2CH_3$ | - | |
| 568 | 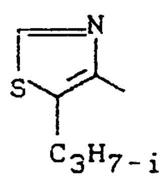 | - | 0 | Cl | (6)-Cl | |
| 569 | 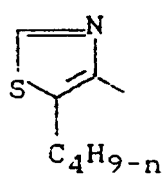 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 570 | 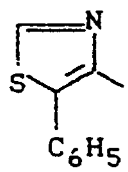 | - | 0 | $CO_2C_2H_5$ | - | |
| 571 | 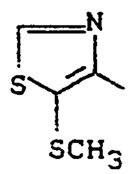 | - | 0 | $OC_2H_5$ | - | |
| 572 | 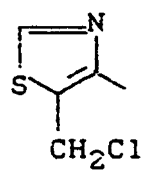 | - | 0 | $CO_2CH_3$ | - | |
| 573 | 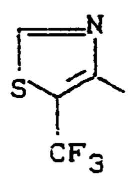 | - | 0 | $CO_2C_2H_5$ | - | |
| 574 | 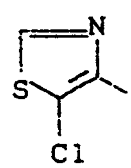 | - | 0 | $CO_2C_3H_{7-n}$ | - | |
| 575 | 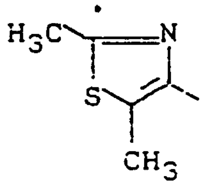 | - | 0 | $CO_2CH_3$ | - | |
| 576 | 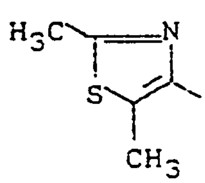 | - | 0 | $CO_2CH_3$ | (6)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 577 | 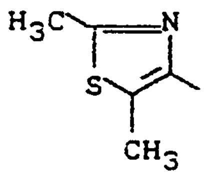 | - | 0 | F | - | |
| 578 | 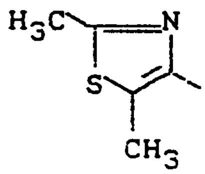 | $CH_2$ | 1 | Cl | - | |
| 579 | 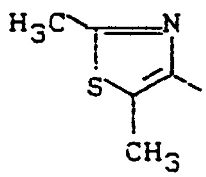 | - | 0 | $OCH_3$ | - | |
| 580 | 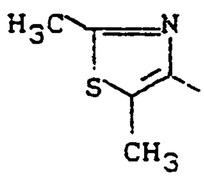 | - | 0 | $SC_3H_7$-i | - | |
| 581 | 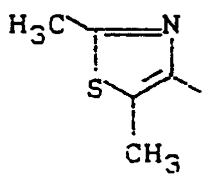 | - | 0 | $SO_2C_2H_5$ | - | |
| 582 | 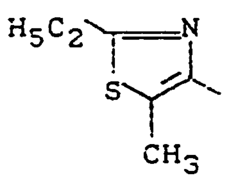 | - | 0 | $CO_2CH_3$ | - | |
| 583 | 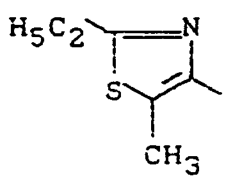 | - | 0 | $CO_2CH_2CH_2OCH_3$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 584 | 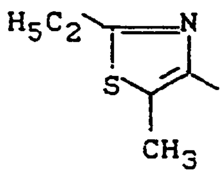 | - | 0 | $OCF_3$ | - | |
| 585 | 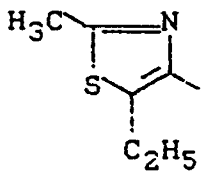 | - | 0 | $C_6H_5$ | - | |
| 586 | 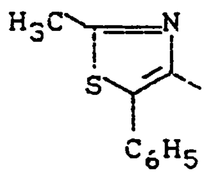 | - | 0 | $CO_2CH_3$ | - | |
| 587 | 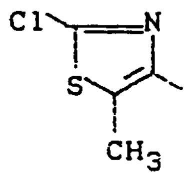 | - | 0 | $CO_2CH_3$ | - | |
| 588 | 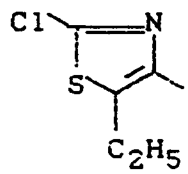 | - | 0 | $CO_2CH_3$ | (5)-Cl | |
| 589 | 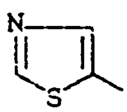 | - | 0 | $CO_2CH_3$ | - | |
| 590 | 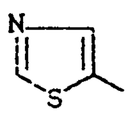 | - | 0 | $CF_3$ | - | |
| 591 | 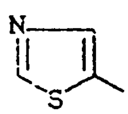 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 592 | 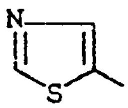 | - | 0 | $SCH_3$ | - | |
| 593 | 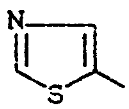 | - | 0 | $SO_2C_2H_5$ | - | |
| 594 | 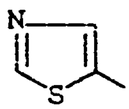 | - | 0 | Cl | (6)-$CH_3$ | |
| 595 | 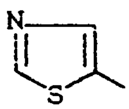 | - | 0 | $C_6H_5$ | - | |
| 596 | 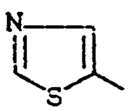 | - | 0 | $OCF_3$ | - | |
| 597 | 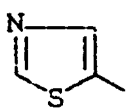 | - | 0 | $CO_2CH_3$ | (5)-Cl | |
| 598 | 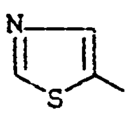 | - | 0 | F | - | |
| 599 | 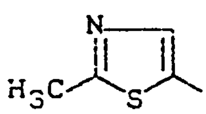 | - | 0 | $CO_2CH_3$ | - | |
| 600 | 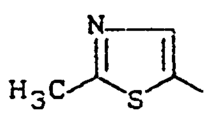 | - | 0 | Br | - | |
| 601 | 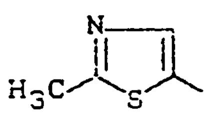 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 602 | 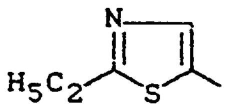 | - | 0 | $SC_3H_7$-i | - | |
| 603 | 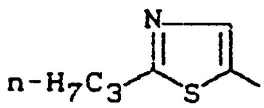 | - | 0 | $CO_2CH_3$ | - | |
| 604 | 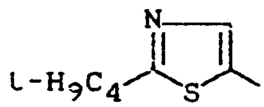 | - | 0 | $CO_2CH_3$ | - | |
| 605 | 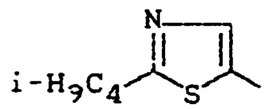 | - | 0 | $CO_2C_2H_5$ | - | |
| 606 | 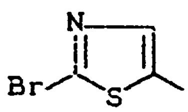 | - | 0 | $SO_2C_2H_5$ | - | |
| 607 | 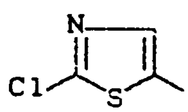 | - | 0 | $CO_2CH_3$ | (6)-Cl | |
| 608 | 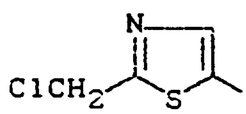 | - | 0 | $C_6H_5$ | - | |
| 609 | 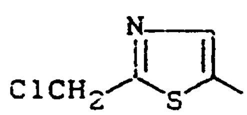 | $CH_2$ | 1 | Cl | - | |
| 610 | 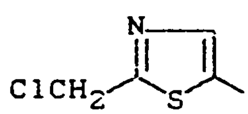 | - | 0 | $OCH_3$ | - | |
| 611 | 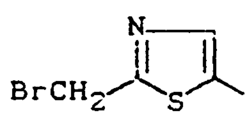 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 612 | 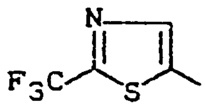 | - | 0 | $OC_2H_5$ | - | |
| 613 | 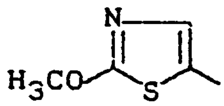 | - | 0 | $CO_2CH_3$ | - | |
| 614 | 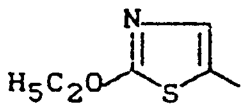 | - | 0 | $SO_2C_3H_7$-n | - | |
| 615 | 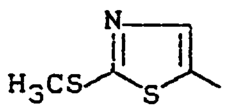 | - | 0 | $CO_2C_2H_5$ | - | |
| 616 | 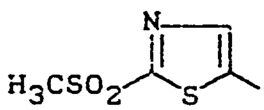 | - | 0 | $SO_2N(CH_3)_2$ | - | |
| 617 | 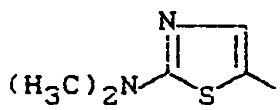 | - | 0 | $CO_2CH_3$ | - | |
| 618 | 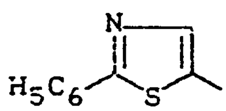 | - | 0 | $C_6H_5$ | - | |
| 619 | 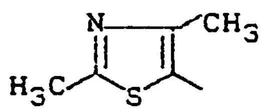 | - | 0 | $CO_2CH_3$ | - | |
| 620 | 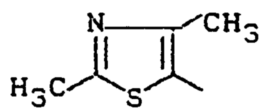 | - | 0 | Cl | - | |
| 621 | 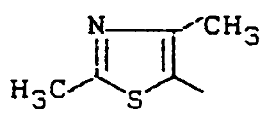 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 622 | 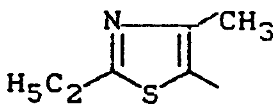 | - | 0 | $SCH_3$ | - | |
| 623 | 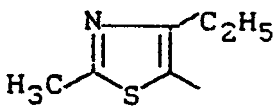 | - | 0 | $SO_2C_2H_5$ | - | |
| 624 | 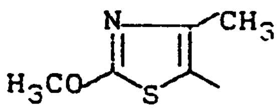 | - | 0 | $C_6H_5$ | - | |
| 625 | 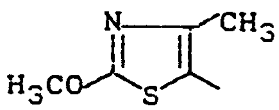 | - | 0 | $OC_2H_5$ | - | |
| 626 | 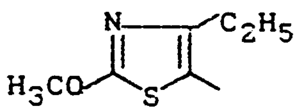 | - | 0 | Cl | (6)-Cl | |
| 627 | 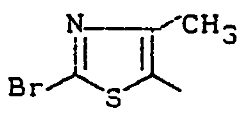 | - | 0 | $CO_2CH_3$ | - | |
| 628 | 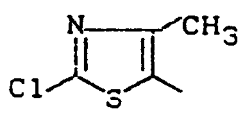 | - | 0 | $CF_3$ | - | |
| 629 | 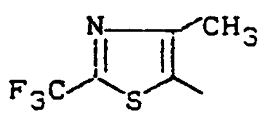 | - | 0 | $CO_2CH_3$ | - | |
| 630 | 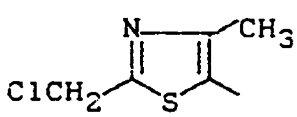 | - | 0 | $OC_2H_5$ | - | |
| 631 | 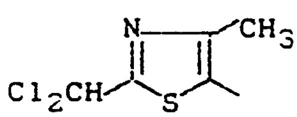 | - | 0 | $SC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 632 | 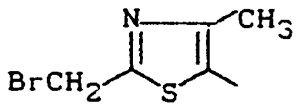 | - | 0 | Cl | (6)-Cl | |
| 633 | 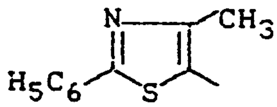 | - | 0 | $SO_2N(CH_3)_2$ | - | |
| 634 | 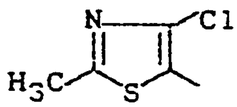 | - | 0 | $CO_2C_2H_5$ | - | |
| 635 | 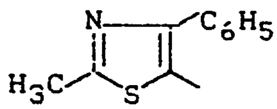 | - | 0 | $C_6H_5$ | - | |
| 636 | 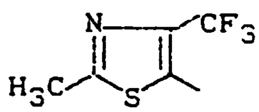 | - | 0 | $CH_3$ | - | |
| 637 | 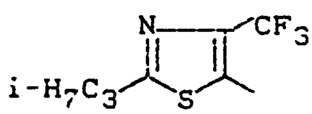 | - | 0 | $OCF_3$ | - | |
| 638 | 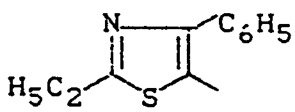 | $CH_2$ | 1 | $CO_2CH_3$ | - | |
| 639 | 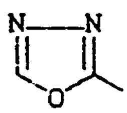 | - | 0 | $CO_2CH_3$ | - | |
| 640 | 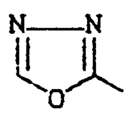 | - | 0 | $CO_2CH_3$ | (5)-Cl | |
| 641 | 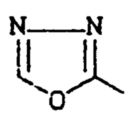 | - | 0 | $CO_2CH_3$ | (6)-Cl | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 642 | 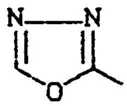 | - | 0 | $OC_2H_5$ | - | |
| 643 | 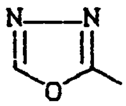 | - | 0 | $SC_2H_5$ | - | |
| 644 | 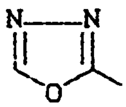 | - | 0 | $SOC_3H_7$-n | - | |
| 645 | 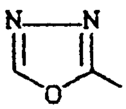 | - | 0 | Cl | - | |
| 646 | 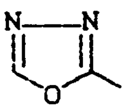 | $CH_2$ | 1 | Cl | - | |
| 647 | 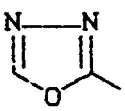 | - | 0 | $OCHF_2$ | - | |
| 648 | 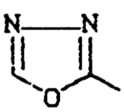 | - | 0 | $OCF_3$ | - | |
| 649 | 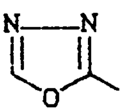 | - | 0 | $SO_2C_2H_5$ | - | |
| 650 | 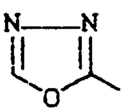 | - | 0 | $CO_2C_2H_5$ | - | |
| 651 | 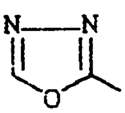 | - | 0 | $CF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 652 | 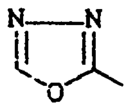 | - | 0 | $CH_3$ | - | |
| 653 | 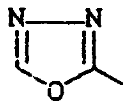 | - | 0 | $C_6H_5$ | - | |
| 654 | 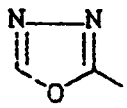 | - | 0 | $SO_2N(CH_3)_2$ | - | |
| 655 | 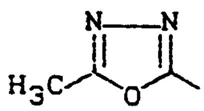 | - | 0 | $CO_2CH_3$ | (5)-Cl | |
| 656 | 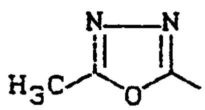 | - | 0 | $OCF_3$ | - | |
| 657 | 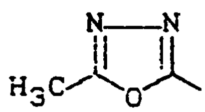 | - | 0 | $OC_3H_{7-i}$ | - | |
| 658 | 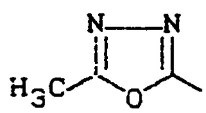 | - | 0 | $SC_2H_5$ | - | |
| 659 | 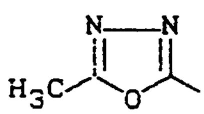 | $CH_2$ | 1 | $CO_2CH_3$ | - | |
| 660 | 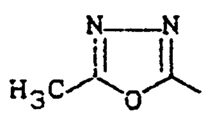 | - | 0 | $SO_2C_2H_5$ | - | |
| 661 | 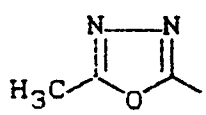 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 662 | 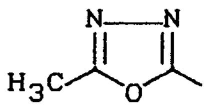 | - | 0 | $C_6H_5$ | - | 148 |
| 663 | 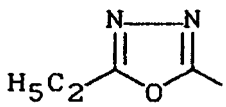 | - | 0 | $CO_2C_3H_7$ | - | |
| 664 | 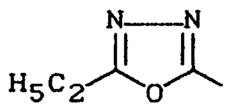 | - | 0 | $OC_2H_5$ | - | |
| 665 | 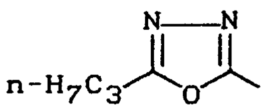 | - | 0 | $SC_3H_7$-i | - | |
| 666 | 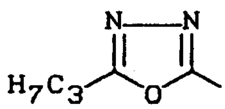 | - | 0 | $SO_2N(CH_3)_2$ | - | |
| 667 | 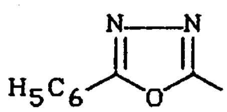 | - | 0 | $CO_2CH_3$ | - | |
| 668 | 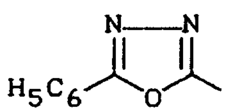 | - | 0 | $SC_2H_5$ | - | |
| 669 | 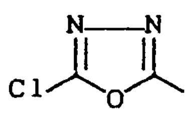 | - | 0 | $CO_2CH_3$ | - | |
| 670 | 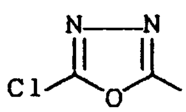 | - | 0 | Br | - | |
| 671 | 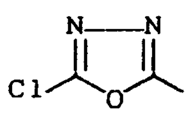 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 672 | 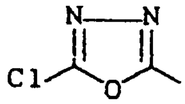 | - | 0 | $SC_2H_5$ | - | |
| 673 | 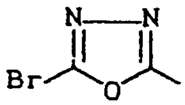 | - | 0 | $CO_2CH_3$ | (5)-Cl | |
| 674 | 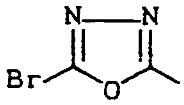 | - | 0 | Cl | - | |
| 675 | 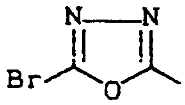 | - | 0 | $OCF_3$ | - | |
| 676 | 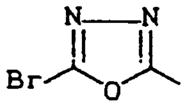 | - | 0 | $C_6H_5$ | - | |
| 677 | 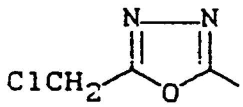 | - | 0 | $CO_2CH_3$ | - | |
| 678 | 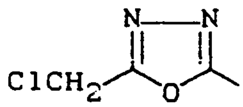 | - | 0 | $SO_2C_2H_5$ | - | |
| 679 | 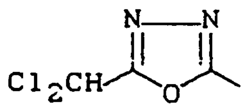 | - | 0 | $CO_2CH_3$ | - | |
| 680 | 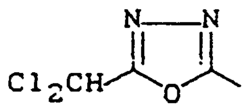 | - | 0 | $OCF_3$ | - | |
| 681 | 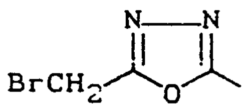 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 682 | 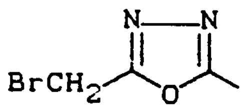 | - | 0 | $SO_2C_2H_5$ | - | |
| 683 | 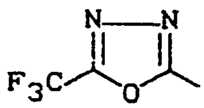 | - | 0 | $CO_2C_2H_5$ | - | |
| 684 | 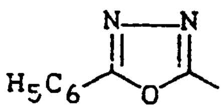 | - | 0 | $OC_2H_5$ | - | |
| 685 | 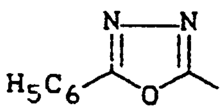 | - | 0 | $CO_2CH_3$ | (5)-Cl | |
| 686 | 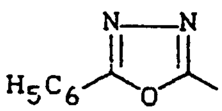 | - | 0 | 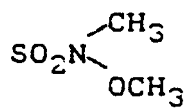 | - | |
| 687 | 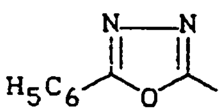 | $CH_2$ | 1 | $CO_2CH_3$ | - | |
| 688 | 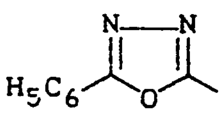 | - | 0 | Cl | - | |
| 689 | 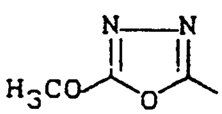 | - | 0 | $CO_2CH_3$ | - | |
| 690 | 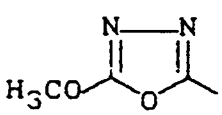 | - | 0 | $C_6H_5$ | - | |
| 691 | 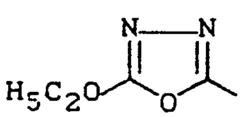 | - | 0 | Br | | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 692 | 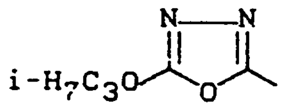 | - | 0 | $OCH_3$ | - | |
| 693 | 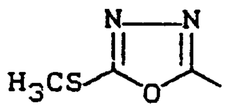 | - | 0 | $OCF_3$ | - | |
| 694 | 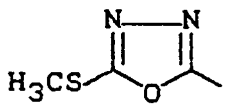 | - | 0 | Cl | (6)-Cl | |
| 695 | 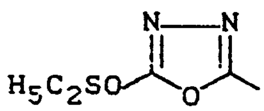 | - | 0 | $CF_3$ | - | |
| 696 | 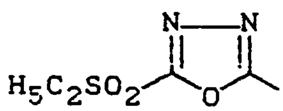 | - | 0 | $CO_2CH_3$ | - | |
| 697 | 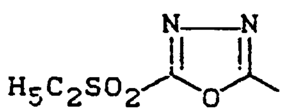 | - | 0 | $OC_2H_5$ | - | |
| 698 | 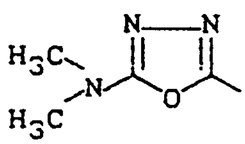 | - | 0 | $SO_2C_3H_7$-i | - | |
| 699 | 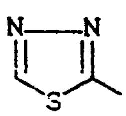 | - | 0 | $CO_2CH_3$ | - | |
| 700 | 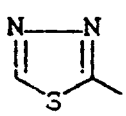 | - | 0 | $CO_2CH_3$ | (5)-Cl | |
| 701 | 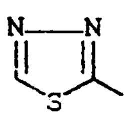 | - | 0 | $CO_2CH_3$ | (6)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 702 | 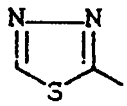 | - | 0 | $OC_2H_5$ | - | |
| 703 | 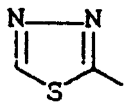 | - | 0 | $SC_2H_5$ | - | |
| 704 | 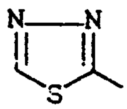 | - | 0 | $SOC_3H_{7-n}$ | - | |
| 705 | 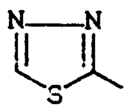 | - | 0 | $SO_2C_2H_5$ | - | |
| 706 | 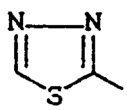 | - | 0 | Cl | - | |
| 707 | 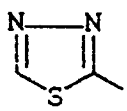 | - | 0 | $OCF_3$ | - | |
| 708 | 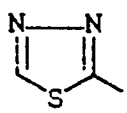 | - | 0 | $OCHF_2$ | - | |
| 709 | 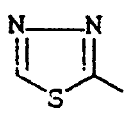 | - | 0 | $CF_3$ | - | |
| 710 | 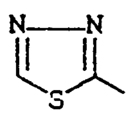 | - | 0 | Cl | (6)-Cl | |
| 711 | 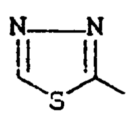 | - | 0 | $CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 712 | 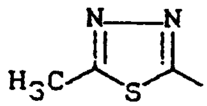 | - | 0 | $CO_2CH_3$ | (5)-Cl | |
| 713 | 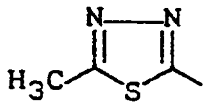 | - | 0 | Cl | - | |
| 714 | 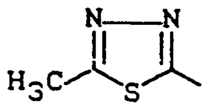 | - | 0 | $OCF_3$ | - | |
| 715 | 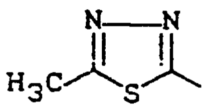 | - | 0 | $OCHF_2$ | - | |
| 716 | 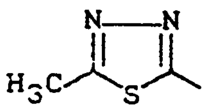 | - | 0 | $OC_2H_5$ | - | |
| 717 | 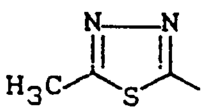 | - | 0 | $SC_2H_5$ | - | |
| 718 | 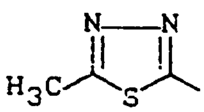 | - | 0 | $SOC_2H_5$ | - | |
| 719 | 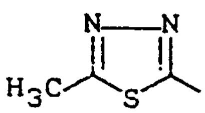 | - | 0 | $SO_2C_2H_5$ | - | |
| 720 | 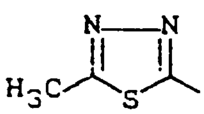 | - | 0 | $C_6H_5$ | - | |
| 721 | 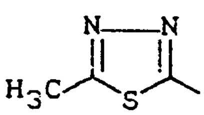 | - | 0 | Cl | (6)-$CH_3$ | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 722 | 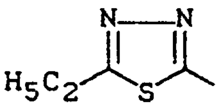 | - | 0 | Cl | - | |
| 723 | 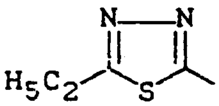 | - | 0 | $CO_2CH_3$ | - | |
| 724 | 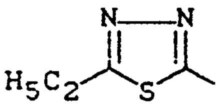 | - | 0 | $CO_2C_2H_5$ | - | |
| 725 | 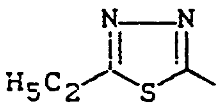 | - | 0 | $SCH_3$ | - | |
| 726 | 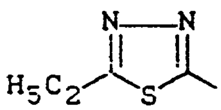 | - | 0 | $SC_2H_5$ | - | |
| 727 | 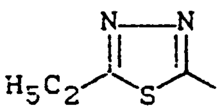 | - | 0 | $OC_2H_5$ | - | |
| 728 | 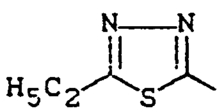 | - | 0 | $SO_2C_2H_5$ | - | |
| 729 | 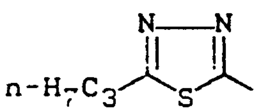 | - | 0 | $CO_2CH_3$ | - | |
| 730 | 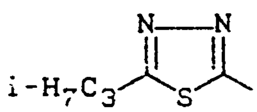 | - | 0 | $CO_2CH_3$ | - | |
| 731 | 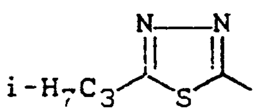 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 732 | 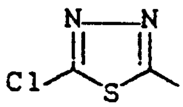 | - | 0 | $CO_2CH_3$ | - | |
| 733 | 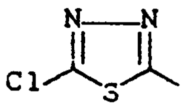 | - | 0 | $OCH_3$ | - | |
| 734 | 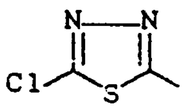 | - | 0 | $CO_2CH_3$ | (5)-Cl | |
| 735 | 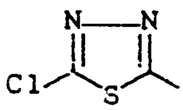 | - | 0 | $CO_2CH_3$ | (6)-Cl | |
| 736 | 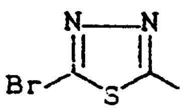 | - | 0 | $CO_2CH_3$ | - | |
| 737 | 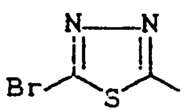 | - | 0 | $SC_2H_5$ | - | |
| 738 | 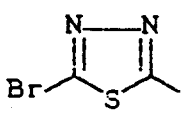 | - | 0 | $C_6H_5$ | - | |
| 739 | 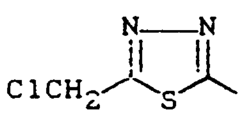 | - | 0 | $CO_2CH_3$ | - | |
| 740 | 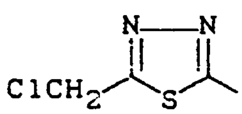 | - | 0 | $OC_2H_5$ | - | |
| 741 | 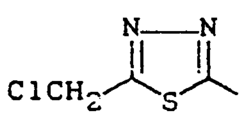 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 742 | 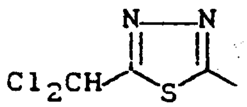 | - | 0 | $CO_2CH_3$ | - | |
| 743 | 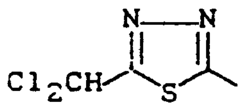 | - | 0 | $C_6H_5$ | - | |
| 744 | 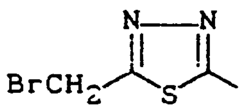 | - | 0 | $CO_2CH_3$ | - | |
| 745 | 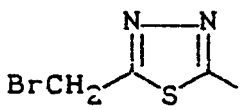 | - | 0 | $CO_2C_2H_5$ | - | |
| 746 | 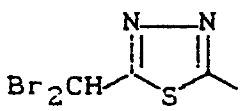 | - | 0 | $CO_2CH_3$ | - | |
| 747 | 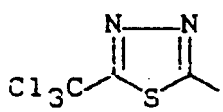 | - | 0 | $CO_2CH_3$ | - | |
| 748 | 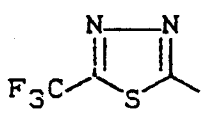 | - | 0 | $CO_2CH_3$ | - | |
| 749 | 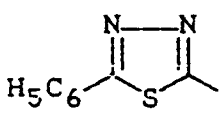 | - | 0 | $CO_2CH_3$ | - | |
| 750 | 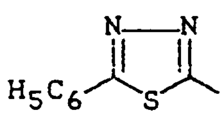 | - | 0 | $CO_2C_2H_5$ | - | |
| 751 | 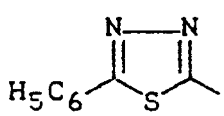 | - | 0 | Cl | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 752 | 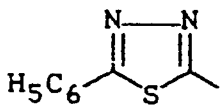 | - | 0 | $OCF_3$ | - | |
| 753 | 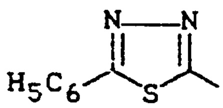 | - | 0 | $OCH_3$ | - | |
| 754 | 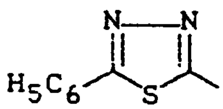 | - | 0 | $OC_2H_5$ | - | |
| 755 | 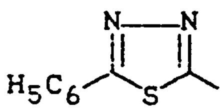 | - | 0 | $SC_2H_5$ | - | |
| 756 | 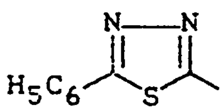 | - | 0 | $SO_2C_3H_7$-n | - | |
| 757 | 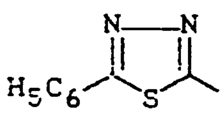 | - | 0 | $CO_2CH_3$ | (6)-Cl | |
| 758 | 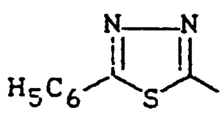 | - | 0 | $C_6H_5$ | - | |
| 759 | 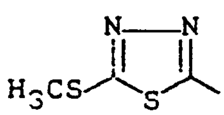 | - | 0 | $CO_2CH_3$ | - | |
| 760 | 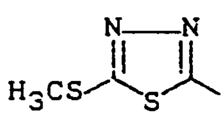 | - | 0 | $SO_2N(CH_3)_2$ | - | |
| 761 | 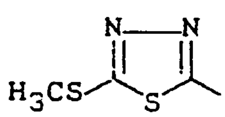 | - | 0 | $SC_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 762 | 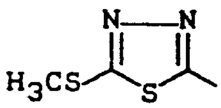 | - | 0 | $C_6H_5$ | - | |
| 763 | 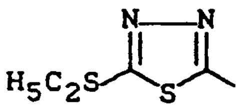 | - | 0 | $CO_2CH_3$ | - | |
| 764 | 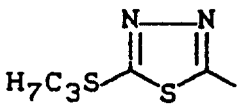 | - | 0 | $CO_2CH_3$ | - | |
| 765 | 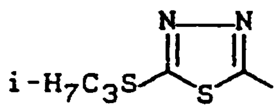 | - | 0 | $CO_2CH_3$ | - | |
| 766 | 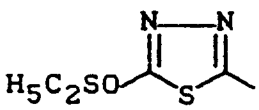 | - | 0 | $CO_2C_2H_5$ | - | |
| 767 | 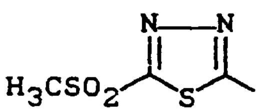 | - | 0 | $CO_2CH_3$ | - | |
| 768 | 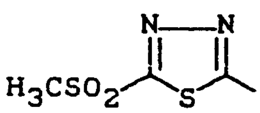 | - | 0 | $OC_2H_5$ | - | |
| 769 | 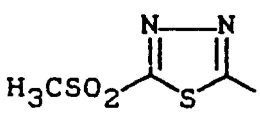 | $CH_2$ | 1 | $CO_2CH_3$ | - | |
| 770 | 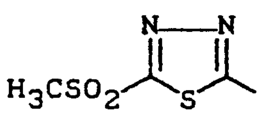 | - | 0 | $OCF_3$ | - | |
| 771 | 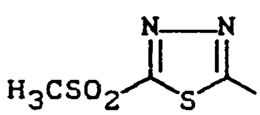 | - | 0 | $C_6H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 772 | 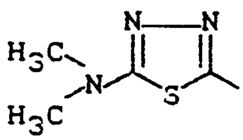 | - | 0 | $CO_2CH_3$ | - | |
| 773 | 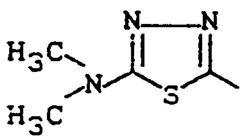 | - | 0 | $CO_2C_2H_5$ | - | |
| 774 | 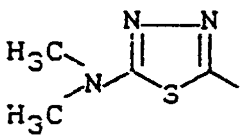 | - | 0 | $OC_2H_5$ | - | |
| 775 | 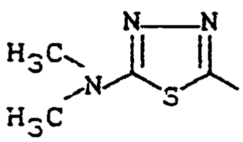 | - | 0 | $SC_2H_5$ | - | |
| 776 | 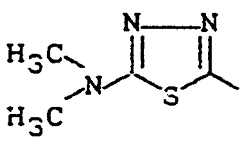 | - | 0 | $SO_2C_3H_7$ | - | |
| 777 | 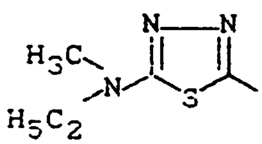 | - | 0 | $CO_2CH_3$ | - | |
| 778 | 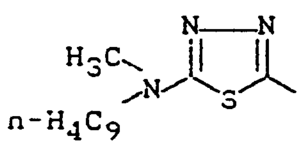 | - | 0 | $CO_2CH_3$ | - | |
| 779 | 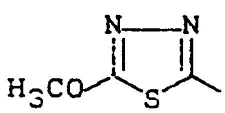 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 780 | 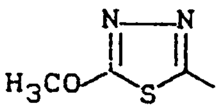 | - | 0 | $CO_2C_2H_5$ | - | |
| 781 | 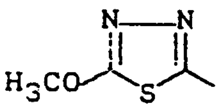 | - | 0 | $OC_2H_5$ | - | |
| 782 | 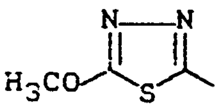 | - | 0 | $SC_2H_5$ | - | |
| 783 | 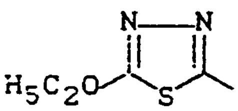 | - | 0 | $CO_2CH_3$ | - | |
| 784 | 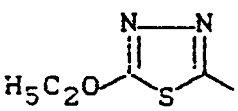 | - | 0 | $CO_2CH_3$ | (5)-Cl | |
| 785 | 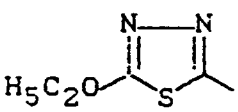 | - | 0 | $OCF_3$ | - | |
| 786 | 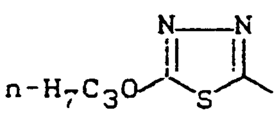 | - | 0 | $CO_2CH_3$ | - | |
| 787 | 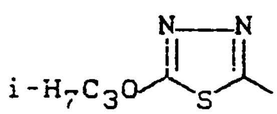 | - | 0 | $CO_2CH_3$ | - | |
| 788 | 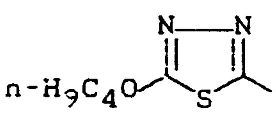 | - | 0 | $CO_2CH_3$ | - | |
| 789 | 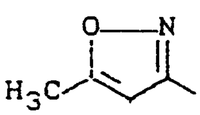 | - | 0 | $CO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 790 | 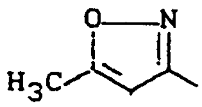 | - | 0 | $CO_2CH_3$ | (5)-Cl | |
| 791 | 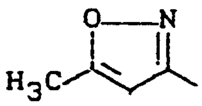 | - | 0 | $CO_2CH_3$ | (6)-Cl | |
| 792 | 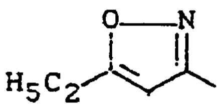 | - | 0 | $CO_2CH_3$ | - | |
| 793 | 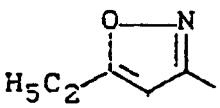 | - | 0 | $CO_2C_2H_5$ | - | |
| 794 | 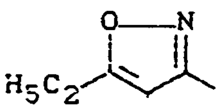 | - | 0 | $OCH_3$ | - | |
| 795 | 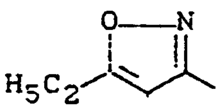 | - | 0 | $OC_2H_5$ | - | |
| 796 | 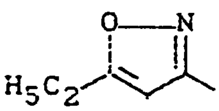 | - | 0 | $SC_2H_5$ | - | |
| 797 | 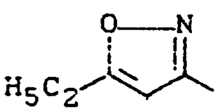 | - | 0 | $SCH_3$ | - | |
| 798 | 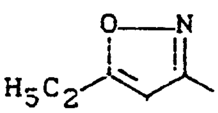 | - | 0 | $SO_2CH_3$ | - | |
| 799 | 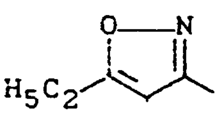 | - | 0 | $SO_2C_2H_5$ | - | |

Tabelle 1 - Fortsetzung

| Bsp.-Nr. | $R^1$ | A | n | $R^2$ | (Posi-Lion-) $R^3$ | Schmelzpunkt (° C) |
|---|---|---|---|---|---|---|
| 800 | 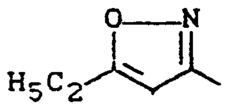 | - | 0 | $OCF_3$ | - | |
| 801 | 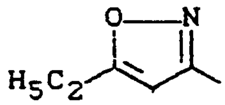 | - | 0 | $C_6H_5$ | - | |
| 802 | 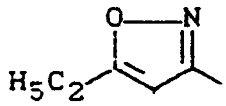 | - | 0 | Cl | (6)-$CH_3$ | |
| 803 | 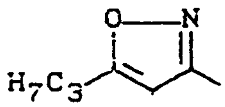 | - | 0 | $CO_2CH_3$ | - | |
| 804 | 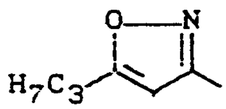 | - | 0 | $OC_2H_5$ | - | |
| 805 | 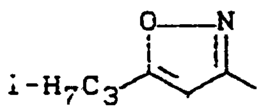 | - | 0 | $CO_2CH_3$ | - | |
| 806 | 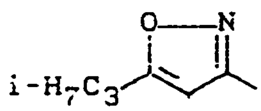 | - | 0 | $SO_2CH_3$ | - | |
| 807 | 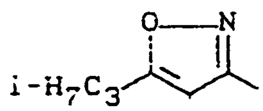 | - | 0 | $SO_2N(CH_3)_2$ | - | |
| 808 | 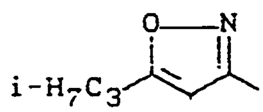 | - | 0 | $OCF_3$ | - | |
| 809 | 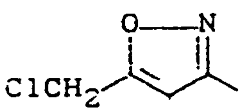 | - | 0 | $CO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 810 | 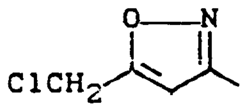 | - | 0 | $OC_2H_5$ | - | |
| 811 | 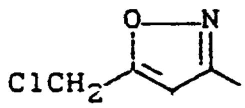 | - | 0 | $SC_2H_5$ | - | |
| 812 | 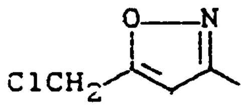 | - | 0 | $SO_2C_2H_5$ | - | |
| 813 | 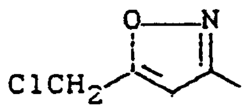 | - | 0 | $C_6H_5$ | - | |
| 814 | 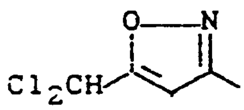 | - | 0 | $CO_2CH_3$ | - | |
| 815 | 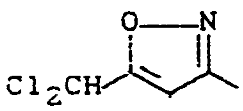 | - | 0 | $CO_2C_2H_5$ | - | |
| 816 | 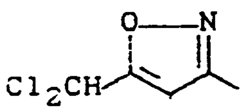 | - | 0 | $SO_2C_2H_5$ | - | |
| 817 | 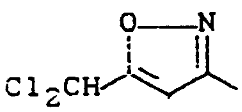 | - | 0 | $OCH_3$ | - | |
| 818 | 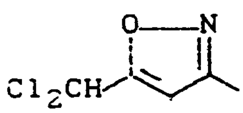 | - | 0 | $OC_2H_5$ | - | |
| 819 | 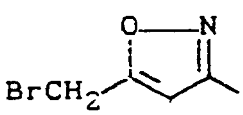 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 820 | 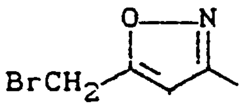 | - | 0 | $CO_2C_2H_5$ | - | |
| 821 | 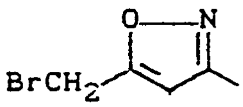 | - | 0 | Cl | - | |
| 822 | 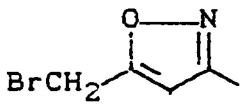 | - | 0 | $OC_2H_5$ | - | |
| 823 | 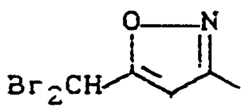 | - | 0 | $CO_2CH_3$ | - | |
| 824 | 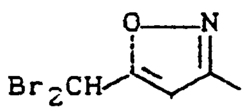 | - | 0 | $SO_2C_2H_5$ | - | |
| 825 | 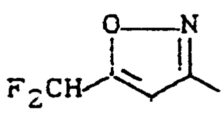 | - | 0 | $CO_2CH_3$ | - | |
| 826 | 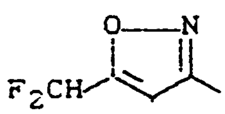 | - | 0 | $C_6H_5$ | - | |
| 827 | 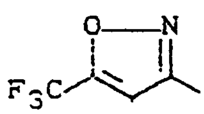 | - | 0 | $CO_2CH_3$ | - | |
| 828 | 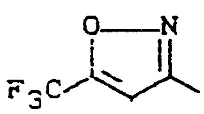 | - | 0 | $OC_2H_5$ | - | |
| 829 | 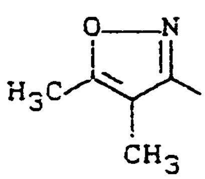 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 830 | 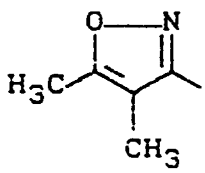 | - | 0 | $CO_2C_2H_5$ | - | |
| 631 | 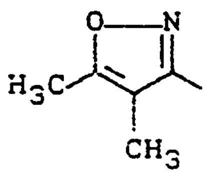 | - | 0 | Cl | - | |
| 632 | 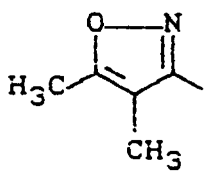 | - | 0 | Cl | (6)-Cl | |
| 833 | 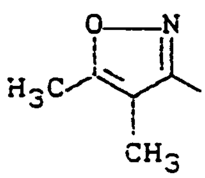 | - | 0 | $OCH_3$ | - | |
| 834 | 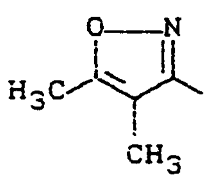 | - | 0 | $OC_2H_5$ | - | |
| 835 | 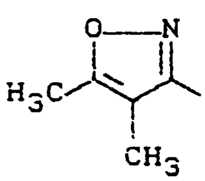 | - | 0 | $SC_2H_5$ | - | |
| 836 | 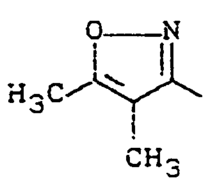 | - | 0 | $SO_2C_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 837 | 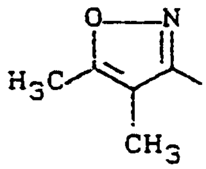 | - | 0 | $C_6H_5$ | - | |
| 838 | 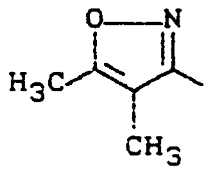 | - | 0 | $OCF_3$ | - | |
| 839 | 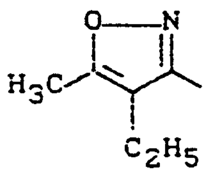 | - | 0 | $CO_2CH_3$ | - | |
| 840 | 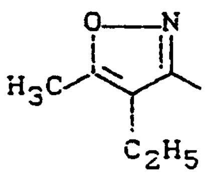 | - | 0 | $CO_2C_2H_5$ | - | |
| 841 | 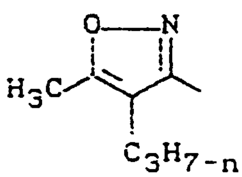 | - | 0 | $CF_3$ | - | |
| 842 | 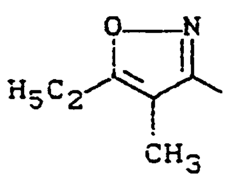 | - | 0 | $SO_2N(CH_3)_2$ | - | |
| 843 | 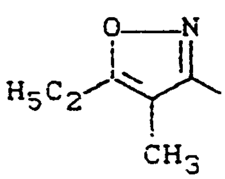 | - | 0 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 844 | 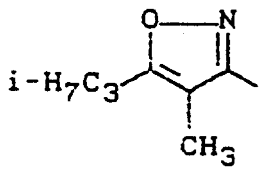 | - | 0 | $OCH_3$ | - | |
| 845 | 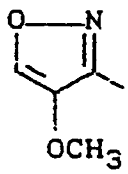 | - | 0 | $OC_2H_5$ | - | |
| 846 | 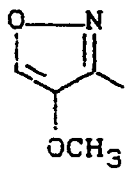 | - | 0 | $CO_2CH_3$ | - | |
| 847 | 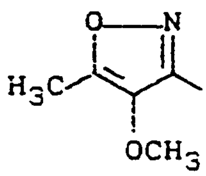 | - | 0 | $SO_2C_2H_5$ | - | |
| 848 | 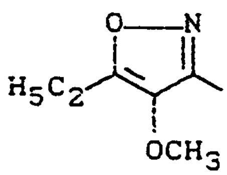 | - | 0 | $CO_2CH_3$ | - | |
| 849 | 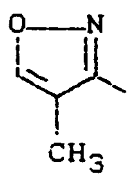 | - | 0 | $CO_2CH_3$ | - | |
| 850 | 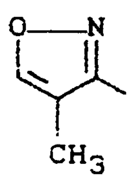 | - | 0 | Br | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 851 | 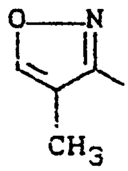 | - | 0 | $OC_2H_5$ | - | |
| 852 | 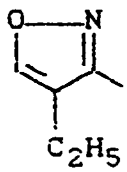 | - | 0 | $CO_2CH_3$ | - | |
| 853 | 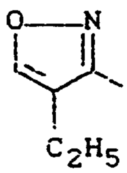 | - | 0 | $OCF_3$ | - | |
| 854 | 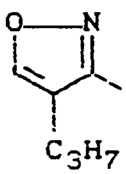 | - | 0 | $SO_2C_2H_5$ | - | |
| 855 | 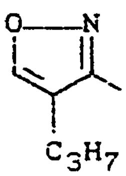 | - | 0 | $CO_2CH_3$ | - | |
| 856 | 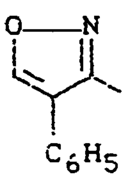 | - | 0 | $CO_2CH_3$ | - | |
| 857 | 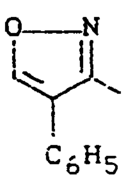 | - | 0 | $CO_2C_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 858 | 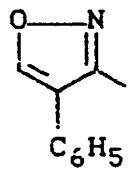 | - | 0 | $SC_2H_5$ | - | |
| 859 | 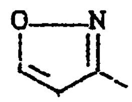 | - | 0 | $CO_2C_2H_5$ | - | |
| 860 | 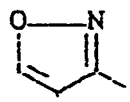 | - | 0 | $CO_2CH_3$ | (5)-Cl | |
| 861 | 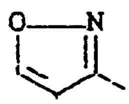 | - | 0 | $CO_2CH_3$ | (6)-Cl | |
| 862 | 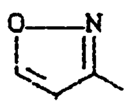 | - | 0 | F | - | |
| 863 | 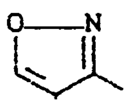 | - | 0 | Cl | - | |
| 864 | 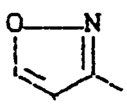 | - | 0 | $OCH_3$ | - | |
| 865 | 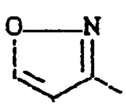 | - | 0 | $OC_2H_5$ | - | |
| 866 | 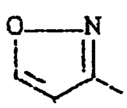 | - | 0 | $SC_2H_5$ | - | |
| 867 | 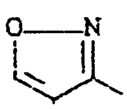 | - | 0 | $SO_2C_3H_7$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 868 | 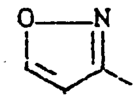 | - | 0 | $C_6H_5$ | - | |
| 869 | 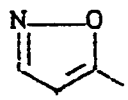 | - | 0 | $CO_2C_2H_5$ | - | |
| 870 | 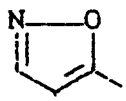 | - | 0 | $CO_2CH_3$ | (5)-Cl | |
| 871 | 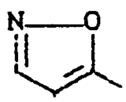 | - | 0 | $CO_2CH_3$ | (6)-Cl | |
| 872 | 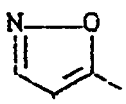 | - | 0 | $OCF_3$ | - | |
| 873 | 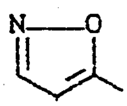 | - | 0 | Cl | - | |
| 874 | 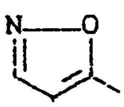 | - | 0 | $OC_2H_5$ | - | |
| 875 | 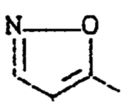 | - | 0 | $SCH_3$ | - | |
| 876 | 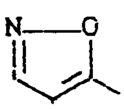 | - | 0 | $SO_2C_2H_5$ | - | |
| 877 | 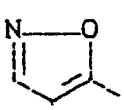 | - | 0 | $C_6H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 878 | 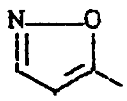 | - | 0 | $OCHF_2$ | - | |
| 879 | 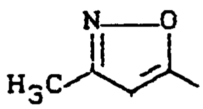 | - | 0 | $CO_2C_2H_5$ | - | |
| 880 | 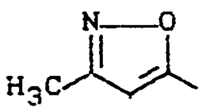 | - | 0 | Cl | - | |
| 881 | 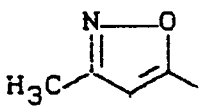 | - | 0 | $OCH_3$ | - | |
| 882 | 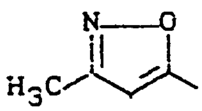 | - | 0 | $OC_2H_5$ | - | |
| 883 | 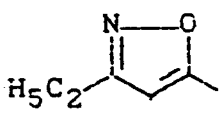 | - | 0 | $CO_2CH_3$ | - | |
| 884 | 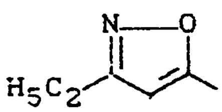 | - | 0 | $OCF_3$ | - | |
| 885 | 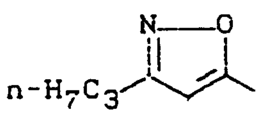 | - | 0 | $SO_2N(CH_3)_2$ | - | |
| 886 | 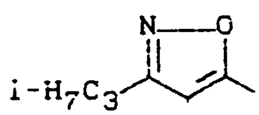 | - | 0 | $C_6H_5$ | - | |
| 887 | 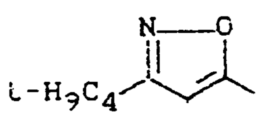 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 888 | 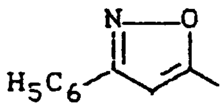 | - | 0 | $CO_2CH_3$ | - | |
| 889 | 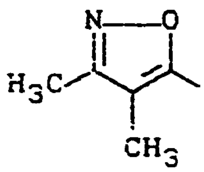 | - | 0 | $CO_2CH_3$ | - | |
| 890 | 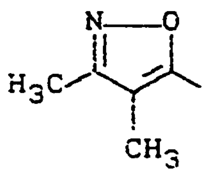 | $CH_2$ | 1 | $CO_2CH_3$ | - | |
| 891 | 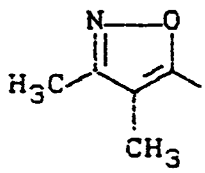 | - | 0 | $OCHF_2$ | - | |
| 892 | 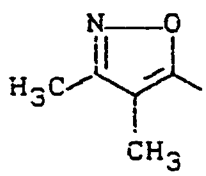 | - | 0 | $C_6H_5$ | - | |
| 893 | 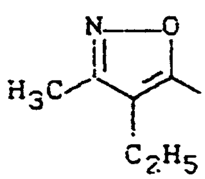 | - | 0 | $CO_2CH_3$ | - | |
| 894 | 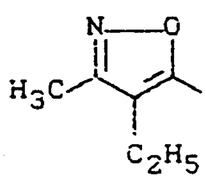 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 895 | 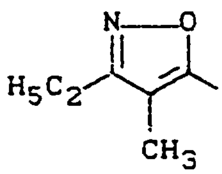 | - | 0 | $CO_2CH_3$ | - | |
| 896 | 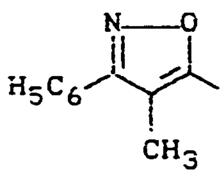 | - | 0 | $CO_2CH_3$ | - | |
| 897 | 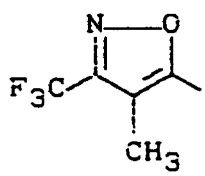 | - | 0 | $SO_2C_2H_5$ | - | |
| 898 | 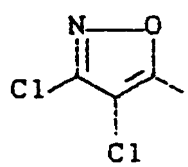 | - | 0 | $CO_2CH_3$ | - | |
| 899 | 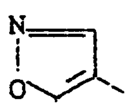 | - | 0 | $CO_2CH_3$ | - | |
| 900 | 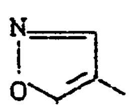 | - | 0 | $CO_2CH_3$ | (5)-Cl | |
| 901 | 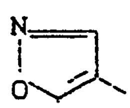 | - | 0 | $CO_2C_2H_5$ | - | |
| 902 | 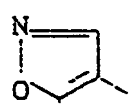 | - | 0 | $OCH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 903 | 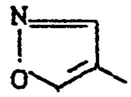 | - | 0 | $OC_2H_5$ | - | |
| 904 | 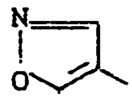 | - | 0 | $OCF_3$ | - | |
| 905 | 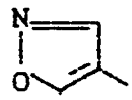 | - | 0 | Cl | - | |
| 906 | 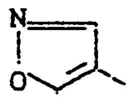 | - | 0 | $SC_2H_5$ | - | |
| 907 | 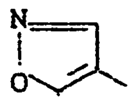 | - | 0 | $SO_2C_2H_5$ | - | |
| 908 | 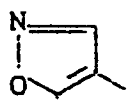 | - | 0 | $C_3H_7$ | - | |
| 909 | 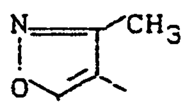 | - | 0 | $CO_2CH_3$ | - | |
| 910 | 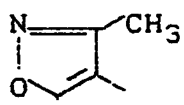 | - | 0 | $CO_2C_2H_5$ | - | |
| 911 | 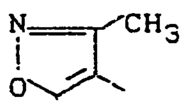 | - | 0 | $SC_2H_5$ | - | |
| 912 | 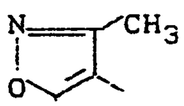 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 913 | 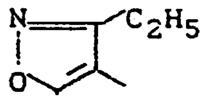 | - | 0 | $CO_2CH_3$ | - | |
| 914 | 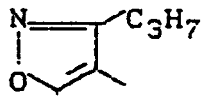 | - | 0 | $CO_2CH_3$ | - | |
| 915 | 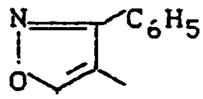 | - | 0 | $CO_2CH_3$ | - | |
| 916 | 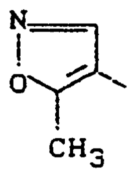 | - | 0 | $CO_2CH_3$ | - | |
| 917 | 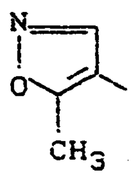 | - | 0 | $CO_2CH_3$ | (5)-Cl | |
| 918 | 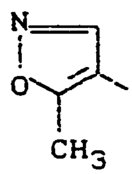 | - | 0 | $CO_2CH_3$ | (6)-Cl | |
| 919 | 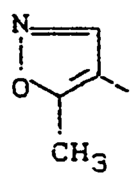 | - | 0 | $OCH_3$ | - | |
| 920 | 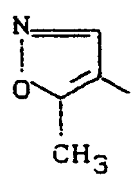 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 921 | 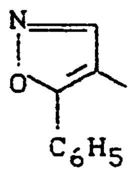 | - | 0 | $CO_2CH_3$ | - | |
| 922 | 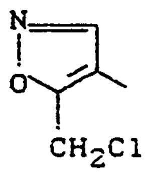 | - | 0 | $CO_2CH_3$ | - | |
| 923 | 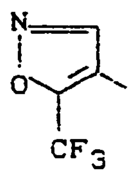 | - | 0 | $CO_2CH_3$ | - | |
| 924 | 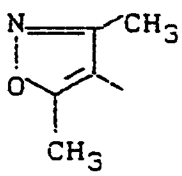 | - | 0 | $CO_2C_2H_5$ | - | |
| 925 | 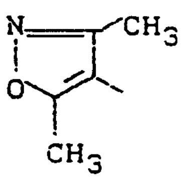 | - | 0 | $CO_2CH_3$ | (6)-Cl | |
| 926 | 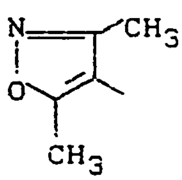 | - | 0 | $OCF_3$ | - | |
| 927 | 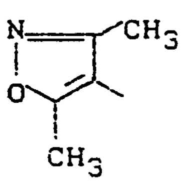 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 928 | 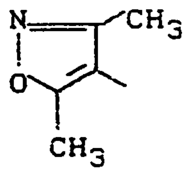 | - | 0 | $SC_3H_{7-i}$ | - | |
| 929 | 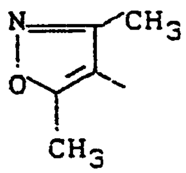 | - | 0 | $SO_2C_2H_5$ | - | |
| 930 | 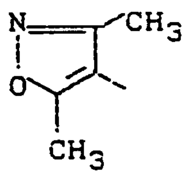 | - | 0 | $SO_2C_3H_{7-i}$ | - | |
| 931 | 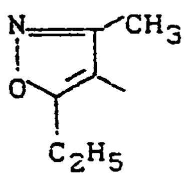 | - | 0 | $CO_2CH_3$ | - | |
| 932 | 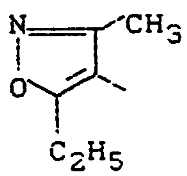 | - | 0 | $OCH_3$ | - | |
| 933 | 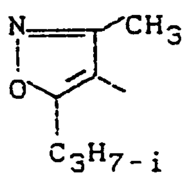 | - | 0 | $OC_2H_5$ | - | |
| 934 | 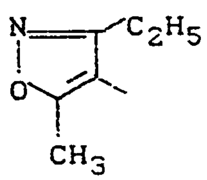 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 935 | 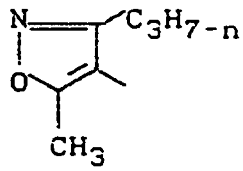 | - | 0 | $OCF_3$ | - | |
| 936 | 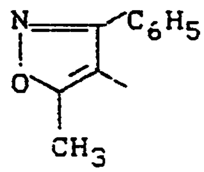 | - | 0 | $SO_2N(CH_3)_2$ | - | |
| 937 | 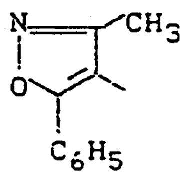 | - | 0 | $CO_2CH_3$ | - | |
| 938 | 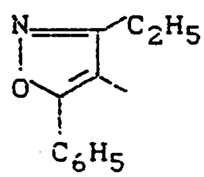 | - | 0 | $CO_2CH_3$ | - | |
| 939 | 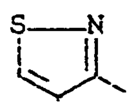 | - | 0 | $CO_2CH_3$ | - | |
| 940 | 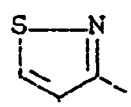 | - | 0 | $CO_2C_2H_5$ | - | |
| 941 | 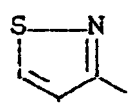 | - | 0 | $CO_2CH_3$ | (5)-Cl | |
| 942 | 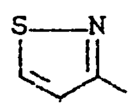 | - | 0 | Cl | (6)-Cl | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 943 | 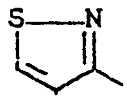 | - | 0 | $OCH_3$ | - | |
| 944 | 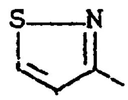 | - | 0 | $OC_2H_5$ | - | |
| 945 | 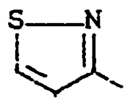 | - | 0 | $OCF_3$ | - | |
| 946 | 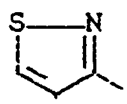 | - | 0 | $SC_2H_5$ | - | |
| 947 | 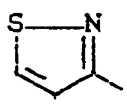 | - | 0 | $SO_2C_2H_5$ | - | |
| 948 | 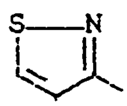 | - | 0 | $C_6H_5$ | - | |
| 949 | 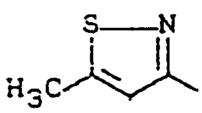 | - | 0 | $CO_2CH_3$ | - | |
| 950 | 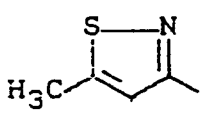 | - | 0 | $CO_2C_2H_5$ | - | |
| 951 | 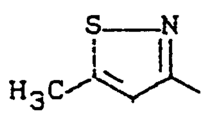 | - | 0 | $OC_2H_5$ | - | |
| 952 | 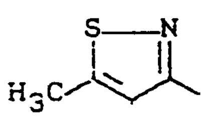 | - | 0 | $SC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 953 | 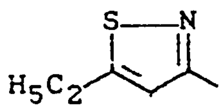 | - | 0 | $CO_2CH_3$ | - | |
| 954 | 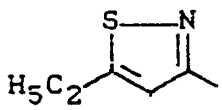 | - | 0 | $CO_2C_2H_5$ | - | |
| 955 | 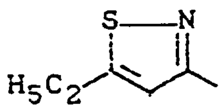 | - | 0 | $OCH_3$ | - | |
| 956 | 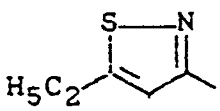 | - | 0 | $SCH_3$ | - | |
| 957 | 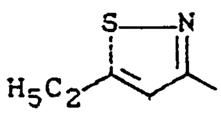 | - | 0 | $SC_2H_5$ | - | |
| 958 | 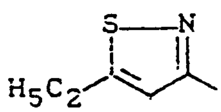 | - | 0 | $OCF_3$ | - | |
| 959 | 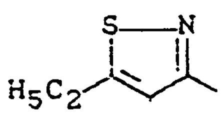 | - | 0 | $SO_2C_2H_5$ | - | |
| 960 | 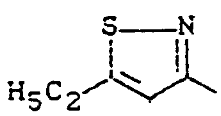 | - | 0 | $C_6H_5$ | - | |
| 961 | 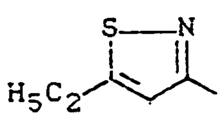 | $CH_2$ | 1 | $CO_2CH_3$ | - | |
| 962 | 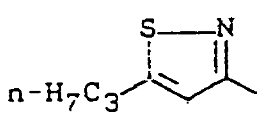 | - | 0 | $CO_2CH_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 963 | 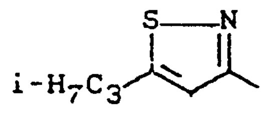 | - | 0 | $CO_2CH_3$ | - | |
| 964 | 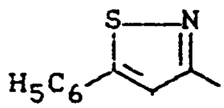 | - | 0 | $CO_2CH_3$ | - | |
| 965 | 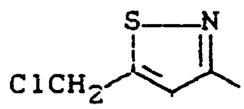 | - | 0 | $CO_2CH_3$ | - | |
| 966 | 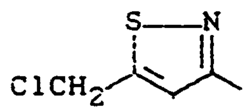 | - | 0 | $OC_2H_5$ | - | |
| 967 | 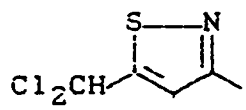 | - | 0 | $CO_2CH_3$ | - | |
| 968 | 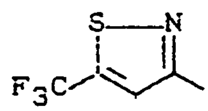 | - | 0 | $CO_2CH_3$ | - | |
| 969 | 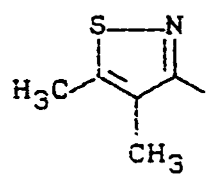 | - | 0 | $CO_2CH_3$ | - | |
| 970 | 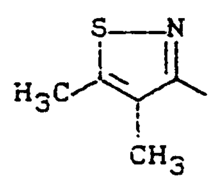 | $CH_2$ | 1 | $CO_2CH_3$ | - | |
| 971 | 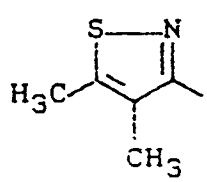 | - | 0 | Cl | - | |

<u>Tabelle 1</u> - Fortsetzung

| Bsp.-Nr. | $R^1$ | A | n | $R^2$ | (Position-) $R^3$ | Schmelzpunkt (°C) |
|---|---|---|---|---|---|---|
| 972 | 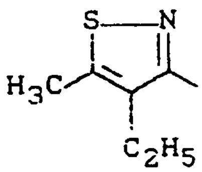 | - | 0 | $CO_2CH_3$ | - | |
| 973 | 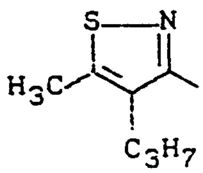 | - | 0 | $SCH_3$ | - | |
| 974 | 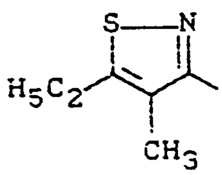 | - | 0 | $SO_2C_2H_5$ | - | |
| 975 | 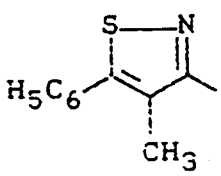 | - | 0 | $CO_2CH_3$ | - | |
| 976 | 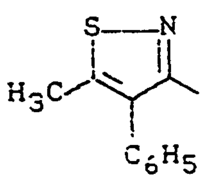 | - | 0 | $CO_2CH_3$ | - | |
| 977 | 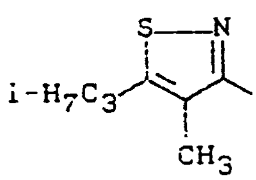 | - | 0 | $OCF_3$ | - | |
| 978 | 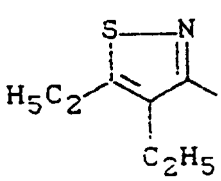 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 979 | 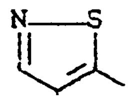 | - | 0 | $CO_2CH_3$ | - | |
| 980 | 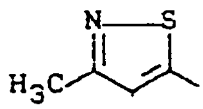 | - | 0 | $CO_2C_2H_5$ | - | |
| 981 | 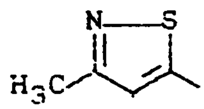 | - | 0 | $CO_2CH_3$ | - | |
| 982 | 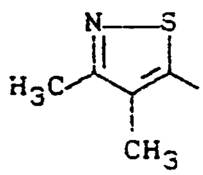 | - | 0 | $CO_2CH_3$ | - | |
| 983 | 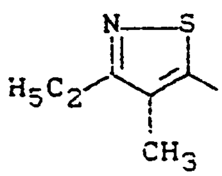 | - | 0 | $C_6H_5$ | - | |
| 984 | 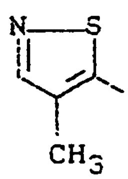 | - | 0 | $CO_2CH_3$ | - | |
| 985 | 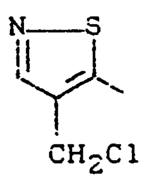 | - | 0 | $CO_2CH_3$ | - | |
| 986 | 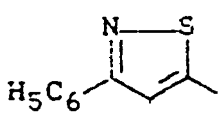 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 987 | 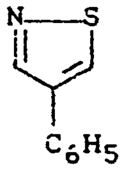 | - | 0 | $CO_2C_2H_5$ | - | |
| 988 | 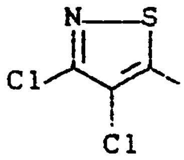 | - | 0 | $CO_2CH_3$ | - | |
| 989 | 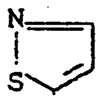 | - | 0 | $CO_2CH_3$ | - | |
| 990 | 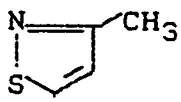 | - | 0 | $CO_2CH_3$ | - | |
| 991 | 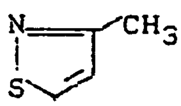 | - | 0 | $OCF_3$ | - | |
| 992 | 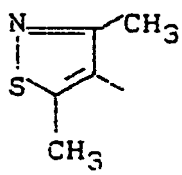 | - | 0 | $CO_2CH_3$ | - | |
| 993 | 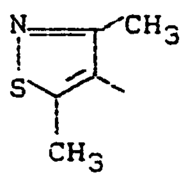 | - | 0 | $CO_2C_2H_5$ | - | |
| 994 | 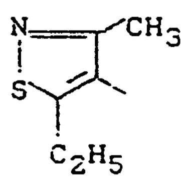 | - | 0 | $OC_2H_5$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 995 | 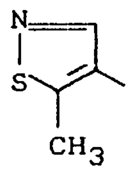 | - | 0 | $CO_2CH_3$ | - | |
| 996 | 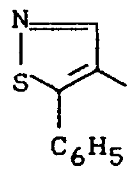 | - | 0 | $CO_2C_2H_5$ | - | |
| 997 | 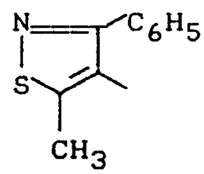 | - | 0 | $SO_2C_2H_5$ | - | |
| 998 | 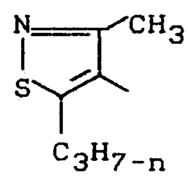 | - | 0 | $CO_2CH_3$ | - | |
| 999 | 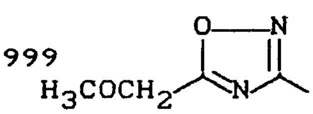 | - | 0 | $CO_2CH_3$ | - | 106 |
| 1000 | 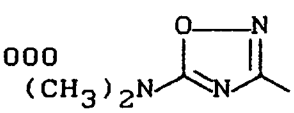 | - | 0 | $OC_2H_5$ | - | 159 |
| 1001 | 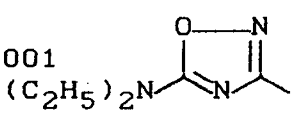 | - | 0 | $OC_2H_5$ | - | 160 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 1002 | 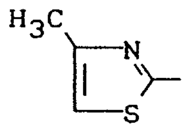 | - | 0 | $CH_3$ | (6)-Cl | 209 |
| 1003 | 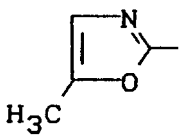 | - | 0 | $CO_2CH_3$ | (5)-Cl | 145 |
| 1004 | 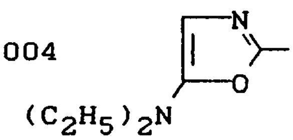 | - | 0 | $CO_2CH_3$ | - | 135 |
| 1005 | 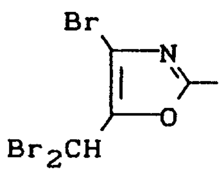 | - | 0 | $CO_2CH_3$ | - | 186 |
| 1006 | 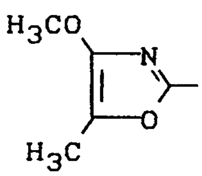 | - | 0 | $CO_2CH_3$ | - | 92 |
| 1007 | 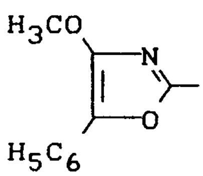 | - | 0 | $CO_2CH_3$ | - | 186 |
| 1008 | 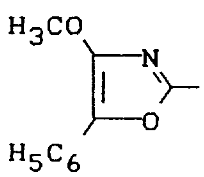 | - | 0 | $OCF_3$ | - | 189 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 1009 | 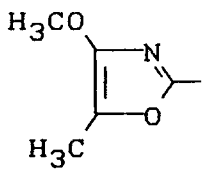 | - | 0 | $OCF_3$ | - | 115 |
| 1010 | 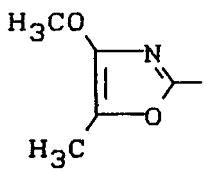 | - | 0 | Cl | - | 140 |
| 1011 | 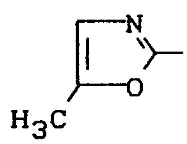 | - | 0 | $OC_2H_5$ | - | 143 |
| 1012 | 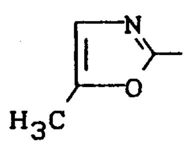 | - | 0 | $OC_3H_7$-n | - | 101 |
| 1013 | 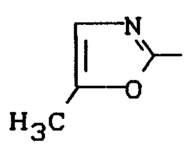 | - | 0 | $OC_3H_7$-i | - | 128 |
| 1014 | 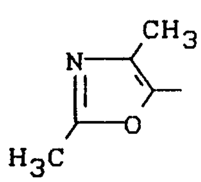 | - | 0 | $CO_2C_2H_5$ | - | 152 |
| 1015 | 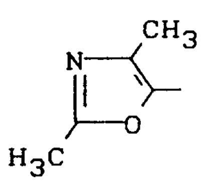 | - | 0 | $CO_2C_3H_7$-n | - | 151 |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 1016 | 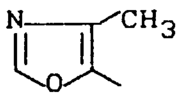 | - | 0 | $CO_2CH_3$ | - | 113 |
| 1017 | 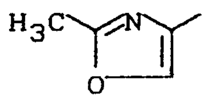 | - | 0 | $CO_2CH_3$ | - | 137 |
| 1018 | 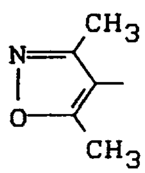 | - | 0 | $CO_2CH_3$ | - | 119 |
| 1019 | 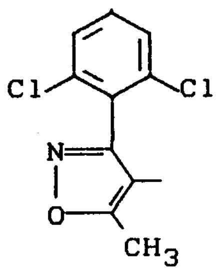 | - | 0 | $CO_2CH_3$ | - | 135 |
| 1020 | 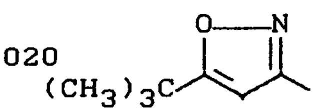 | - | 0 | $OC_2CH_3$ | - | 184 |
| 1021 | 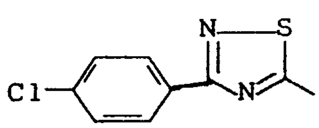 | - | 0 | $OCF_3$ | - | 165 |
| 1022 | 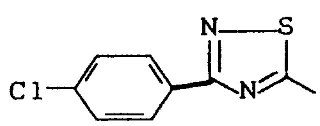 | - | 0 | $CO_2CH_3$ | - | 215 |

Tabelle 1 - Fortsetzung

| Bsp.-Nr. | $R^1$ | A | n | $R^2$ | (Position-) $R^3$ | Schmelzpunkt (° C) |
|---|---|---|---|---|---|---|
| 1023 | 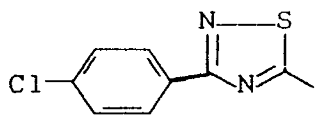 | - | 0 | $C_6H_5$ | - | 196 |
| 1024 | 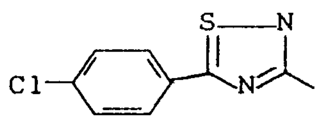 | - | 0 | $CO_2CH_3$ | - | 215 |
| 1025 | 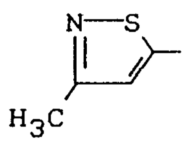 | - | 0 | $CO_2CH_3$ | - | 203 |
| 1026 | 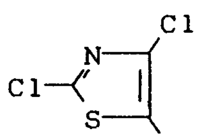 | - | 0 | $CO_2CH_3$ | - | 160 |
| 1027 | 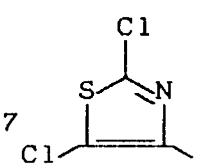 | - | 0 | $CO_2CH_3$ | - | 136 |
| 1028 | 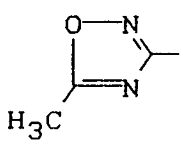 | NH | 1 | $CO_2CH_3$ | - | |
| 1029 | 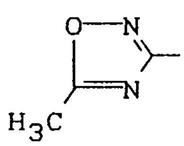 | NH | 1 | $OCF_3$ | - | |

Tabelle 1 - Fortsetzung

| Bsp.-Nr. | $R^1$ | A | n | $R^2$ | (Position-) $R^3$ | Schmelzpunkt (°C) |
|---|---|---|---|---|---|---|
| 1030 | 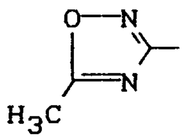 | NH | 1 | $CF_3$ | - | |
| 1031 | 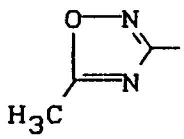 | NH | 1 | $OC_2H_5$ | - | |
| 1032 | 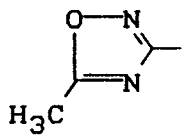 | NH | 1 | $CO_2C_2H_5$ | - | |
| 1033 | 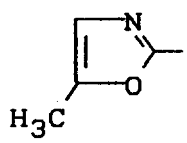 | NH | 1 | $CO_2CH_3$ | - | |
| 1034 | 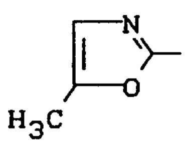 | NH | 1 | $C_6H_5$ | - | |
| 1035 | 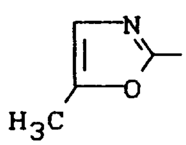 | NH | 1 | $OC_2H_5$ | - | |
| 1036 | 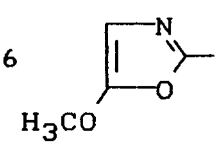 | NH | 1 | $CO_2CH_3$ | - | |

Analog Beispiel 3 kann auch das Natriumsalz der in Tabelle 1 als Beispiel 27 aufgeführten Verbindung (27a) erhalten werden:

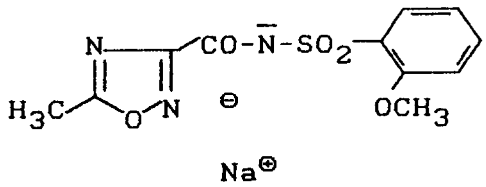

Fp.: ~ 100 °C (Zersetzung)

Ausgangsstoffe der Formel (IV)

- bzw. reaktionsfähige Derivate

Beispiel (IV-1)

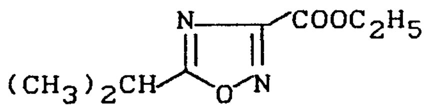

1. Stufe:

$(CH_3)_2CH\text{-}CO\text{-}NH\text{-}CH(COOC_2H_5)_2$

48,7 g (0,23 Mol) Aminomalonsäurediethylester-hydrochlorid werden in 500 ml Methylenchlorid gelöst und die Lösung auf -10°C abgekühlt. Dann gibt man 50,5 g (0,5 Mol) Triethylamin zu und tropft anschließend 25,6 g (0,24 Mol) Isobuttersäurechlorid zu, Man läßt das Reaktionsgemisch auf Raumtemperatur kommen, rührt noch 15 Stunden bei Raumtemperatur, filtriert den Niederschlag ab, engt das Filtrat ein und bringt den Rückstand durch Verreiben mit Diethylether zur Kristallisation.

Man erhält 55 g (0,22 Mol) (98% der Theorie) N-Isobutyroylaminomalonsäurediethylester vom Schmelzpunkt 74°C.

2. Stufe:

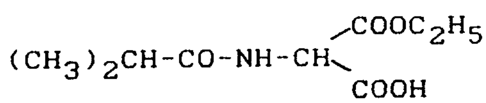

53,9 g (0,22 Mol) N-Isobutyroylaminomalonsäurediethylester werden in 200 ml Ethanol gelöst. Zu dieser Lösung tropft man unter Kühlung eine Lösung von 12,3 g (0,22 Mol) Kaliumhydroxid in 20 ml Wasser so zu, daß 25°C nicht überschritten werden, Man rührt das Reaktionsgemisch 48 Stunden bei 20°C bis 25°C, neutralisiert unter Kühlung mit 37%iger Salzsäure, saugt das Reaktionsprodukt ab, wäscht mit Diethylether nach und trocknet es im Exsikkator.

Man erhält 25,6 g (53,7% der Theorie) N-Isobutyroylaminomalonsäuremonoethylester vom Schmelzpunkt 116°C.

3. Stufe:

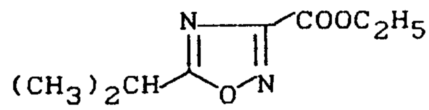

23,9 g (0,11 Mol) N-Isobutyroylaminomalonsäuremonoethylester werden in 100 ml Eisessig gelöst. Nach dem Abkühlen auf 0°C wird zu dieser Lösung eine Lösung von 8,3 g (0,12 Mol) Natriumnitrit in 20 ml Wasser getropft. Man rührt das Reaktionsgemisch 15 Stunden bei Raumtemperatur, verdünnt mit 100 ml Wasser und extrahiert mit 200 ml Diethylether. Die Etherphase wird abgetrennt, über Natriumsulfat getrocknet, filtriert und eingeengt.

Als öliger Rückstand verbleiben 17,2 g (85% der Theorie) 5-Isopropyl-1,2,4-oxadiazol-3-carbonsäureethylester.

Beispiel (IV-2)

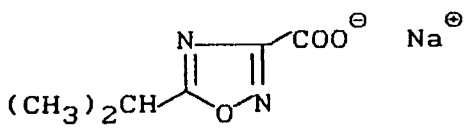

9,2 g (0,05 Mol) 5-Isopropyl-1,2,4-oxadiazol-3-carbonsäureethylester werden in 100 ml Ethanol gelöst, 2 g Natriumhydroxid, gelöst in 10 ml Wasser zugegeben und 4 Stunden bei Raumtemperatur gerührt. Dann wird das Reaktionsgemisch unter vermindertem Druck eingeengt und der Rückstand mit Diethylether verrührt. Das Produkt wird abgesaugt und an der Luft getrocknet.

Man erhält 8,3 g (93% der Theorie) des Natrium-Salzes der 5-Isopropyl-1,2,4-oxadiazol-3-carbonsäure, welches bei 137°C unter Zersetzung schmilzt.

Anwendungsbeispiele

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine sehr gute herbizide Wirksamkeit bei guter Nutzpflanzenselektivität zeigen in diesem Test z.B. die Verbindungen gemäß folgender Herstellungsbeispiele: (2), (3), (3a), (3b), (3c), (14), (19), (20), (21), (25), (26) und (28).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: (2), (3), (3a), (3b), (3c), (4), (5), (6), (14), (15), (20), (21), (25), (26), (28).

**Patentansprüche**

**1.** Sulfonylierte Carbonsäureamide der allgemeinen Formel (I)

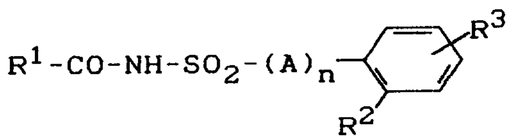

(I)

in welcher

n für die Zahlen 0 oder 1 steht,

A für Sauerstoff, Imino (NH) oder Methylen ($CH_2$) steht,

$R^1$ für einen fünfgliedrigen Heteroarylrest aus der Reihe Oxazolyl, Isoxazolyl, Thiazolyl, Isothiazolyl, Oxadiazolyl, Thiadiazolyl steht, welcher gegebenenfalls einfach oder mehrfach, insbesondere einfach oder zweifach, gleich oder verschieden durch Halogen oder durch gegebenenfalls halogen-substituiertes $C_1$-$C_6$-Alkyl, $C_1$-$C_4$-Alkoxy-$C_1$-$C_4$-alkyl, Phenyl, $C_1$-$C_6$-Alkoxy, $C_1$-$C_6$-Alkylthio, $C_1$-$C_6$-Alkylsulfinyl, $C_1$-$C_6$-Alkylsulfonyl, $C_1$-$C_6$-Alkylamino oder Di-($C_1$-$C_4$-alkyl)-amino substituiert ist,

$R^2$ für Fluor, Chlor, Brom, Cyano, Nitro, Carboxy, Phenyl oder für jeweils gegebenenfalls durch Fluor und/oder Chlor substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, für Di-($C_1$-$C_2$-alkyl)-aminosulfonyl, für N-($C_1$-$C_2$-Alkyl)-N-($C_1$-$C_2$-alkoxy)-aminosulfonyl oder für gegebenenfalls durch Fluor, Chlor oder $C_1$-$C_2$-Alkoxy substituiertes $C_1$-$C_4$-Alkoxy-carbonyl steht, und

$R^3$ für Wasserstoff, Fluor, Chlor oder Methyl steht,

sowie Salze von Verbindungen der Formel (I).

**2.** Sulfonylierte Carbonsäureamide der allgemeinen Formel (I) gemäß Anspruch 1, dadurch gekennzeichnet, daß

n für die Zahlen 0 oder 1 steht,

A für Sauerstoff, Imino (NH) oder Methylen ($CH_2$) steht,

$R^1$ für einen fünfgliedrigen Heteroarylrest aus der Reihe Oxazolyl, Isoxazolyl, Thiazolyl, Isothiazolyl, 1,2,4-Oxadiazolyl, 1,3,4-Oxadiazolyl, 1,2,4-Thiadiazolyl, 1,3,4-Thiadiazolyl steht, welcher gegebenenfalls durch Fluor, Chlor, Brom, Methyl, Ethyl, Propyl, Isopropyl, Butyl, Isobutyl, sec-Butyl, tert-Butyl, Fluormethyl, Chlormethyl, Difluormethyl, Dichlormethyl, Trifluormethyl, Trichlormethyl, Chlorfluormethyl, Chlordifluormethyl, Fluordichlormethyl, Brommethyl, Dibrommethyl, Methoxymethyl, Phenyl, Methoxy, Ethoxy, Propoxy, Isopropyloxy, Difluormethoxy, Trifluormethoxy, Chlordifluormethoxy, Methylthio, Ethylthio, Propylthio, Isopropylthio, Difluormethylthio, Trifluormethylthio, Chlordifluormethylthio, Methylsulfinyl, Ethylsulfinyl, Propylsulfinyl, Methylsulfonyl, Ethylsulfonyl, Propylsulfonyl, Methylamino, Ethylamino, Propylamino, Isopropylamino und/oder Dimethylamino substituiert ist,

$R^2$ für Fluor, Chlor, Brom, Cyano, Nitro, Carboxy, Phenyl, Methyl, Ethyl, Trifluormethyl, Methoxy, Ethoxy, 2-Chlor-ethoxy, Difluormethoxy, Trifluormethoxy, Methylthio, Ethylthio, Propylthio, Methylsulfinyl, Ethylsulfinyl, Propylsulfinyl, Methylsulfonyl, Ethylsulfonyl, Propylsulfonyl, Dimethylaminosulfonyl, Diethylaminosulfonyl, N-Methoxy-N-methyl-aminosulfonyl, Methoxycarbonyl, Ethoxycarbonyl, Propoxycarbonyl, Isopropoxycarbonyl, Chlorethoxycarbonyl oder Methoxyethoxycarbonyl, steht und

$R^3$ für Wasserstoff, Fluor, Chlor oder Methyl steht,

sowie deren Salze (I) mit Basen, wie z.B. Natrium-, Kalium- oder Calcium-hydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen, Tri-($C_1$-$C_4$-alkyl)-aminen oder Tris-(2-hydroxyethyl)amin.

**3.** Sulfonylierte Carbonsäureamide der allgemeinen Formel (I) gemäß Anspruch 1, dadurch gekennzeichnet, daß

n für die Zahlen 0 oder 1 steht,

A für Methylen steht,

$R^1$ für 5-Methyl-1,2,4-oxadiazol-3-yl, 5-Ethyl-1,2,4-oxadiazol-3-yl, 5-Propyl-1,2,4-oxadiazol-3-yl, 5-Isopropyl-1,2,4-oxadiazol-3-yl, 4-Methyl-1,3-oxazol-2-yl, 4-Ethyl-1,3-oxazol-2-yl, 5-Methyl-1,3-oxazol-2-yl, 5-Ethyl-1,3-oxazol-2-yl oder 4,5-Dimethyl-1,3-oxazol-2-yl steht,

$R^2$ für Trifluormethyl, Phenyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Methoxycarbonyl oder Ethoxycarbonyl, steht und

$R^3$ für Wasserstoff, Chlor oder Methyl steht, sowie deren Salze mit Basen wie z.B. Natrium-, Kalium- oder Calziumhydroxid, -hydrid, -amid oder -carbonat, Natrium- oder Kalium-$C_1$-$C_4$-alkyl)-aminen, tri-($C_1$-$C_4$-alkyl)-amiden oder Tris(2-hydroxyethyl)amin.

**4.** Verfahren zur Herstellung von sulfonylierten Carbonsäureamiden der allgemeinen Formel (I)

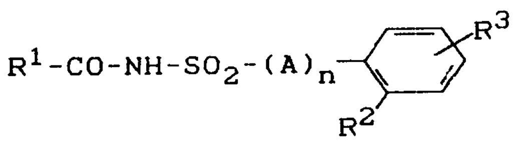

(I)

in welcher

n für die Zahlen 0 oder 1 steht,

A für Sauerstoff, Imino (NH) oder Methylen ($CH_2$) steht,

$R^1$ für einen fünfgliedrigen Heteroarylrest aus der Reihe Oxazolyl, Isoxazolyl, Thiazolyl, Isothiazolyl, Oxadiazolyl, Thiadiazolyl steht, welcher gegebenenfalls einfach oder mehrfach, insbesondere einfach oder zweifach, gleich oder verschieden durch Halogen oder durch gegebenenfalls halogen-substituiertes $C_1$-$C_6$-Alkyl, Phenyl, $C_1$-$C_6$-Alkoxy, $C_1$-$C_6$-Alkylthio, $C_1$-$C_6$-Alkylsulfinyl, $C_1$-$C_6$-Alkylsulfonyl, $C_1$-$C_6$-Alkylamino oder Di-($C_1$-$C_4$-alkyl)-amino substituiert ist,

$R^2$ für Fluor, Chlor, Brom, Cyano, Nitro, Carboxy, Phenyl oder für jeweils gegebenenfalls durch Fluor und/oder Chlor substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, für Di-($C_1$-$C_2$-alkyl)-aminosulfonyl, für N-($C_1$-$C_2$-Alkyl)-N-($C_1$-$C_2$-alkoxy)-aminosulfonyl oder für gegebenenfalls durch Fluor, Chlor oder $C_1$-$C_2$-Alkoxy substituiertes $C_1$-$C_4$-Alkoxy-carbonyl steht, und

$R^3$ für Wasserstoff, Fluor, Chlor oder Methyl steht,

sowie Salze von Verbindungen der Formel (I)

dadurch gekennzeichnet, daß man

(a) Carbonsäureamide der allgemeinen Formel (II)

$R^1$-CO-$NH_2$ (II)

in welcher

$R^1$ die oben angegebene Bedeutung hat,

mit Sulfonylierungsmitteln der allgemeinen Formel (III)

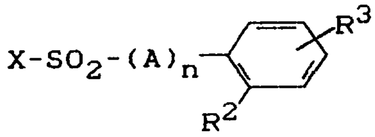

(III)

in welcher

n, A, $R^2$ und $R^3$ die oben angegebene Bedeutung haben und

X für Halogen steht,

gegebenenfalls in Gegenwart eines Säureakzeptors und gegebenenfalls in Gegenwart eines Verdünnungsmittels umsetzt, oder daß man

(b) Carbonsäuren der allgemeinen Formel (IV)

$R^1$-COOH (IV)

in welcher

$R^1$ die oben angegebene Bedeutung hat,

oder reaktionsfähige Derivate der Carbonsäuren der Formel (IV)

mit Aminosulfonylverbindungen der allgemeinen Formel (V)

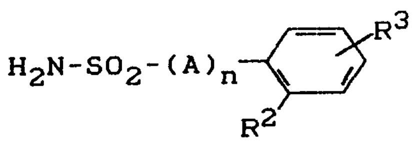

(V)

in welcher

n, A, $R^1$ und $R^2$ die oben angegebene Bedeutung haben,

oder mit reaktionsfähigen Derivaten der Verbindungen der Formel (V)

gegebenenfalls in Gegenwart von Reaktionshilfsmitteln und gegebenenfalls in Gegenwart von Verdünnungsmitteln umsetzt und gegebenenfalls die nach Verfahren (a) oder (b) erhaltenen Produkte nach üblichen Methoden in Salze überführt.

**5.** Herbizide Mittel, gekennzeichnet durch einen Gehalt an mindestens einem sulfonylierten Carbonsäureamid der Formel (I) gemäß den Ansprüchen 1 oder 4.

**6.** Verfahren zur Bekämpfung von unerwünschten Pflanzen, dadurch gekennzeichnet, daß man sulfonylierte Carbonsäureamide der Formel (I) gemäß Ansprüchen 1 oder 4 auf die Pflanzen und/oder ihren Lebensraum einwirken läßt.

**7.** Verwendung von sulfonylierten Carbonsäureamiden der Formel (I) gemäß den Ansprüchen 1 oder 4 zur Bekämpfung von unerwünschten Pflanzen.

**8.** Verfahren zur Herstellung von herbiziden Mitteln, dadurch gekennzeichnet, daß man sulfonylierte Carbonsäureamide der Formel (I) gemäß den Ansprüchen 1 oder 4 mit Streckmitteln und/oder oberflächenaktiven Substanzen vermischt.

Europäisches Patentamt

# EUROPÄISCHER RECHERCHENBERICHT

Nummer der Anmeldung
EP 93 10 7139

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 482 349 (BASF AG)<br>29. April 1992<br>Tabelle 1 A=A1-**, B=(Iso)oxazole, (Iso)thiazole, Oxadiazole oder Thiadiazole<br>* Anspruch 1 *<br>--- | 1-8 | C07D261/06<br>A01N43/00<br>C07D263/30<br>C07D271/06<br>C07D271/08<br>C07D277/20<br>C07D285/08 |
| D,X | EP-A-0 459 244 (BAYER AG)<br>4. Dezember 1991<br>Tabelle 1 und 2<br>R2=orthosubstituiertes-Phenyl<br>* Anspruch 1 *<br>--- | 1-8 | |
| D,X | DE-A-4 029 753 (BASF AG)<br>26. März 1992<br>Tabelle 1 A=A1-**, B=(Iso)oxazole, (Iso)thiazole, Thiadiazole<br>* Anspruch 1 *<br>--- | 1-8 | |
| D,A | EP-A-0 244 166 (E.I. DU PONT DE NEMOURS)<br>4. November 1987<br>* Anspruch 1 *<br>--- | 1-8 | RECHERCHIERTE SACHGEBIETE (Int. Cl.5) |
| D,A | US-A-4 838 925 (E.I. DU PONT DE NEMOURS)<br>13. Juni 1989<br>* Anspruch 1 *<br>----- | 1-8 | C07D<br>A01N |

Der vorliegende Recherchenbericht wurde für alle Patentansprüche erstellt

| Recherchenort | Abschlußdatum der Recherche | Prüfer |
|---|---|---|
| MUENCHEN | 25 AUGUST 1993 | GETTINS M.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument